# EUROPEAN PATENT APPLICATION

(11) **EP 4 112 642 A1**
(43) Date of publication of application: **04.01.2023**
(21) Application number: 21760663.1
(22) Date of filing: 26.02.2021
(51) Int. Cl.: C07K 16/28, C12N 15/13, C12N 15/63, C12N 5/10, A61K 39/395, A61P 35/00

(54) **ANTI-CD137 CONSTRUCT AND USE THEREOF**

(30) Priority: 28.02.2020 CN 202010128290
(71) Applicant: Shanghai Henlius Biotech, Inc., Shanghai 201210 (CN)
(72) Inventor: XUE, Jie, Shanghai 201210 (CN); JIANG, Wei-Dong, Shanghai 201210 (CN); XU, Wenfeng, Shanghai 201210 (CN)
(74) Representative: Rentsch Partner AG
(86) International application number: PCT/CN2021/078047
(87) International publication number: WO 2021/170067

(57) **Abstract**

The present application provides anti-CD137 constructs that bind to CD137 (e.g., anti-CD137 monoclonal antibodies and multispecific antibodies), nucleic acid molecules encoding an amino acid sequence of the anti-CD137 constructs, vectors comprising the nucleic acid molecules, host cells containing the vectors, methods of preparing the anti-CD 137 constructs, pharmaceutical compositions containing the anti-CD137 constructs, and methods of using the anti-CD137 constructs or compositions.

## Description

### CROSS-REFERENCE TO RELATED APPLICATIONS

This application claims priority to Chinese Patent Application with Application No. CN202010128290.3, filed February 28, 2020, the contents of which is incorporated by reference in its entirety, and to which priority is claimed.

### FIELD

The present application relates to antibodies that bind to CD137 and uses thereof including treating diseases or conditions.

### BACKGROUND

CD137 (also referred to as 4-1BB and TNFRSF9) is a transmembrane protein of the Tumor Necrosis Factor receptor superfamily (TNFRS). Current understanding of CD137 indicates that expression is generally activation dependent and is present in a broad subset of immune cells including activated NK and NKT cells, regulatory T cells, dendritic cells (DC), stimulated mast cells, differentiating myeloid cells, monocytes, neutrophils, and eosinophils (Wang, 2009, Immunological Reviews 229: 192-215). CD137 expression has also been demonstrated on tumor vasculature (Broll, 2001, Amer. J. Clin. Pathol. 115(4):543-549; Seaman, 2007, Cancer Cell 11: 539-554) and at sites of inflamed or atherosclerotic endothelium (Drenkard, 2007 FASEB J. 21: 456-463; Olofsson, 2008, Circulation 117: 1292-1301). The ligand that stimulates CD137, *i.e.,* CD137 Ligand (4-1BBL), is expressed on activated antigen-presenting cells (APCs), myeloid progenitor cells, and hematopoietic stem cells.

Human CD137 is expressed on the cell surface in monomer and dimer forms and likely trimerizes with CD137 ligand to activate downstream signals. Studies of murine and human T cells indicate that CD137 promotes enhanced cellular proliferation, survival, and cytokine production (Croft, 2009, Nat Rev Immunol 9:271-285). Studies have indicated that some CD137 agonist mAbs increase costimulatory molecule expression and markedly enhance cytolytic T lymphocyte responses, resulting in anti-tumor efficacy in various models. CD137 agonist mAbs have demonstrated efficacy in prophylactic and therapeutic settings. Further, CD137 monotherapy and combination therapy tumor models have established durable anti-tumor protective T cell memory responses (Lynch, 2008, Immunol Rev. 22: 277-286). CD137 agonists also have been shown to inhibit autoimmune reactions in a variety of art-recognized autoimmunity models (Vinay, 2006, J Mol Med 84:726-736). This dual activity of CD137 offers the potential to provide anti-tumor activity while dampening autoimmune side effects that can be associated with immunotherapy approaches that break immune tolerance.

The contents of all publications, patents, patent applications and published patent applications referred to in this application are incorporated by reference in their entireties.

### SUMMARY OF THE APPLICATION

The present application provides anti-CD137 constructs (e.g., anti-CD137 monoclonal antibodies and anti-CD137 multispecific antibodies), polynucleotides encoding anti-CD137 constructs, kits, methods of modulating cell composition, and methods of treating an individual using the anti-CD 137 constructs. The invention is based, in part, on the discovery of anti-CD137 monospecific antibodies and multispecific antibodies that exhibit improved safety profile and enhanced anti-tumor efficacy compared to existing anti-CD137 antibodies in clinical trials.

One aspect of the present application provides isolated anti-CD137 constructs comprising an antibody moiety that binds to CD137, comprising a heavy chain variable region (V_{H}) and a light chain variable region (V_{L}), wherein: a) the V_{H} comprises: i) an HC-CDR1 comprising an amino acid sequence of GFX₁X₂X₃DTYIX₄ (SEQ ID NO: 177), wherein X₁=N or C; X₂=I, P, L or M; X₃=K, N, R, C or Q; X₄=H or Q, ii) an HC-CDR2 comprising an amino acid sequence of X₁IDPANGX₂X₃X₄ (SEQ ID NO:178), wherein X₁=K or R; X₂=N, G, F, Y, A, D, L, M or Q; X₃=S or T; X₄=E or M, and iii) an HC-CDR3 comprising an amino acid sequence of GNLHYX₁LMD (SEQ ID NO: 179), wherein X₁=Y, A or G; and b) the V_{L} comprises: i) an LC-CDR1 comprising an amino acid sequence of KASQX₁X₂X₃TYX₄S (SEQ ID NO: 180), wherein X₁=A, P or T; X₂=I, T or P; X₃=N or A; X₄=L, G or H, ii) an LC-CDR2 comprising an amino acid sequence of RX₁NRX₂X₃X₄ (SEQ ID NO: 181), wherein X₁=A, Y, V or D; X₂=M, K, V or A; X₃=V, P, Y or G; X₄=D or G, and iii) an LC-CDR3 comprising an amino acid sequence of LQX₁X₂DFPYX₃ (SEQ ID NO: 182), wherein X₁=Y, S or F; X₂=D, V, L, R, E or Q; X₃=T or K.

In some embodiments, the HC-CDR1 comprises an amino acid sequence of any one of SEQ ID NOs: 1, 11, 21, 31, 41, 51, 61, 71, 81, 91, 101, 111, 121, 131, 141, 211 and 221, or a variant thereof comprising up to about 3 amino acid substitutions; the HC-CDR2 comprises an amino acid sequence of any one of SEQ ID NOs: 2, 12, 22, 32, 42, 52, 62, 72, 82, 92, 102, 112, 122, 132, 142, 212 and 222, or a variant thereof comprising up to about 3 amino acid substitutions; the HC-CDR3 comprises an amino acid sequence of any one of SEQ ID NOs: 3, 13, 23, 33, 43, 53, 63, 73, 83, 93, 103, 113, 123, 133, 143, 213 and 223, or a variant thereof comprising up to about 3 amino acid substitutions; the LC-CDR1 comprises an amino acid sequence of any one of SEQ ID NOs: 4, 14, 24, 34, 44, 54, 64, 74, 84, 94, 104, 114, 124, 134, 144, 214 and 224, or a variant thereof comprising up to about 3 amino acid substitutions; the LC-CDR2 comprises an amino acid sequence of any one of SEQ ID NOs: 5, 15, 25, 35, 45, 55, 65, 75, 85, 95, 105, 115, 125, 135, 145, 215 and 225, or a variant thereof comprising up to about 3 amino acid substitutions; and the LC-CDR3 comprises an amino acid sequence of any one of SEQ ID NOs: 6, 16, 26, 36, 46, 56, 66, 76, 86, 96, 106, 116, 126, 136, 146, 216 and 226, or a variant thereof comprising up to about 3 amino acid substitutions.

Another aspect of the present application provides anti-CD137 constructs comprising an anti-CD137 antibody moiety that cross-competes for binding to CD137 with a reference anti-CD137 construct comprising a heavy chain variable region (V_{H}) comprising HC-CDR1, HC-CDR2 and HC-CDR3 domains and a light chain variable region (V_{L}) comprising LC-CDR1, LC-CDR2 and LC-CDR3 domains that are selected from the group consisting of: a) the V_{H} comprises the HC-CDR1 comprising the amino acid sequence of SEQ ID NO: 1, the HC-CDR2 comprising the amino acid sequence of SEQ ID NO: 2, and the HC-CDR3 comprising the amino acid sequence of SEQ ID NO: 3, and the V_{L} comprises the LC-CDR1 comprising the amino acid sequence of SEQ ID NO: 4, the LC-CDR2 comprising the amino acid sequence of SEQ ID NO: 5, and the LC-CDR3 comprising the amino acid sequence of SEQ ID NO: 6; b) the V_{H} comprises the HC-CDR1 comprising the amino acid sequence of SEQ ID NO: 11, the HC-CDR2 comprising the amino acid sequence of SEQ ID NO: 12, and the HC-CDR3 comprising the amino acid sequence of SEQ ID NO: 13, and the V_{L} comprises the LC-CDR1 comprising the amino acid sequence of SEQ ID NO: 14, the LC-CDR2 comprising the amino acid sequence of SEQ ID NO: 15, and the LC-CDR3 comprising the amino acid sequence of SEQ ID NO: 16; c) the V_{H} comprises the HC-CDR1 comprising the amino acid sequence of SEQ ID NO: 21, the HC-CDR2 comprising the amino acid sequence of SEQ ID NO: 22, and the HC-CDR3 comprising the amino acid sequence of SEQ ID NO: 23, and the V_{L} comprises the LC-CDR1 comprising the amino acid sequence of SEQ ID NO: 24, the LC-CDR2 comprising the amino acid sequence of SEQ ID NO: 25, and the LC-CDR3 comprising the amino acid sequence of SEQ ID NO: 26; d) the V_{H} comprises the HC-CDR1 comprising the amino acid sequence of SEQ ID NO: 31, the HC-CDR2 comprising the amino acid sequence of SEQ ID NO: 32, and the HC-CDR3 comprising the amino acid sequence of SEQ ID NO: 33, and the V_{L} comprises the LC-CDR1 comprising the amino acid sequence of SEQ ID NO: 34, the LC-CDR2 comprising the amino acid sequence of SEQ ID NO: 35, and the LC-CDR3 comprising the amino acid sequence of SEQ ID NO: 36; e) the V_{H} comprises the HC-CDR1 comprising the amino acid sequence of SEQ ID NO: 41, the HC-CDR2 comprising the amino acid sequence of SEQ ID NO: 42, and the HC-CDR3 comprising the amino acid sequence of SEQ ID NO: 43, and the V_{L} comprises the LC-CDR1 comprising the amino acid sequence of SEQ ID NO: 44, the LC-CDR2 comprising the amino acid sequence of SEQ ID NO: 45, and the LC-CDR3 comprising the amino acid sequence of SEQ ID NO: 46; f) the V_{H} comprises the HC-CDR1 comprising the amino acid sequence of SEQ ID NO: 51, the HC-CDR2 comprising the amino acid sequence of SEQ ID NO: 52, and the HC-CDR3 comprising the amino acid sequence of SEQ ID NO: 53, and the V_{L} comprises the LC-CDR1 comprising the amino acid sequence of SEQ ID NO: 54, the LC-CDR2 comprising the amino acid sequence of SEQ ID NO: 55, and the LC-CDR3 comprising the amino acid sequence of SEQ ID NO: 56; g) the V_{H} comprises the HC-CDR1 comprising the amino acid sequence of SEQ ID NO: 61, the HC-CDR2 comprising the amino acid sequence of SEQ ID NO: 62, and the HC-CDR3 comprising the amino acid sequence of SEQ ID NO: 63, and the V_{L} comprises the LC-CDR1 comprising the amino acid sequence of SEQ ID NO: 64, the LC-CDR2 comprising the amino acid sequence of SEQ ID NO: 65, and the LC-CDR3 comprising the amino acid sequence of SEQ ID NO: 66; h) the V_{H} comprises the HC-CDR1 comprising the amino acid sequence of SEQ ID NO: 71, the HC-CDR2 comprising the amino acid sequence of SEQ ID NO: 72, and the HC-CDR3 comprising the amino acid sequence of SEQ ID NO: 73, and the V_{L} comprises the LC-CDR1 comprising the amino acid sequence of SEQ ID NO: 74, the LC-CDR2 comprising the amino acid sequence of SEQ ID NO: 75, and the LC-CDR3 comprising the amino acid sequence of SEQ ID NO: 76; i) the V_{H} comprises the HC-CDR1 comprising the amino acid sequence of SEQ ID NO: 81, the HC-CDR2 comprising the amino acid sequence of SEQ ID NO: 82, and the HC-CDR3 comprising the amino acid sequence of SEQ ID NO: 83, and the V_{L} comprises the LC-CDR1 comprising the amino acid sequence of SEQ ID NO: 84, the LC-CDR2 comprising the amino acid sequence of SEQ ID NO: 85, and the LC-CDR3 comprising the amino acid sequence of SEQ ID NO: 86; j) the V_{H} comprises the HC-CDR1 comprising the amino acid sequence of SEQ ID NO: 91, the HC-CDR2 comprising the amino acid sequence of SEQ ID NO: 92, and the HC-CDR3 comprising the amino acid sequence of SEQ ID NO: 93, and the V_{L} comprises the LC-CDR1 comprising the amino acid sequence of SEQ ID NO: 94, the LC-CDR2 comprising the amino acid sequence of SEQ ID NO: 95, and the LC-CDR3 comprising the amino acid sequence of SEQ ID NO: 96; k) the V_{H} comprises the HC-CDR1 comprising the amino acid sequence of SEQ ID NO: 101, the HC-CDR2 comprising the amino acid sequence of SEQ ID NO: 102, and the HC-CDR3 comprising the amino acid sequence of SEQ ID NO: 103, and the V_{L} comprises the LC-CDR1 comprising the amino acid sequence of SEQ ID NO: 104, the LC-CDR2 comprising the amino acid sequence of SEQ ID NO: 105, and the LC-CDR3 comprising the amino acid sequence of SEQ ID NO:106; l) the V_{H} comprises the HC-CDR1 comprising the amino acid sequence of SEQ ID NO: 111, the HC-CDR2 comprising the amino acid sequence of SEQ ID NO: 112, and the HC-CDR3 comprising the amino acid sequence of SEQ ID NO: 113, and the V_{L} comprises the LC-CDR1 comprising the amino acid sequence of SEQ ID NO: 114, the LC-CDR2 comprising the amino acid sequence of SEQ ID NO: 115, and the LC-CDR3 comprising the amino acid sequence of SEQ ID NO: 116; m) the V_{H} comprises the HC-CDR1 comprising the amino acid sequence of SEQ ID NO: 121, the HC-CDR2 comprising the amino acid sequence of SEQ ID NO: 122, and the HC-CDR3 comprising the amino acid sequence of SEQ ID NO: 123, and the V_{L} comprises the LC-CDR1 comprising the amino acid sequence of SEQ ID NO: 124, the LC-CDR2 comprising the amino acid sequence of SEQ ID NO: 125, and the LC-CDR3 comprising the amino acid sequence of SEQ ID NO: 126; n) the V_{H} comprises the HC-CDR1 comprising the amino acid sequence of SEQ ID NO: 131, the HC-CDR2 comprising the amino acid sequence of SEQ ID NO: 132, and the HC-CDR3 comprising the amino acid sequence of SEQ ID NO: 133, and the V_{L} comprises the LC-CDR1 comprising the amino acid sequence of SEQ ID NO: 134, the LC-CDR2 comprising the amino acid sequence of SEQ ID NO: 135, and the LC-CDR3 comprising the amino acid sequence of SEQ ID NO: 136; o) the V_{H} comprises the HC-CDR1 comprising the amino acid sequence of SEQ ID NO: 211, the HC-CDR2 comprising the amino acid sequence of SEQ ID NO: 212, and the HC-CDR3 comprising the amino acid sequence of SEQ ID NO: 213, and the V_{L} comprises the LC-CDR1 comprising the amino acid sequence of SEQ ID NO: 214, the LC-CDR2 comprising the amino acid sequence of SEQ ID NO: 215, and the LC-CDR3 comprising the amino acid sequence of SEQ ID NO: 216; and p) the V_{H} comprises the HC-CDR1 comprising the amino acid sequence of SEQ ID NO: 221, the HC-CDR2 comprising the amino acid sequence of SEQ ID NO: 222, and the HC-CDR3 comprising the amino acid sequence of SEQ ID NO: 223, and the V_{L} comprises the LC-CDR1 comprising the amino acid sequence of SEQ ID NO: 224, the LC-CDR2 comprising the amino acid sequence of SEQ ID NO: 225, and the LC-CDR3 comprising the amino acid sequence of SEQ ID NO: 226.

In some embodiments according to any of the anti-CD137 constructs described herein, the construct comprises an anti-CD137 antibody moiety comprising a heavy chain variable region (V_{H}) that comprises HC-CDR1, HC-CDR2, and HC-CDR3 domains; and a light chain variable region (V_{L}) that comprises LC-CDR1, LC-CDR2, and LC-CDR3 domains, wherein the V_{H} and the V_{L} are selected from the group consisting of: a) the V_{H} comprises the HC-CDR1 comprising the amino acid sequence of SEQ ID NO: 1, the HC-CDR2 comprising the amino acid sequence of SEQ ID NO: 2, and the HC -CDR3 comprising the amino acid sequence of SEQ ID NO: 3, and the V_{L} comprises the LC-CDR1 comprising the amino acid sequence of SEQ ID NO: 4, the LC-CDR2 comprising the amino acid sequence of SEQ ID NO: 5, and the LC-CDR3 comprising the amino acid sequence of SEQ ID NO: 6; b) the V_{H} comprises the HC-CDR1 comprising the amino acid sequence of SEQ ID NO: 11, the HC-CDR2 comprising the amino acid sequence of SEQ ID NO: 12, and the HC-CDR3 comprising the amino acid sequence of SEQ ID NO: 13, and the V_{L} comprises the LC-CDR1 comprising the amino acid sequence of SEQ ID NO: 14, the LC-CDR2 comprising the amino acid sequence of SEQ ID NO: 15, and the LC-CDR3 comprising the amino acid sequence of SEQ ID NO: 16; c) the V_{H} comprises the HC-CDR1 comprising the amino acid sequence of SEQ ID NO: 21, the HC-CDR2 comprising the amino acid sequence of SEQ ID NO: 22, and the HC-CDR3 comprising the amino acid sequence of SEQ ID NO: 23, and the V_{L} comprises the LC-CDR1 comprising the amino acid sequence of SEQ ID NO: 24, the LC-CDR2 comprising the amino acid sequence of SEQ ID NO: 25, and the LC-CDR3 comprising the amino acid sequence of SEQ ID NO: 26; d) the V_{H} comprises the HC-CDR1 comprising the amino acid sequence of SEQ ID NO: 31, the HC-CDR2 comprising the amino acid sequence of SEQ ID NO: 32, and the HC-CDR3 comprising the amino acid sequence of SEQ ID NO: 33, and the V_{L} comprises the LC-CDR1 comprising the amino acid sequence of SEQ ID NO: 34, the LC-CDR2 comprising the amino acid sequence of SEQ ID NO: 35, and the LC-CDR3 comprising the amino acid sequence of SEQ ID NO: 36; e) the V_{H} comprises the HC-CDR1 comprising the amino acid sequence of SEQ ID NO: 41, the HC-CDR2 comprising the amino acid sequence of SEQ ID NO: 42, and the HC-CDR3 comprising the amino acid sequence of SEQ ID NO: 43, and the V_{L} comprises the LC-CDR1 comprising the amino acid sequence of SEQ ID NO: 44, the LC-CDR2 comprising the amino acid sequence of SEQ ID NO: 45, and the LC-CDR3 comprising the amino acid sequence of SEQ ID NO: 46; f) the V_{H} comprises the HC-CDR1 comprising the amino acid sequence of SEQ ID NO: 51, the HC-CDR2 comprising the amino acid sequence of SEQ ID NO: 52, and the HC-CDR3 comprising the amino acid sequence of SEQ ID NO: 53, and the V_{L} comprises the LC-CDR1 comprising the amino acid sequence of SEQ ID NO: 54, the LC-CDR2 comprising the amino acid sequence of SEQ ID NO: 55, and the LC-CDR3 comprising the amino acid sequence of SEQ ID NO: 56; g) the V_{H} comprises the HC-CDR1 comprising the amino acid sequence of SEQ ID NO: 61, the HC-CDR2 comprising the amino acid sequence of SEQ ID NO: 62, and the HC-CDR3 comprising the amino acid sequence of SEQ ID NO: 63, and the V_{L} comprises the LC-CDR1 comprising the amino acid sequence of SEQ ID NO: 64, the LC-CDR2 comprising the amino acid sequence of SEQ ID NO: 65, and the LC-CDR3 comprising the amino acid sequence of SEQ ID NO: 66; h) the V_{H} comprises the HC-CDR1 comprising the amino acid sequence of SEQ ID NO: 71, the HC-CDR2 comprising the amino acid sequence of SEQ ID NO: 72, and the HC-CDR3 comprising the amino acid sequence of SEQ ID NO: 73, and the V_{L} comprises the LC-CDR1 comprising the amino acid sequence of SEQ ID NO: 74, the LC-CDR2 comprising the amino acid sequence of SEQ ID NO: 75, and the LC-CDR3 comprising the amino acid sequence of SEQ ID NO: 76; i) the V_{H} comprises the HC-CDR1 comprising the amino acid sequence of SEQ ID NO: 81, the HC-CDR2 comprising the amino acid sequence of SEQ ID NO: 82, and the HC-CDR3 comprising the amino acid sequence of SEQ ID NO: 83, and the V_{L} comprises the LC-CDR1 comprising the amino acid sequence of SEQ ID NO: 84, the LC-CDR2 comprising the amino acid sequence of SEQ ID NO: 85, and the LC-CDR3 comprising the amino acid sequence of SEQ ID NO: 86; j) the V_{H} comprises the HC-CDR1 comprising the amino acid sequence of SEQ ID NO: 91, the HC-CDR2 comprising the amino acid sequence of SEQ ID NO: 92, and the HC-CDR3 comprising the amino acid sequence of SEQ ID NO: 93, and the V_{L} comprises the LC-CDR1 comprising the amino acid sequence of SEQ ID NO: 94, the LC-CDR2 comprising the amino acid sequence of SEQ ID NO: 95, and the LC-CDR3 comprising the amino acid sequence of SEQ ID NO: 96; k) the V_{H} comprises the HC-CDR1 comprising the amino acid sequence of SEQ ID NO: 101, the HC-CDR2 comprising the amino acid sequence of SEQ ID NO: 102, and the HC-CDR3 comprising the amino acid sequence of SEQ ID NO: 103, and the V_{L} comprises the LC-CDR1 comprising the amino acid sequence of SEQ ID NO: 104, the LC-CDR2 comprising the amino acid sequence of SEQ ID NO: 105, and the LC-CDR3 comprising the amino acid sequence of SEQ ID NO:106; l) the V_{H} comprises the HC-CDR1 comprising the amino acid sequence of SEQ ID NO: 111, the HC-CDR2 comprising the amino acid sequence of SEQ ID NO: 112, and the HC-CDR3 comprising the amino acid sequence of SEQ ID NO: 113, and the V_{L} comprises the LC-CDR1 comprising the amino acid sequence of SEQ ID NO: 114, the LC-CDR2 comprising the amino acid sequence of SEQ ID NO: 115, and the LC-CDR3 comprising the amino acid sequence of SEQ ID NO: 116; m) the V_{H} comprises the HC-CDR1 comprising the amino acid sequence of SEQ ID NO: 121, the HC-CDR2 comprising the amino acid sequence of SEQ ID NO: 122, and the HC-CDR3 comprising the amino acid sequence of SEQ ID NO: 123, and the V_{L} comprises the LC-CDR1 comprising the amino acid sequence of SEQ ID NO: 124, the LC-CDR2 comprising the amino acid sequence of SEQ ID NO: 125, and the LC-CDR3 comprising the amino acid sequence of SEQ ID NO: 126; n) the V_{H} comprises the HC-CDR1 comprising the amino acid sequence of SEQ ID NO: 131, the HC-CDR2 comprising the amino acid sequence of SEQ ID NO: 132, and the HC-CDR3 comprising the amino acid sequence of SEQ ID NO: 133, and the V_{L} comprises the LC-CDR1 comprising the amino acid sequence of SEQ ID NO: 134, the LC-CDR2 comprising the amino acid sequence of SEQ ID NO: 135, and the LC-CDR3 comprising the amino acid sequence of SEQ ID NO: 136; o) the V_{H} comprises the HC-CDR1 comprising the amino acid sequence of SEQ ID NO: 211, the HC-CDR2 comprising the amino acid sequence of SEQ ID NO: 212, and the HC-CDR3 comprising the amino acid sequence of SEQ ID NO: 213, and the V_{L} comprises the LC-CDR1 comprising the amino acid sequence of SEQ ID NO: 214, the LC-CDR2 comprising the amino acid sequence of SEQ ID NO: 215, and the LC-CDR3 comprising the amino acid sequence of SEQ ID NO: 216; and p) the V_{H} comprises the HC-CDR1 comprising the amino acid sequence of SEQ ID NO: 221, the HC-CDR2 comprising the amino acid sequence of SEQ ID NO: 222, and the HC-CDR3 comprising the amino acid sequence of SEQ ID NO: 223, and the V_{L} comprises the LC-CDR1 comprising the amino acid sequence of SEQ ID NO: 224, the LC-CDR2 comprising the amino acid sequence of SEQ ID NO: 225, and the LC-CDR3 comprising the amino acid sequence of SEQ ID NO: 226.

In some embodiments according to any one of the anti-CD 137 constructs described herein, the V_{H} comprises the HC-CDR1 comprising the amino acid sequence of SEQ ID NO: 1, the HC-CDR2 comprising the amino acid sequence of SEQ ID NO: 2, and the HC-CDR3 comprising the amino acid sequence of SEQ ID NO: 3, and the V_{L} comprises the LC-CDR1 comprising the amino acid sequence of SEQ ID NO: 4, the LC-CDR2 comprising the amino acid sequence of SEQ ID NO: 5, and the LC-CDR3 comprising the amino acid sequence of SEQ ID NO: 6.

In some embodiments according to any one of the anti-CD 137 constructs described herein, the V_{H} comprises the HC-CDR1 comprising the amino acid sequence of SEQ ID NO: 11, the HC-CDR2 comprising the amino acid sequence of SEQ ID NO: 12, and the HC-CDR3 comprising the amino acid sequence of SEQ ID NO: 13, and the V_{L} comprises the LC-CDR1 comprising the amino acid sequence of SEQ ID NO: 14, the LC-CDR2 comprising the amino acid sequence of SEQ ID NO: 15, and the LC-CDR3 comprising the amino acid sequence of SEQ ID NO: 16.

In some embodiments according to any one of the anti-CD 137 constructs described herein, the V_{H} comprises the HC-CDR1 comprising the amino acid sequence of SEQ ID NO: 21, the HC-CDR2 comprising the amino acid sequence of SEQ ID NO: 22, and the HC-CDR3 comprising the amino acid sequence of SEQ ID NO: 23, and the V_{L} comprises the LC-CDR1 comprising the amino acid sequence of SEQ ID NO: 24, the LC-CDR2 comprising the amino acid sequence of SEQ ID NO: 25, and the LC-CDR3 comprising the amino acid sequence of SEQ ID NO: 26.

In some embodiments according to any one of the anti-CD 137 constructs described herein, the V_{H} comprises the HC-CDR1 comprising the amino acid sequence of SEQ ID NO: 31, the HC-CDR2 comprising the amino acid sequence of SEQ ID NO: 32, and the HC-CDR3 comprising the amino acid sequence of SEQ ID NO: 33, and the V_{L} comprises the LC-CDR1 comprising the amino acid sequence of SEQ ID NO: 34, the LC-CDR2 comprising the amino acid sequence of SEQ ID NO: 35, and the LC-CDR3 comprising the amino acid sequence of SEQ ID NO: 36.

In some embodiments according to any one of the anti-CD 137 constructs described herein, the V_{H} comprises the HC-CDR1 comprising the amino acid sequence of SEQ ID NO: 41, the HC-CDR2 comprising the amino acid sequence of SEQ ID NO: 42, and the HC-CDR3 comprising the amino acid sequence of SEQ ID NO: 43, and the V_{L} comprises the LC-CDR1 comprising the amino acid sequence of SEQ ID NO: 44, the LC-CDR2 comprising the amino acid sequence of SEQ ID NO: 45, and the LC-CDR3 comprising the amino acid sequence of SEQ ID NO: 46.

In some embodiments according to any one of the anti-CD 137 constructs described herein, the V_{H} comprises the HC-CDR1 comprising the amino acid sequence of SEQ ID NO: 51, the HC-CDR2 comprising the amino acid sequence of SEQ ID NO: 52, and the HC-CDR3 comprising the amino acid sequence of SEQ ID NO: 53, and the V_{L} comprises the LC-CDR1 comprising the amino acid sequence of SEQ ID NO: 54, the LC-CDR2 comprising the amino acid sequence of SEQ ID NO: 55, and the LC-CDR3 comprising the amino acid sequence of SEQ ID NO: 56.

In some embodiments according to any one of the anti-CD 137 constructs described herein, the V_{H} comprises the HC-CDR1 comprising the amino acid sequence of SEQ ID NO: 61, the HC-CDR2 comprising the amino acid sequence of SEQ ID NO: 62, and the HC-CDR3 comprising the amino acid sequence of SEQ ID NO: 63, and the V_{L} comprises the LC-CDR1 comprising the amino acid sequence of SEQ ID NO: 64, the LC-CDR2 comprising the amino acid sequence of SEQ ID NO: 65, and the LC-CDR3 comprising the amino acid sequence of SEQ ID NO: 66.

In some embodiments according to any one of the anti-CD 137 constructs described herein, the V_{H} comprises the HC-CDR1 comprising the amino acid sequence of SEQ ID NO: 71, the HC-CDR2 comprising the amino acid sequence of SEQ ID NO: 72, and the HC-CDR3 comprising the amino acid sequence of SEQ ID NO: 73, and the V_{L} comprises the LC-CDR1 comprising the amino acid sequence of SEQ ID NO: 74, the LC-CDR2 comprising the amino acid sequence of SEQ ID NO: 75, and the LC-CDR3 comprising the amino acid sequence of SEQ ID NO: 76.

In some embodiments according to any one of the anti-CD137 constructs described herein, the V_{H} comprises the HC-CDR1 comprising the amino acid sequence of SEQ ID NO: 81, the HC-CDR2 comprising the amino acid sequence of SEQ ID NO: 82, and the HC-CDR3 comprising the amino acid sequence of SEQ ID NO: 83, and the V_{L} comprises the LC-CDR1 comprising the amino acid sequence of SEQ ID NO: 84, the LC-CDR2 comprising the amino acid sequence of SEQ ID NO: 85, and the LC-CDR3 comprising the amino acid sequence of SEQ ID NO: 86.

In some embodiments according to any one of the anti-CD 137 constructs described herein, the V_{H} comprises the HC-CDR1 comprising the amino acid sequence of SEQ ID NO: 91, the HC-CDR2 comprising the amino acid sequence of SEQ ID NO: 92, and the HC-CDR3 comprising the amino acid sequence of SEQ ID NO: 93, and the V_{L} comprises the LC-CDR1 comprising the amino acid sequence of SEQ ID NO: 94, the LC-CDR2 comprising the amino acid sequence of SEQ ID NO: 95, and the LC-CDR3 comprising the amino acid sequence of SEQ ID NO: 96.

In some embodiments according to any one of the anti-CD 137 constructs described herein, the V_{H} comprises the HC-CDR1 comprising the amino acid sequence of SEQ ID NO: 101, the HC-CDR2 comprising the amino acid sequence of SEQ ID NO: 102, and the HC-CDR3 comprising the amino acid sequence of SEQ ID NO: 103, and the V_{L} comprises the LC-CDR1 comprising the amino acid sequence of SEQ ID NO: 104, the LC-CDR2 comprising the amino acid sequence of SEQ ID NO: 105, and the LC-CDR3 comprising the amino acid sequence of SEQ ID NO:106.

In some embodiments according to any one of the anti-CD 137 constructs described herein, the V_{H} comprises the HC-CDR1 comprising the amino acid sequence of SEQ ID NO: 111, the HC-CDR2 comprising the amino acid sequence of SEQ ID NO: 112, and the HC-CDR3 comprising the amino acid sequence of SEQ ID NO: 113, and the V_{L} comprises the LC-CDR1 comprising the amino acid sequence of SEQ ID NO: 114, the LC-CDR2 comprising the amino acid sequence of SEQ ID NO: 115, and the LC-CDR3 comprising the amino acid sequence of SEQ ID NO: 116.

In some embodiments according to any one of the anti-CD 137 constructs described herein, the V_{H} comprises the HC-CDR1 comprising the amino acid sequence of SEQ ID NO: 121, the HC-CDR2 comprising the amino acid sequence of SEQ ID NO: 122, and the HC-CDR3 comprising the amino acid sequence of SEQ ID NO: 123, and the V_{L} comprises the LC-CDR1 comprising the amino acid sequence of SEQ ID NO: 124, the LC-CDR2 comprising the amino acid sequence of SEQ ID NO: 125, and the LC-CDR3 comprising the amino acid sequence of SEQ ID NO: 126.

In some embodiments according to any one of the anti-CD 137 constructs described herein, the V_{H} comprises the HC-CDR1 comprising the amino acid sequence of SEQ ID NO: 131, the HC-CDR2 comprising the amino acid sequence of SEQ ID NO: 132, and the HC-CDR3 comprising the amino acid sequence of SEQ ID NO: 133, and the V_{L} comprises the LC-CDR1 comprising the amino acid sequence of SEQ ID NO: 134, the LC-CDR2 comprising the amino acid sequence of SEQ ID NO: 135, and the LC-CDR3 comprising the amino acid sequence of SEQ ID NO: 136.

In some embodiments according to any one of the anti-CD 137 constructs described herein, the V_{H} comprises the HC-CDR1 comprising the amino acid sequence of SEQ ID NO: 141, the HC-CDR2 comprising the amino acid sequence of SEQ ID NO: 142, and the HC-CDR3 comprising the amino acid sequence of SEQ ID NO: 143, and the V_{L} comprises the LC-CDR1 comprising the amino acid sequence of SEQ ID NO: 144, the LC-CDR2 comprising the amino acid sequence of SEQ ID NO: 145, and the LC-CDR3 comprising the amino acid sequence of SEQ ID NO: 146.

In some embodiments according to any one of the anti-CD 137 constructs described herein, the V_{H} comprises the HC-CDR1 comprising the amino acid sequence of SEQ ID NO: 211, the HC-CDR2 comprising the amino acid sequence of SEQ ID NO: 212, and the HC-CDR3 comprising the amino acid sequence of SEQ ID NO: 213, and the V_{L} comprises the LC-CDR1 comprising the amino acid sequence of SEQ ID NO: 214, the LC-CDR2 comprising the amino acid sequence of SEQ ID NO: 215, and the LC-CDR3 comprising the amino acid sequence of SEQ ID NO: 216.

In some embodiments according to any one of the anti-CD 137 constructs described herein, the V_{H} comprises the HC-CDR1 comprising the amino acid sequence of SEQ ID NO: 221, the HC-CDR2 comprising the amino acid sequence of SEQ ID NO: 222, and the HC-CDR3 comprising the amino acid sequence of SEQ ID NO: 223, and the V_{L} comprises the LC-CDR1 comprising the amino acid sequence of SEQ ID NO: 224, the LC-CDR2 comprising the amino acid sequence of SEQ ID NO: 225, and the LC-CDR3 comprising the amino acid sequence of SEQ ID NO: 226.

Another aspect of the present application provides isolated anti-CD137 constructs comprising an antibody moiety that binds to CD137, comprising: a) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 7; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 8; b) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 17; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 18; c) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 27; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 28; d) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 37; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 38; e) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 47; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 48; f) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 57; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 58; g) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 67; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 68; h) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 77; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 78; i) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 87; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 88; j) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 97; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 98; k) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 107; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 108; l) a HC-CDR1, a HC-CDR2, and a HC-CDR3, respectively comprising the amino acid sequences of a CDR1, a CDR2, and a CDR3 within a V_{H} chain region having the sequence set forth in SEQ ID NO: 117; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 118; m) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 127; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 128; n) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 137; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 138; o) a HC-CDR1, a HC-CDR2, and a HC-CDR3, respectively comprising the amino acid sequences of a CDR1, a CDR2, and a CDR3 within a VH chain region having the sequence set forth in SEQ ID No: 217; and a LC-CDR1, a LC-CDR2, and a LC-CDR3, respectively comprising the amino acid sequences of a CDR1, a CDR2, and a CDR3 within a VL chain region having the sequence set forth in SEQ ID No: 218; or p) a HC-CDR1, a HC-CDR2, and a HC-CDR3, respectively comprising the amino acid sequences of a CDR1, a CDR2, and a CDR3 within a VH chain region having the sequence set forth in SEQ ID No: 227; and a LC-CDR1, a LC-CDR2, and a LC-CDR3, respectively comprising the amino acid sequences of a CDR1, a CDR2, and a CDR3 within a VL chain region having the sequence set forth in SEQ ID No: 228.

In some embodiments according to any one of the anti-CD 137 constructs described herein, the construct comprises an anti-CD137 antibody moiety comprising (a) a heavy chain variable region comprising amino acids having the sequence set forth in SEQ ID NO: 7; and a light chain variable region comprising amino acids having the sequence set forth in SEQ ID NO: 8; (b) a heavy chain variable region comprising amino acids having the sequence set forth in SEQ ID NO: 17; and a light chain variable region comprising amino acids having the sequence set forth in SEQ ID NO: 18; (c) a heavy chain variable region comprising amino acids having the sequence set forth in SEQ ID NO: 27; and a light chain variable region comprising amino acids having the sequence set forth in SEQ ID NO: 28; (d) a heavy chain variable region comprising amino acids having the sequence set forth in SEQ ID NO: 37; and a light chain variable region comprising amino acids having the sequence set forth in SEQ ID NO: 38; (e) a heavy chain variable region comprising amino acids having the sequence set forth in SEQ ID NO: 47; and a light chain variable region comprising amino acids having the sequence set forth in SEQ ID NO: 48; (f) a heavy chain variable region comprising amino acids having the sequence set forth in SEQ ID NO: 57; and a light chain variable region comprising amino acids having the sequence set forth in SEQ ID NO: 58; (g) a heavy chain variable region comprising amino acids having the sequence set forth in SEQ ID NO: 67; and a light chain variable region comprising amino acids having the sequence set forth in SEQ ID NO: 68; (h) a heavy chain variable region comprising amino acids having the sequence set forth in SEQ ID NO: 77; and a light chain variable region comprising amino acids having the sequence set forth in SEQ ID NO: 78; (i) a heavy chain variable region comprising amino acids having the sequence set forth in SEQ ID NO: 87; and a light chain variable region comprising amino acids having the sequence set forth in SEQ ID NO: 88; (j) a heavy chain variable region comprising amino acids having the sequence set forth in SEQ ID NO: 97; and a light chain variable region comprising amino acids having the sequence set forth in SEQ ID NO: 98; (k) a heavy chain variable region comprising amino acids having the sequence set forth in SEQ ID NO: 107; and a light chain variable region comprising amino acids having the sequence set forth in SEQ ID NO: 108; (l) a heavy chain variable region comprising amino acids having the sequence set forth in SEQ ID NO: 117; and a light chain variable region comprising amino acids having the sequence set forth in SEQ ID NO: 118; (m) a heavy chain variable region comprising amino acids having the sequence set forth in SEQ ID NO: 127; and a light chain variable region comprising amino acids having the sequence set forth in SEQ ID NO: 128; (n) a heavy chain variable region comprising amino acids having the sequence set forth in SEQ ID NO: 137; and a light chain variable region comprising amino acids having the sequence set forth in SEQ ID NO: 138; (o) a heavy chain variable region comprising amino acids having the sequence set forth in SEQ ID NO: 217; and a light chain variable region comprising amino acids having the sequence set forth in SEQ ID NO: 218; or (p) a heavy chain variable region comprising amino acids having the sequence set forth in SEQ ID NO: 227; and a light chain variable region comprising amino acids having the sequence set forth in SEQ ID NO: 228.

Another aspect of the present application provides isolated anti-CD137 constructs comprising an anti-CD137 antibody moiety that binds to CD137, comprising a heavy chain variable region (V_{H}) and a light chain variable region (V_{L}), wherein: a) the V_{H} comprises: i) an HC-CDR1 comprising an amino acid sequence of any one of SEQ ID NOs: 151-153, or a variant thereof comprising up to about 3 amino acid substitutions; ii) an HC-CDR2 comprising an amino acid sequence of any one of SEQ ID NOs: 154-156, or a variant thereof comprising up to about 3 amino acid substitutions; iii) an HC-CDR3 comprising an amino acid sequence of any one of SEQ ID NOs: 157-159, or a variant thereof comprising up to about 3 amino acid substitutions; and b) the V_{L} comprises: i) an LC-CDR1 comprising an amino acid sequence of any one of SEQ ID NOs: 160-163, or a variant thereof comprising up to about 3 amino acid substitutions; ii) an HC-CDR2 comprising an amino acid sequence of any one of SEQ ID NOs: 164-166, or a variant thereof comprising up to about 3 amino acid substitutions; iii) an HC-CDR3 comprising an amino acid sequence of any one of SEQ ID NOs: 167-169, or a variant thereof comprising up to about 3 amino acid substitutions.

In some embodiments, the V_{H} comprises the HC-CDR1 comprising the amino acid sequence of SEQ ID NO: 151, an HC-CDR2 comprising the amino acid sequence of SEQ ID NO: 154, and the HC-CDR3 comprising the amino acid sequence of SEQ ID NO: 157, and the V_{L} comprises the LC-CDR1 comprising the amino acid sequence of SEQ ID NO: 160, an LC-CDR2 comprising the amino acid sequence of SEQ ID NO: 164, and the LC-CDR3 comprising the amino acid sequence of SEQ ID NO: 167.

In some embodiments, the V_{H} comprises the HC-CDR1 comprising the amino acid sequence of SEQ ID NO: 151, an HC-CDR2 comprising the amino acid sequence of SEQ ID NO: 154, and the HC-CDR3 comprising the amino acid sequence of SEQ ID NO: 157, and the V_{L} comprises the LC-CDR1 comprising the amino acid sequence of SEQ ID NO: 162, an LC-CDR2 comprising the amino acid sequence of SEQ ID NO: 166, and the LC-CDR3 comprising the amino acid sequence of SEQ ID NO: 169.

In some embodiments, the V_{H} comprises the HC-CDR1 comprising the amino acid sequence of SEQ ID NO: 152, an HC-CDR2 comprising the amino acid sequence of SEQ ID NO: 155, and the HC-CDR3 comprising the amino acid sequence of SEQ ID NO: 158, and the V_{L} comprises the LC-CDR1 comprising the amino acid sequence of SEQ ID NO: 163, an LC-CDR2 comprising the amino acid sequence of SEQ ID NO: 166, and the LC-CDR3 comprising the amino acid sequence of SEQ ID NO: 169,.

In some embodiments, the V_{H} comprises the HC-CDR1 comprising the amino acid sequence of SEQ ID NO: 153, an HC-CDR2 comprising the amino acid sequence of SEQ ID NO: 156, and the HC-CDR3 comprising the amino acid sequence of SEQ ID NO: 159, and the V_{L} comprises the LC-CDR1 comprising the amino acid sequence of SEQ ID NO: 160, an LC-CDR2 comprising the amino acid sequence of SEQ ID NO: 164, and the LC-CDR3 comprising the amino acid sequence of SEQ ID NO: 167.

In some embodiments, the V_{H} comprises the HC-CDR1 comprising the amino acid sequence of SEQ ID NO: 153, an HC-CDR2 comprising the amino acid sequence of SEQ ID NO: 156, and the HC-CDR3 comprising the amino acid sequence of SEQ ID NO: 159, and the V_{L} comprises the LC-CDR1 comprising the amino acid sequence of SEQ ID NO: 161, an LC-CDR2 comprising the amino acid sequence of SEQ ID NO: 165, and the LC-CDR3 comprising the amino acid sequence of SEQ ID NO: 168.

In some embodiments according to any one of the anti-CD 137 constructs described herein, the construct comprises an antibody or antigen-binding fragment thereof selected from the group consisting of a full-length antibody, a multispecific antibody (e.g., a bispecific antibody), a single-chain Fv (scFv), a Fab fragment, a Fab' fragment, a F(ab')2, an Fv fragment, a disulfide stabilized Fv fragment (dsFv), a (dsFv)₂, a V_{H}H, a Fv-Fc fusion, a scFv-Fc fusion, a scFv-Fv fusion, a diabody, a tribody, and a tetrabody. In some embodiments, the construct comprises a humanized anti-CD137 full-length antibody. In some embodiments, the construct comprises a humanized anti-CD137 single chain Fv fragment.

In some embodiments according to any one of the anti-CD 137 constructs described herein, the anti-CD137 antibody moiety is a CD137 agonist antibody.

In some embodiments according to any one of the anti-CD 137 constructs described herein, the construct further comprises a Fc fragment of a human immunoglobulin.

In some embodiments according to any one of the anti-CD 137 constructs described herein, the Fc fragment is selected from the group consisting of Fc fragments of IgG, IgA, IgD, IgE, and IgM.

In some embodiments according to any one of the anti-CD 137 constructs described herein, the Fc fragment is selected from the group consisting of Fc fragments of IgG1, IgG2, IgG3 and IgG4. In some embodiments, the Fc fragment comprises an IgG1 Fc fragment. In some embodiments, the Fc fragment comprises an IgG2 Fc fragment. In some embodiments, the Fc fragment comprises an IgG4 Fc fragment.

In some embodiments according to any one of the anti-CD 137 constructs described herein, the antibody moiety comprises an IgG1 Fc fragment comprising one or more mutation selected from the group consisting of S267E, L328F and combination thereof.

In some embodiments according to any one of the anti-CD 137 constructs described herein, the antibody moiety comprises an IgG2 Fc fragment comprising one or more mutation selected from the group consisting of S267E, L328F and combination thereof.

In some embodiments according to any one of the anti-CD 137 constructs described herein, the antibody moiety comprises an IgG4 Fc fragment comprising a S228P mutation.

In some embodiments according to any one of the anti-CD 137 constructs described herein, the antibody moiety binds to a human CD137 and a simian CD137. In some embodiments according to any one of the anti-CD137 constructs described herein, the antibody moiety does not bind to a murine CD137.

In some embodiments according to any one of the anti-CD 137 constructs described herein, the construct is a multispecific antibody. In some embodiments according to any one of the anti-CD137 constructs described herein, the construct further comprises a second antibody moiety that binds to a second antigen, wherein the second antibody moiety comprises a second heavy chain variable region (V_{H-2}) and a second light chain variable region (V_{L-2}). In some embodiments, the second antigen is a tumor associated antigen.

In some embodiments, the tumor associated antigen is selected from the group consisting of Her-2, EGFR, PD-L1, c-Met, B Cell Maturation Antigen (BCMA), carbonic anhydrase IX (CA1X), carcinoembryonic antigen (CEA), CD5, CD7, CD10, CD19, CD20, CD22, CD30, CD33, CD34, CD38, CD41, CD44, CD49f, CD56, CD74, CD123, CD133, CD138, CD276 (B7H3), epithelial glycoprotein (EGP2), trophoblast cell-surface antigen 2 (TROP-2), epithelial glycoprotein- 40 (EGP-40), epithelial cell adhesion molecule (EpCAM), receptor tyrosine-protein kinases erb-B2,3,4, folate-binding protein (FBP), fetal acetylcholine receptor (AChR), folate receptor-a, Ganglioside G2 (GD2), Ganglioside G3 (GD3), human telomerase reverse transcriptase (hTERT), kinase insert domain receptor (KDR), Lewis A (CA 1.9.9), Lewis Y (LeY), Glypican-3 (GPC3), L1 cell adhesion molecule (L1CAM), Mucin 16 (Muc-16), Mucin 1 (Muc-1), NG2D ligands, oncofetal antigen (h5T4), prostate stem cell antigen (PSCA), prostate-specific membrane antigen (PSMA), tumor- associated glycoprotein 72 (TAG-72), Claudin18.2 (CLDN18.2), vascular endothelial growth factor R2 (VEGF- R2), Wilms tumor protein (WT-1), type 1 tyrosine-protein kinase transmembrane receptor (ROR1) and any combination thereof.

Another aspect of the present application provides immunoconjugate comprising any one of the anti-CD137 constructs described herein, linked to a therapeutic agent or a label. In some embodiments, the label is selected from the group consisting of a radioisotope, a fluorescent dye and an enzyme.

Another aspect of the present application provides pharmaceutical compositions comprising any one of the anti-CD137 constructs or the immunoconjugates described herein and a pharmaceutically acceptable carrier.

Another aspect of the present application provides isolated nucleic acids encoding any one of the anti-CD137 constructs described herein or a portion thereof.

Another aspect of the present application provides vectors comprising any one of the isolated nucleic acid described herein.

Another aspect of the present application provides isolated host cells comprising any one of the isolated nucleic acids or the vectors described herein.

Another aspect of the present application provides methods of producing an anti-CD137 construct or a multispecific antibody, comprising: a) culturing any one of the isolated host cells described herein under conditions effective to express the anti-CD137 construct; and b) obtaining the expressed anti-CD137 construct or multispecific antibody from the host cell.

Another aspect of the present application provides methods of treating or preventing a disease in an individual, comprising administering to the individual an effective amount of any one of a) the anti-CD137 constructs, b) the multispecific antibodies, or c) the pharmaceutical compositions described herein. In some embodiments, the disease is a cancer or a tumor. In some embodiments, the cancer is selected from the group consisting of breast cancer, gastric cancer, ovarian cancer, lung cancer, mesothelioma, endometrial cancer, cervical cancer, esophageal cancer, bladder cancer, salivary gland cancer, testicular cancer, renal cancer, liver cancer, pancreatic cancer, colorectal cancer, skin cancer, thymus cancer, adrenal cancer, head and neck cancer, brain cancer, thyroid cancer, sarcoma, myeloma and leukemia. In some embodiments, the cancer is breast cancer or gastric cancer. In some embodiments, the cancer is lung cancer, colorectal cancer or head and neck cancer.

In some embodiments, any one of the anti-CD137 construct, the multispecific antibody or the pharmaceutical composition is administered parenterally into the individual. In some embodiments, the multispecific antibody or the pharmaceutical composition is administered intravenously into the individual. In some embodiments, the individual is a human.

Another aspect of the present application provides any one of the anti-CD137 constructs described herein for use as a medicament.

Another aspect of the present application provides any one of the anti-CD137 constructs described herein for use in treating cancer. In some embodiments, the cancer is selected from the group consisting of breast cancer, gastric cancer, ovarian cancer, lung cancer, mesothelioma, endometrial cancer, cervical cancer, esophageal cancer, bladder cancer, salivary gland cancer, testicular cancer, renal cancer, liver cancer, pancreatic cancer, colorectal cancer, skin cancer, thymus cancer, adrenal cancer, head and neck cancer, brain cancer, thyroid cancer, sarcoma, myeloma and leukemia.

Another aspect of the present application provides kits comprising any one of a) the anti-CD137 constructs, b) the multispecific antibodies, c) the immunoconjugates, d) the pharmaceutical compositions, e) the nucleic acids, f) the vectors or g) the host cells described herein. In some embodiments, the kit further comprising a written instruction for treating and/or preventing a cancer or a tumor.

### BRIEF DESCRIPTION OF THE DRAWINGS

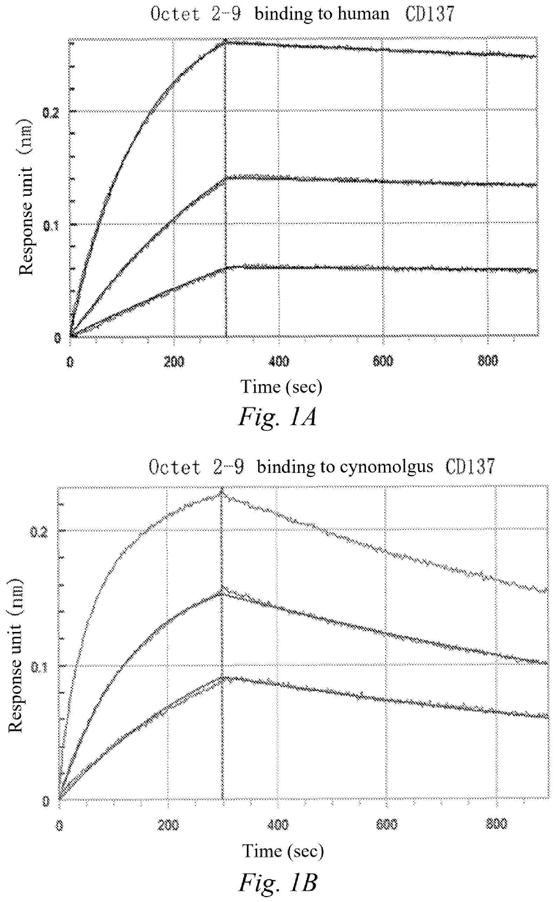

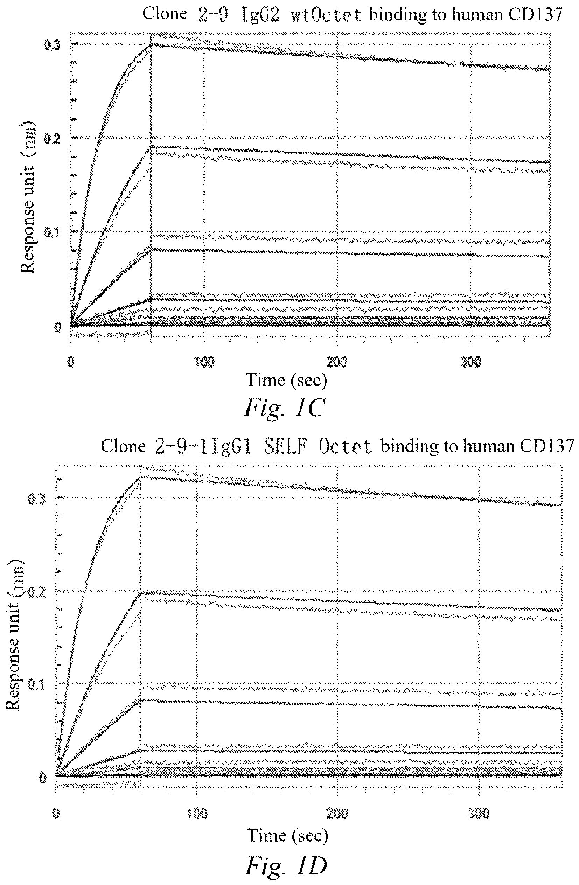

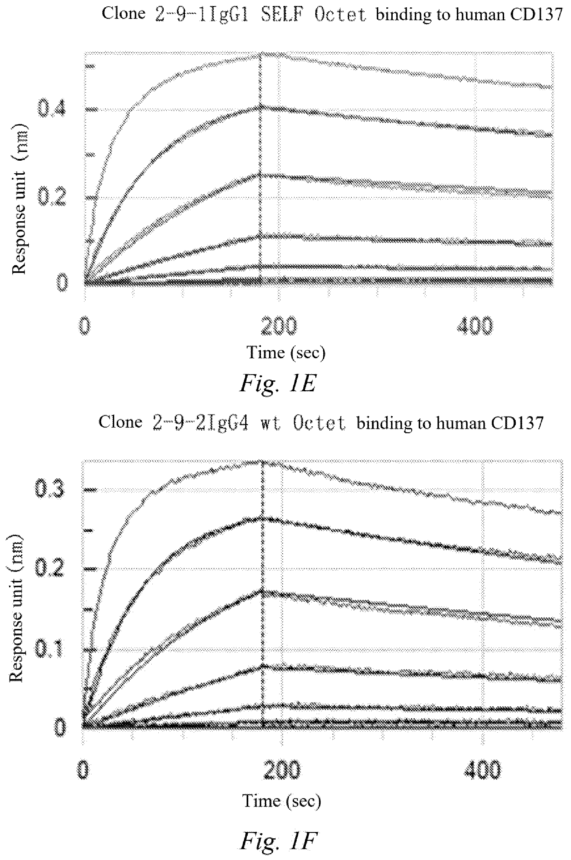

FIGS. 1A-1F show the binding affinities of exemplary anti-CD137 antibody clone 2-9 variants to human CD137 and cynomolgus monkey CD137. FIG. 1A shows the binding of anti-CD137 antibody clone 2-9 IgG2 wt to human CD137. FIG. 1B shows the binding of anti-CD137 antibody clone 2-9 IgG2 wt to cynomolgus monkey CD137. FIG. 1C shows the binding of anti-CD137 antibody clone 2-9 IgG2 wt to human CD137. FIG. ID shows the binding of anti-CD137 antibody clone 2-9-1 IgG1 SELF to human CD137. FIG. IE shows the binding of anti-CD137 antibody clone 2-9-1 IgG1 SELF to human CD137. FIG. 1F shows the binding of anti-CD137 antibody clone 2-9-2 IgG4 wt to human CD137.

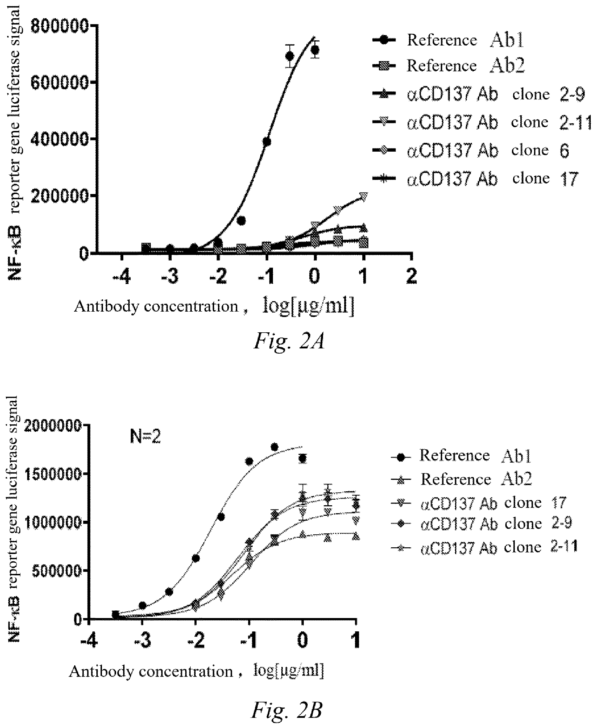

FIGS. 2A and 2B show luciferase activities in NF-κB reporter assay in the absence (2A) or presence (2B) of FcγRIIB-expressing 293F cells. Reference Ab 1 and Reference Ab 2 are reference anti-CD137 antibodies described in Example 2. αCD137 Ab clones indicates different anti-CD137 monoclonal antibodies.

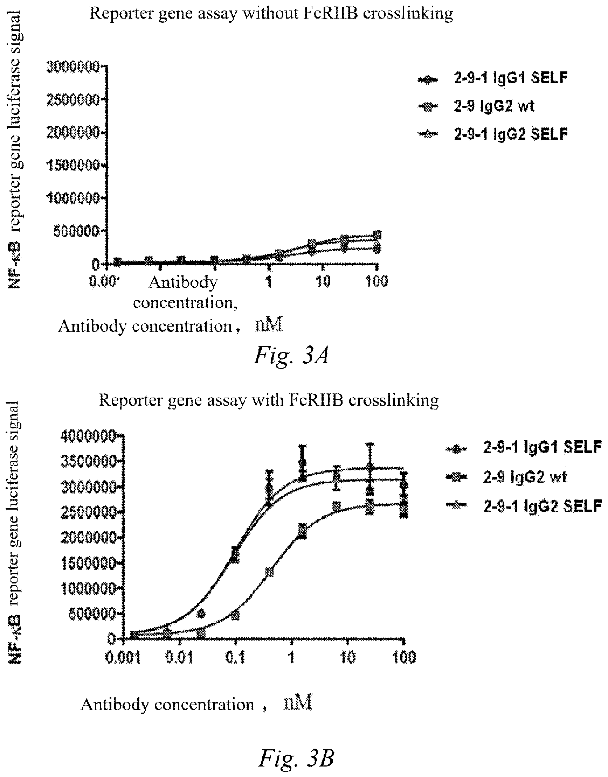

FIGS. 3A-3C show luciferase activities in NF-κB reporter assay in the absence (3A) or presence (3B and 3C) of FcγRIIB-expressing 293F cells. Reference Ab 1 and Reference Ab 2 are reference anti-CD137 antibodies described in Example 2. 2-9-1 IgG1 SELF comprises IgG1 Fc and mutations of S267E/L328F. 2-9-1 IgG2 SELF comprises IgG2 Fc and mutations of S267E/L328F. 2-9 IgG2 wt comprises wild type human IgG2 Fc. 2-9-4 IgG2 wt comprises wild type human IgG4 Fc.

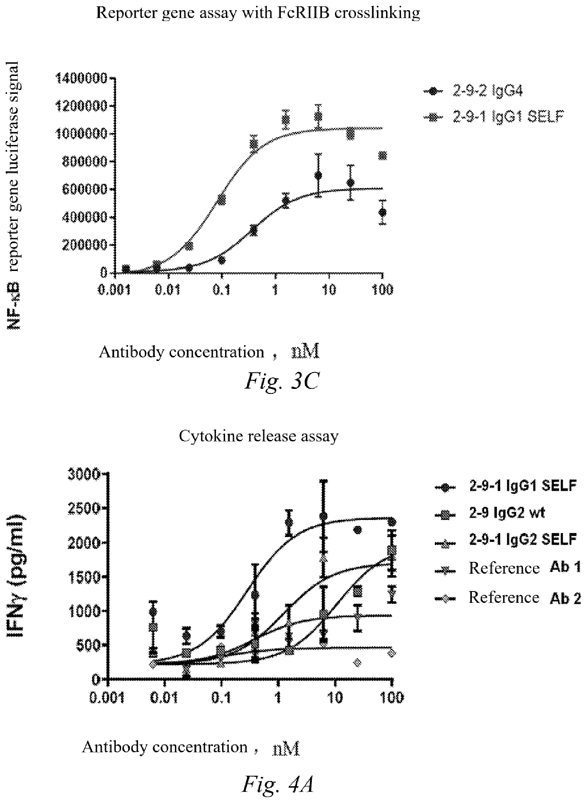

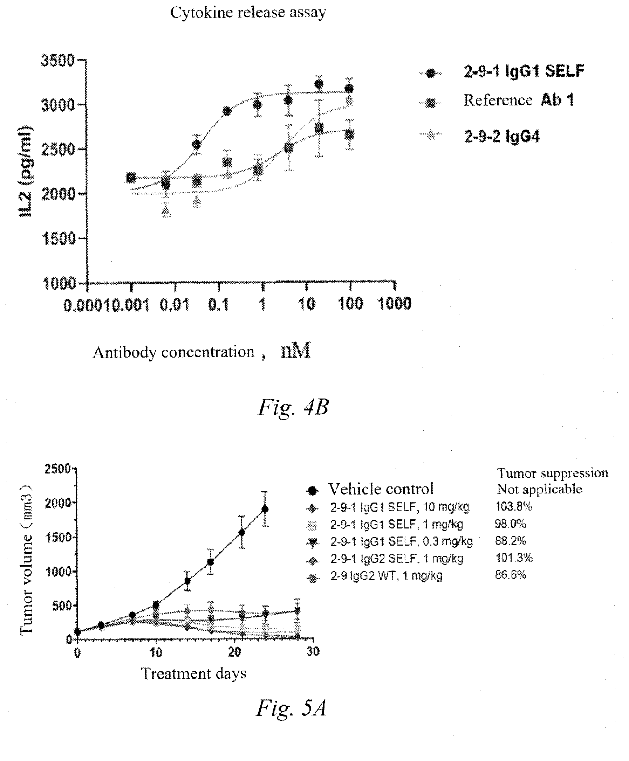

FIGS. 4A and 4B shows IFN-γ (4A) and IL2 (4B) production of PBMCs obtained from donors in the presence of different anti-CD137 antibodies at various concentrations. Reference Ab 1 and Reference Ab 2 are reference anti-CD137 antibodies described in Example 2. 2-9-1 IgG1 SELF comprises IgG1 Fc and mutations of S267E/L328F. 2-9-1 IgG2 SELF comprises IgG2 Fc and mutations of S267E/L328F. 2-9 IgG2 wt comprises wild type human IgG2 Fc. 2-9-4 IgG2 wt comprises wild type human IgG4 Fc.

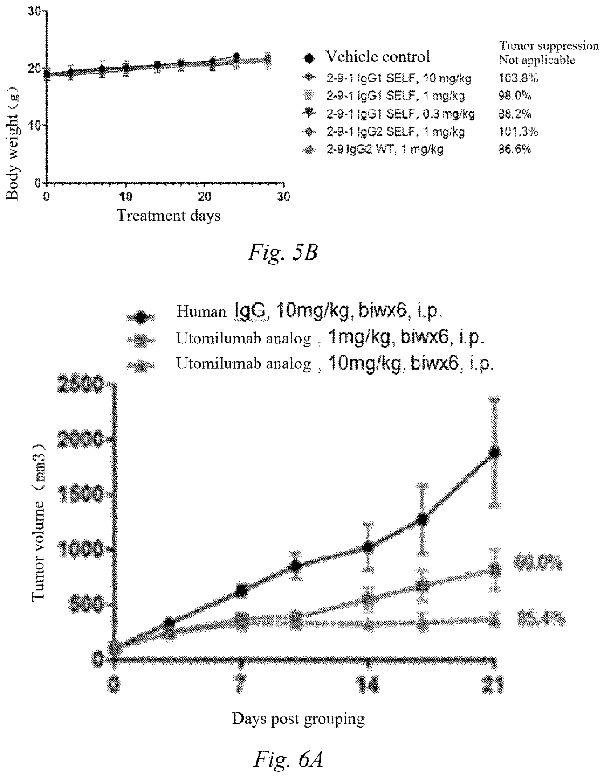

FIGS. 5A and 5B show in vivo study results of 2-9 variants in a MC38 murine colon cancer model. FIG. 5A shows tumor growths curves of vehicle control group and treatment groups. FIG. 5B shows mouse body weight changes during the treatment.

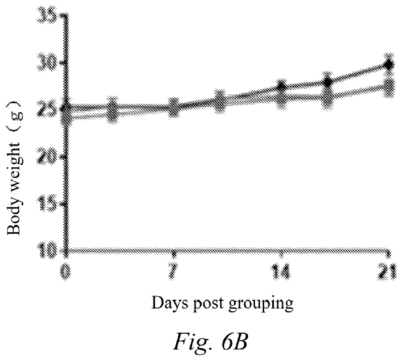

FIGS. 6A and 6B show in vivo study results of a utomilumab analog in a MC38 murine colon cancer model. FIG. 6A shows tumor growths curves of control IgG group and treatment groups. FIG. 6B shows mouse body weight changes during the treatment.

### DETAILED DESCRIPTION OF THE APPLICATION

The present application provides novel anti-CD137 constructs that specifically bind to CD137 (such as anti-CD137 scFvs, monoclonal antibodies, and multispecific antibodies that bind to a tumor-associated antigen (TAA)), methods of preparing the anti-CD 137 constructs, methods of using the constructs (e.g., methods of treating a disease or condition, methods of modulating an immune response, or methods of modulating a cell composition). The invention is based, in part, on the discovery of anti-CD137 monospecific antibodies and multispecific antibodies that exhibit improved safety profile and enhanced anti-tumor efficacy compared to existing anti-CD137 antibodies in clinical trials.

### I. Definitions

The term "antibody" is used in its broadest sense and encompasses various antibody structures, including but not limited to monoclonal antibodies, polyclonal antibodies, multispecific antibodies (e.g., bispecific antibodies), full-length antibodies and antigen-binding fragments thereof, so long as they exhibit the desired antigen-binding activity. The term "antibody moiety" refers to a full-length antibody or an antigen-binding fragment thereof.

A full-length antibody comprises two heavy chains and two light chains. The variable regions of the light and heavy chains are responsible for antigen binding. The variable domains of the heavy chain and light chain may be referred to as "V_{H}" and "V_{L}", respectively. The variable regions in both chains generally contain three highly variable loops called the complementarity determining regions (CDRs) (light chain (LC) CDRs including LC-CDR1, LC-CDR2, and LC-CDR3, heavy chain (HC) CDRs including HC-CDR1, HC-CDR2, and HC-CDR3). CDR boundaries for the antibodies and antigen-binding fragments disclosed herein may be defined or identified by the conventions of Kabat, Chothia, or Al-Lazikani (Al-Lazikani 1997; Chothia 1985; Chothia 1987; Chothia 1989; Kabat 1987; Kabat 1991). The three CDRs of the heavy or light chains are interposed between flanking stretches known as framework regions (FRs), which are more highly conserved than the CDRs and form a scaffold to support the hypervariable loops. The constant regions of the heavy and light chains are not involved in antigen binding, but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of α, δ, ε, γ, and µ heavy chains, respectively. Several of the major antibody classes are divided into subclasses such as lgG1 (γ1 heavy chain), lgG2 (γ2 heavy chain), lgG3 (γ3 heavy chain), lgG4 (γ4 heavy chain), lgA1 (α1 heavy chain), or lgA2 (α2 heavy chain).

The term "antigen-binding fragment" as used herein refers to an antibody fragment including, for example, a diabody, a Fab, a Fab', a F(ab')2, an Fv fragment, a disulfide stabilized Fv fragment (dsFv), a (dsFv)2, a multispecific dsFv (dsFv-dsFv'), a disulfide stabilized diabody (ds diabody), a single-chain Fv (scFv), an scFv dimer (bivalent diabody), a multispecific antibody formed from a portion of an antibody comprising one or more CDRs, a camelized single domain antibody, a nanobody, a domain antibody, a bivalent domain antibody, or any other antibody fragment that binds to an antigen but does not comprise a complete antibody structure. An antigen-binding fragment is capable of binding to the same antigen to which the parent antibody or a parent antibody fragment (*e.g.,* a parent scFv) binds. In some embodiments, an antigen-binding fragment may comprise one or more CDRs from a particular human antibody grafted to a framework region from one or more different human antibodies.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the heavy and light chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv," also abbreviated as "sFv" or "scFv," are antibody fragments that comprise the V_{H} and V_{L} antibody domains connected into a single polypeptide chain. In some embodiments, the scFv polypeptide further comprises a polypeptide linker between the V_{H} and V_{L} domains which enables the scFv to form the desired structure for antigen binding. For a review of scFv, *see* Plückthun in The Pharmacology of Monoclonal Antibodies, vol. 113, Rosenburg and Moore eds., Springer-Verlag, New York, pp. 269-315 (1994).

As used herein, the term "CDR" or "complementarity determining region" is intended to mean the non-contiguous antigen combining sites found within the variable region of both heavy and light chain polypeptides. These particular regions have been described by Kabat et al., J. Biol. Chem. 252:6609-6616 (1977); Kabat et al., U.S. Dept. of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), where the definitions include overlapping or subsets of amino acid residues when compared against each other. Nevertheless, application of either definition to refer to a CDR of an antibody or grafted antibodies or variants thereof is intended to be within the scope of the term as defined and used herein. The amino acid residues which encompass the CDRs as defined by each of the above cited references are set forth below in Table 1 as a comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present application and for possible inclusion in one or more claims herein.

**TABLE 1: CDR DEFINITIONS**

| | **Kabat¹** | | **Chothia²** | | **MacCallum³** | | **IMGT⁴** | **AHo⁵** |
|---|---|---|---|---|---|---|---|---|
| V_{H} CDR1 | | 31-35 | | 26-32 | | 30-35 | 27-38 | 25-40 |
| V_{H} CDR2 | | 50-65 | | 53-55 | | 47-58 | 56-65 | 58-77 |
| V_{H} CDR3 | | 95-102 | | 96-101 | | 93-101 | 105-117 | 109-137 |
| V_{L} CDR1 | | 24-34 | | 26-32 | | 30-36 | 27-38 | 25-40 |
| V_{L} CDR2 | | 50-56 | | 50-52 | | 46-55 | 56-65 | 58-77 |
| V_{L} CDR3 | | 89-97 | | 91-96 | | 89-96 | 105-117 | 109-137 |

| | | | | | | | | |
|---|---|---|---|---|---|---|---|---|
| ¹Residue numbering follows the nomenclature of Kabat *et al., supra* ²Residue numbering follows the nomenclature of Chothia *et al., supra* ³Residue numbering follows the nomenclature of MacCallum *et al., supra* ⁴Residue numbering follows the nomenclature of Lefranc *et al., supra* ⁵Residue numbering follows the nomenclature of Honegger and Plückthun, *supra* | | | | | | | | |

The expression "variable-domain residue-numbering as in Kabat" or "amino-acid-position numbering as in Kabat," and variations thereof, refers to the numbering system used for heavy-chain variable domains or light-chain variable domains of the compilation of antibodies in Kabat *et al.,* supra. Using this numbering system, the actual linear amino acid sequence may contain fewer or additional amino acids corresponding to a shortening of, or insertion into, a FR or hypervariable region (HVR) of the variable domain. For example, a heavy-chain variable domain may include a single amino acid insert (residue 52a according to Kabat) after residue 52 of H2 and inserted residues (e.g. residues 82a, 82b, and 82c, etc. according to Kabat) after heavy-chain FR residue 82. The Kabat numbering of residues may be determined for a given antibody by alignment at regions of homology of the sequence of the antibody with a "standard" Kabat numbered sequence.

Unless indicated otherwise herein, the amino acid residues which encompass the CDRs of a full-length antibody (e.g., an anti-CD137 antibody disclosed herein) are defined according to the Kabat nomenclature in Kabat et al., supra, and the numbering of the residues in an immunoglobulin heavy chain, e.g., in an Fc region, is that of the EU index as in Kabat et al., supra, except that the amino acid residues which encompass the CDRs of any consensus sequences are defined according to the Kabat nomenclature with modifications based on experimental conditions. The "EU index as in Kabat" refers to the residue numbering of the human IgG1 EU antibody.

"Framework" or "FR" residues are those variable-domain residues other than the CDR residues as herein defined.

"Humanized" forms of non-human (*e.g*., rodent) antibodies are chimeric antibodies that contain minimal sequence derived from the non-human antibody. For the most part, humanized antibodies are human immunoglobulins (recipient antibody) in which residues from a hypervariable region (HVR) of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies can comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, *see* Jones et al., Nature 321:522-525 (1986); Riechmann et al., Nature 332:323-329 (1988); and Presta, Curr. Op. Struct. Biol. 2:593-596 (1992).

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, *e.g.,* immunized xenomice (see, *e.g.,* U.S. Pat. Nos. 6,075,181 and 6,150,584 regarding XENOMOUSE^{™} technology). See also, for example, Li et al., Proc. Natl. Acad. Sci. USA, 103:3557-3562 (2006) regarding human antibodies generated *via* a human B-cell hybridoma technology.

"Percent (%) amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR), or MUSCLE software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program MUSCLE (Edgar, R.C., Nucleic Acids Research 32(5):1792-1797, 2004; Edgar, R.C., BMC Bioinformatics 5(1):113, 2004).

"Homologous" refers to the sequence similarity or sequence identity between two polypeptides or between two nucleic acid molecules. When a position in both of the two compared sequences is occupied by the same base or amino acid monomer subunit, *e.g*., if a position in each of two DNA molecules is occupied by adenine, then the molecules are homologous at that position. The percent of homology between two sequences is a function of the number of matching or homologous positions shared by the two sequences divided by the number of positions compared times 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

The term "constant domain" refers to the portion of an immunoglobulin molecule having a more conserved amino acid sequence relative to the other portion of the immunoglobulin, the variable domain, which contains the antigen-binding site. The constant domain contains the C_{H}1, C_{H}2 and C_{H}3 domains (collectively, C_{H}) of the heavy chain and the CHL (or C_{L}) domain of the light chain.

The "light chains" of antibodies (immunoglobulins) from any mammalian species can be assigned to one of two clearly distinct types, called kappa ("κ") and lambda ("λ"), based on the amino acid sequences of their constant domains.

The "CH1 domain" (also referred to as "C1" of "HI" domain) usually extends from about amino acid 118 to about amino acid 215 (EU numbering system).

"Hinge region" is generally defined as a region in IgG corresponding to Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "CH2 domain" of a human IgG Fc region (also referred to as "C2" domain) usually extends from about amino acid 231 to about amino acid 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, Molec Immunol. 22:161-206 (1985).

The "CH3 domain" (also referred to as "C2" domain) comprises the stretch of residues C-terminal to a CH2 domain in an Fc region (*i.e.* from about amino acid residue 341 to the C-terminal end of an antibody sequence, typically at amino acid residue 446 or 447 of an IgG).

The term "Fc region", "Fc fragment" or "fragment crystallizable region" herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies described herein include human IgG1, IgG2 (IgG2A, IgG2B), IgG3 and IgG4.

"Fc receptor" or "FcR" describes a receptor that binds the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors, FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (See M. Daëron, Annu. Rev. Immunol. 15:203-234 (1997). FcRs are reviewed in Ravetch and Kinet, Annu. Rev. Immunol. 9: 457-92 (1991); Capel et al., Immunomethods 4: 25-34 (1994); and de Haas et al., J. Lab. Clin. Med. 126: 330-41 (1995). Other FcRs, including those to be identified in the future, are encompassed by the term "FcR" herein.

The term "epitope" as used herein refers to the specific group of atoms or amino acids on an antigen to which an antibody or antibody moiety binds. Two antibodies or antibody moieties may bind the same epitope within an antigen if they exhibit competitive binding for the antigen.

As used herein, a first antibody or fragment thereof "competes" for binding to a target antigen with a second antibody or fragment thereof when the first antibody or fragment thereof inhibits the target antigen binding of the second antibody of fragment thereof by at least about 50% (such as at least about any one of 55%, 60%, 65%, 70%, 75%, 80%, 85%, 90%, 95%, 98% or 99%) in the presence of an equimolar concentration of the first antibody or fragment thereof, or *vice versa.* A high throughput process for "binning" antibodies based upon their cross-competition is described in PCT Publication No. WO 03/48731.

As use herein, the terms "specifically binds," "specifically recognizing," and "is specific for" refer to measurable and reproducible interactions, such as binding between a target and an antibody or antibody moiety, which is determinative of the presence of the target in the presence of a heterogeneous population of molecules, including biological molecules. For example, an antibody or antibody moiety that specifically recognizes a target (which can be an epitope) is an antibody or antibody moiety that binds this target with greater affinity, avidity, more readily, and/or with greater duration than its bindings to other targets. In some embodiments, the extent of binding of an antibody to an unrelated target is less than about 10% of the binding of the antibody to the target as measured, *e.g.*, by a radioimmunoassay (RIA). In some embodiments, an antibody that specifically binds a target has a dissociation constant (K_{D}) of ≤10⁻⁵ M, ≤10⁻⁶ M, ≤10⁻⁷ M, ≤10⁻⁸ M, ≤10⁻⁹ M, ≤10⁻¹⁰ M, ≤10⁻¹¹ M, or ≤10⁻¹² M. In some embodiments, an antibody specifically binds an epitope on a protein that is conserved among the protein from different species. In some embodiments, specific binding can include, but does not require exclusive binding. Binding specificity of the antibody or antigen-binding domain can be determined experimentally by methods known in the art. Such methods comprise, but are not limited to Western blots, ELISA-, RIA-, ECL-, IRMA-, EIA-, BIACORE^{™} -tests and peptide scans.

An "isolated" antibody (or construct) is one that has been identified, separated and/or recovered from a component of its production environment (*e.g*., natural or recombinant). Preferably, the isolated polypeptide is free of association with all other components from its production environment.

An "isolated" nucleic acid molecule encoding a construct, antibody, or antigen-binding fragment thereof described herein is a nucleic acid molecule that is identified and separated from at least one contaminant nucleic acid molecule with which it is ordinarily associated in the environment in which it was produced. Preferably, the isolated nucleic acid is free of association with all components associated with the production environment. The isolated nucleic acid molecules encoding the polypeptides and antibodies described herein is in a form other than in the form or setting in which it is found in nature. Isolated nucleic acid molecules therefore are distinguished from nucleic acid encoding the polypeptides and antibodies described herein existing naturally in cells. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "transfected" or "transformed" or "transduced" as used herein refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, and may contain mutations. Mutant progeny that have the same function or biological activity as screened or selected for in the originally transformed cell are included herein.

As used herein, "treatment" or "treating" is an approach for obtaining beneficial or desired results, including clinical results. For purposes of this application, beneficial or desired clinical results include, but are not limited to, one or more of the following: alleviating one or more symptoms resulting from the disease, diminishing the extent of the disease, stabilizing the disease (*e.g*., preventing or delaying the worsening of the disease), preventing or delaying the spread (*e.g*., metastasis) of the disease, preventing or delaying the recurrence of the disease, delaying or slowing the progression of the disease, ameliorating the disease state, providing a remission (partial or total) of the disease, decreasing the dose of one or more other medications required to treat the disease, delaying the progression of the disease, increasing or improving the quality of life, increasing weight gain, and/or prolonging survival. Also encompassed by "treatment" is a reduction of pathological consequence of cancer (such as, for example, tumor volume). The methods of the application contemplate any one or more of these aspects of treatment.

In the context of cancer, the term "treating" includes any or all of: inhibiting growth of cancer cells, inhibiting replication of cancer cells, lessening of overall tumor burden and ameliorating one or more symptoms associated with the disease.

The terms "inhibition" or "inhibit" refer to a decrease or cessation of any phenotypic characteristic or to the decrease or cessation in the incidence, degree, or likelihood of that characteristic. To "reduce" or "inhibit" is to decrease, reduce or arrest an activity, function, and/or amount as compared to that of a reference. In certain embodiments, by "reduce" or "inhibit" is meant the ability to cause an overall decrease of 20% or greater. In another embodiment, by "reduce" or "inhibit" is meant the ability to cause an overall decrease of 50% or greater. In yet another embodiment, by "reduce" or "inhibit" is meant the ability to cause an overall decrease of 75%, 85%, 90%, 95%, or greater.

A "reference" as used herein, refers to any sample, standard, or level that is used for comparison purposes. A reference may be obtained from a healthy and/or non-diseased sample. In some examples, a reference may be obtained from an untreated sample. In some examples, a reference is obtained from a non-diseased or non-treated sample of an individual. In some examples, a reference is obtained from one or more healthy individuals who are not the individual or patient.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

"Preventing" as used herein, includes providing prophylaxis with respect to the occurrence or recurrence of a disease in an individual that may be predisposed to the disease but has not yet been diagnosed with the disease.

As used herein, to "suppress" a function or activity is to reduce the function or activity when compared to otherwise same conditions except for a condition or parameter of interest, or alternatively, as compared to another condition. For example, an antibody which suppresses tumor growth reduces the rate of growth of the tumor compared to the rate of growth of the tumor in the absence of the antibody.

The terms "subject," "individual," and "patient" are used interchangeably herein to refer to a mammal, including, but not limited to, human, bovine, horse, feline, canine, rodent, or primate. In some embodiments, the individual is a human.

An "effective amount" of an agent refers to an amount effective, at dosages and for periods of time necessary, to achieve the desired therapeutic or prophylactic result. The specific dose may vary depending on one or more of: the particular agent chosen, the dosing regimen to be followed, whether it is administered in combination with other compounds, timing of administration, the tissue to be imaged, and the physical delivery system in which it is carried.

A "therapeutically effective amount" of a substance/molecule of the application, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. A therapeutically effective amount may be delivered in one or more administrations.

A "prophylactically effective amount" refers to an amount effective, at dosages and for periods of time necessary, to achieve the desired prophylactic result. Typically, but not necessarily, since a prophylactic dose is used in subjects prior to or at an earlier stage of disease, the prophylactically effective amount will be less than the therapeutically effective amount.

The terms "pharmaceutical formulation" and "pharmaceutical composition" refer to a preparation which is in such form as to permit the biological activity of the active ingredient(s) to be effective, and which contains no additional components which are unacceptably toxic to an individual to which the formulation would be administered. Such formulations may be sterile.

A "pharmaceutically acceptable carrier" refers to a non-toxic solid, semisolid, or liquid filler, diluent, encapsulating material, formulation auxiliary, or carrier conventional in the art for use with a therapeutic agent that together comprise a "pharmaceutical composition" for administration to an individual. A pharmaceutically acceptable carrier is non-toxic to recipients at the dosages and concentrations employed and is compatible with other ingredients of the formulation. The pharmaceutically acceptable carrier is appropriate for the formulation employed.

A "sterile" formulation is aseptic or essentially free from living microorganisms and their spores.

Administration "in combination with" one or more further therapeutic agents includes simultaneous (concurrent) and consecutive or sequential administration in any order.

The term "concurrently" is used herein to refer to administration of two or more therapeutic agents, where at least part of the administration overlaps in time or where the administration of one therapeutic agent falls within a short period of time relative to administration of the other therapeutic agent. For example, the two or more therapeutic agents are administered with a time separation of no more than about 60 minutes, such as no more than about any of 30, 15, 10, 5, or 1 minutes.

The term "sequentially" is used herein to refer to administration of two or more therapeutic agents where the administration of one or more agent(s) continues after discontinuing the administration of one or more other agent(s). For example, administration of the two or more therapeutic agents are administered with a time separation of more than about 15 minutes, such as about any of 20, 30, 40, 50, or 60 minutes, 1 day, 2 days, 3 days, 1 week, 2 weeks, or 1 month, or longer.

As used herein, "in conjunction with" refers to administration of one treatment modality in addition to another treatment modality. As such, "in conjunction with" refers to administration of one treatment modality before, during or after administration of the other treatment modality to the individual.

The term "package insert" is used to refer to instructions customarily included in commercial packages of therapeutic products, that contain information about the indications, usage, dosage, administration, combination therapy, contraindications and/or warnings concerning the use of such therapeutic products.

An "article of manufacture" is any manufacture (*e.g*., a package or container) or kit comprising at least one reagent, *e.g.,* a medicament for treatment of a disease or disorder (*e.g.,* cancer), or a probe for specifically detecting a biomarker described herein. In certain embodiments, the manufacture or kit is promoted, distributed, or sold as a unit for performing the methods described herein.

It is understood that embodiments of the application described herein include "consisting" and/or "consisting essentially of" embodiments.

Reference to "about" a value or parameter herein includes (and describes) variations that are directed to that value or parameter *per se.* For example, description referring to "about X" includes description of "X".

As used herein, reference to "not" a value or parameter generally means and describes "other than" a value or parameter. For example, the method is not used to treat cancer of type X means the method is used to treat cancer of types other than X.

The term "about X-Y" used herein has the same meaning as "about X to about Y."

As used herein and in the appended claims, the singular forms "a," "or," and "the" include plural referents unless the context clearly dictates otherwise.

### II. CD137 (4-1BB)

CD137 (4- 1BB) is a member of the tumor necrosis receptor (TNF-R) gene family, which includes proteins involved in regulation of cell proliferation, differentiation, and programmed cell death. CD137 is a 30 kDa type I membrane glycoprotein expressed as a 55 kDa homodimer. The receptor was initially described in mice (B. Kwon et al., P.N.A.S. USA, 86:1963-7 (1989)), and later identified in humans (M. Alderson et al., Eur. J. Immunol., 24: 2219-27 (1994); Z. Zhou et al., Immunol. Lett., 45:67 (1995)) (See, also, Published PCT Applications WO95/07984 and WO96/29348, and U.S. Patent No. 6,569,997, hereby incorporated by reference (See, SEQ ID NO:2.)). The human and murine forms of CD137 are 60% identical at the amino acid level. Conserved sequences occur in the cytoplasmic domain, as well as 5 other regions of the molecule, indicating that these residues might be important for function of the CD137 molecule (Z. Zhou et al., Immunol. Lett., 45:67 (1995)). Expression of CD137 has been shown to be predominantly on cells of lymphoid lineage such as activated T-cells, activated Natural Killer (NK) cells, NKT-cells, CD4+CD25+ regulatory T-cells, and also on activated thymocytes, and intraepithelial lymphocytes. In addition, CD137 has also been shown to be expressed on cells of myeloid origin like dendritic cells, monocytes, neutrophils, and eosinophils. Even though CD137 expression is mainly restricted to immune/inflammatory cells, there have been reports describing its expression on endothelial cells associated with a small number of tissues from inflammatory sites and tumors.

Functional activities of CD137 on T-cells have been amply characterized. Signaling through CD137 in the presence of suboptimal doses of anti-CD3 has been demonstrated to induce T-cell proliferation and cytokine synthesis (mainly IFN-γ), and to inhibit activated cell death. These effects have been observed with both murine and human T-cells (W. Shuford et al., J. Exp. Med., 186(1):47-55 (1997); D. Vinay et al., Semin. Immunol, 10(6):481-9 (1998); D. Laderach et al., Int. Immunol., 14(10): 1155-67 (2002)). In both humans and mice, co-stimulation enhances effector functions, such as IFN-γ production and cytotoxicity, by augmenting the numbers of antigen-specific and effector CD8+ T-cells. In the absence of anti-CD3 signaling, stimulation through CD137 does not alter T-cell function, indicating that CD137 is a co-stimulatory molecule.

A role for CD137 targeted therapy in the treatment of cancer was suggested by in vivo efficacy studies in mice utilizing agonistic anti-murine CD137 monoclonal antibodies. In a paper by Melero *et al,* agonistic anti-mouse CD137 antibody produced cures in P815 mastocytoma tumors, and in the low immunogenic tumor model Agl04 (I. Melero et al, Nat. Med., 3(6):682-5 (1997)). The anti-tumor effect required both CD4+ and CD 8+ T-cells and NK cells, since selective in vivo depletion of each subpopulation resulted in the reduction or complete loss of the anti-tumor effect. It was also demonstrated that a minimal induction of an immune response was necessary for anti-CD 137 therapy to be effective. Several investigators have used anti-CD137 antibodies to demonstrate the viability of this approach for cancer therapy (J. Kim et al, Cancer Res., 61(5):2031-7 (2001); O. Martinet et al, Gene Ther., 9(12):786-92 (2002); R. Miller et al, J. Immunol, 169(4): 1792-800 (2002); R. Wilcox et al, Cancer Res., 62(15):4413-8 (2002)).

In addition to its role in the development of immunity to cancer, experimental data supports the use of CD137 agonistic antibodies for the treatment of autoimmune and viral diseases (B. Kwon et al, Exp. Mol. Med., 35(1):8-16 (2003); H. Salih et al, J. Immunol, 167(7):4059-66 (2001); E. Kwon et al, P.N.A.S. USA, 96:15074-79 (1999); J. Foell et al, N.Y. Acad. Sci., 987:230-5 (2003); Y. Sun et al, Nat. Med., 8(12): 1405-13 (2002) S. K. Seo et al, Nat. Med. 10;1099-94 (2004)).

### III. Anti-CD137 constructs

In one aspect, the present invention provides novel CD137-specific constructs (such as isolated anti-CD137 constructs) that comprise an antibody moiety that specifically binds to CD137. The specificity of the anti-CD137 construct derives from an anti-CD137 antibody moiety, such as a full-length antibody or antigen-binding fragment thereof, which specifically binds to CD137. In some embodiments, reference to a moiety (such as an antibody moiety) that specifically binds to CD137 means that the moiety binds to the CD137 with an affinity that is at least about 10 times (including for example at least about any of 10, 10², 10³, 10⁴, 10⁵, 10⁶, or 10⁷ times) its binding affinity for non-target. In some embodiments, the non-target is an antigen that is not CD137. Binding affinity can be determined by methods known in the art, such as ELISA, fluorescence activated cell sorting (FACS) analysis, or radioimmunoprecipitation assay (RIA). K_{d} can be determined by methods known in the art, such as surface plasmon resonance (SPR) assay utilizing, for example, Biacore instruments, or kinetic exclusion assay (KinExA) utilizing, for example, Sapidyne instruments.

Contemplated anti-CD137 constructs include, but are not limited to, anti-CD137 scFvs, fusion proteins comprising an anti-CD137 antibody moiety and a half-life extending domain (such as an Fc fragment, albumin-binding domain), anti-CD137 monoclonal antibodies, multi-specific anti-CD137 molecules (such as bispecific antibodies). The exemplary anti-CD137 constructs above are not mutually exclusive and are further discussed in various sections below in more details.

In some embodiments, there is provided an anti-CD137 construct (e.g., an anti-CD137 scFv) comprising an anti-CD137 antibody moiety specifically recognizing CD137 (*e.g.,* human CD137), wherein the anti-CD137 antibody moiety can be any one of the anti-CD 137 antibody moieties described herein.

In some embodiments, there is provided an anti-CD137 construct (e.g., an anti-CD137 scFv) comprising an anti-CD137 antibody moiety that binds to CD137, comprising a heavy chain variable region (V_{H}) and a light chain variable region (V_{L}), wherein: a) the V_{H} comprises: i) an HC-CDR1 comprising an amino acid sequence of GFX₁X₂X₃DTYIX₄ (SEQ ID NO: 177), wherein X₁=N or C; X₂=I, P, L or M; X₃=K, N, R, C or Q; X₄=H or Q, ii) an HC-CDR2 comprising an amino acid sequence of X₁IDPANGX₂X₃X₄ (SEQ ID NO:178), wherein X₁=K or R; X₂=N, G, F, Y, A, D, L, M or Q; X₃=S or T; X₄=E or M, and iii) an HC-CDR3 comprising an amino acid sequence of GNLHYX₁LMD (SEQ ID NO: 179), wherein X₁=Y, A or G; and b) the V_{L} comprises: i) an LC-CDR1 comprising an amino acid sequence of KASQX₁X₂X₃TYX₄S (SEQ ID NO: 180), wherein X₁=A, P or T; X₂=I, T or P; X₃=N or A; X₄=L, G or H, ii) an LC-CDR2 comprising an amino acid sequence of RX₁NRX₂X₃X₄ (SEQ ID NO: 181), wherein X₁=A, Y, V or D; X₂=M, K, V or A; X₃=V, P, Y or G; X₄=D or G, and iii) an LC-CDR3 comprising an amino acid sequence of LQX₁X₂DFPYX₃ (SEQ ID NO: 182), wherein X₁=Y, S or F; X₂=D, V, L, R, E or Q; X₃=T or K.

In some embodiments, the anti-CD 137 antibody moiety that binds to CD137 comprises a) the HC-CDR1 comprises an amino acid sequence of any one of SEQ ID NOs: 1, 11, 21, 31, 41, 51, 61, 71, 81, 91, 101, 111, 121, 131, 141, 211 and 221, or a variant thereof comprising up to about 3 (such as 3, 2, or 1) amino acid substitutions; b) the HC-CDR2 comprises an amino acid sequence of any one of SEQ ID NOs: 2, 12, 22, 32, 42, 52, 62, 72, 82, 92, 102, 112, 122, 132, 142, 212 and 222, or a variant thereof comprising up to about 3 (such as 3, 2, or 1) amino acid substitutions; c) the HC-CDR3 comprises an amino acid sequence of any one of SEQ ID NOs: 3, 13, 23, 33, 43, 53, 63, 73, 83, 93, 103, 113, 123, 133, 143, 213 and 223, or a variant thereof comprising up to about 3 (such as 3, 2, or 1) amino acid substitutions; d) the LC-CDR1 comprises an amino acid sequence of any one of SEQ ID NOs: 4, 14, 24, 34, 44, 54, 64, 74, 84, 94, 104, 114, 124, 134, 144, 214 and 224, or a variant thereof comprising up to about 3 (such as 3, 2, or 1) amino acid substitutions; e) the LC-CDR2 comprises an amino acid sequence of any one of SEQ ID NOs: 5, 15, 25, 35, 45, 55, 65, 75, 85, 95, 105, 115, 125, 135, 145, 215 and 225, or a variant thereof comprising up to about 3 (such as 3, 2, or 1) amino acid substitutions; f) the LC-CDR3 comprises an amino acid sequence of any one of SEQ ID NOs: 6, 16, 26, 36, 46, 56, 66, 76, 86, 96, 106, 116, 126, 136, 146, 216 and 226, or a variant thereof comprising up to about 3 (such as 3, 2, or 1) amino acid substitutions. In some embodiments, the amino acid substitutions are limited to "exemplary substitutions" shown in Table 2 of this application. In some embodiments, the amino acid substitutions are limited to "preferred substitutions" shown in Table 2 of this application.

In some embodiments, there is provided an anti-CD137 construct comprising an anti-CD137 antibody moiety that cross-competes for binding to CD137 with a reference anti-CD137 construct comprising a heavy chain variable region (V_{H}) comprising HC-CDR1, HC-CDR2 and HC-CDR3 domains and a light chain variable region (V_{L}) comprising LC-CDR1, LC-CDR2 and LC-CDR3 domains that are selected from the group consisting of: a) the V_{H} comprises the HC -CDR1 comprising the amino acid sequence of SEQ ID NO: 1, or a variant thereof comprising up to about 3 (such as 3, 2, or 1) amino acid substitutions, the HC -CDR2 comprising the amino acid sequence of SEQ ID NO: 2, or a variant thereof comprising up to about 3 (such as 3, 2, or 1) amino acid substitutions, and the HC -CDR3 comprising the amino acid sequence of SEQ ID NO: 3, or a variant thereof comprising up to about 3 (such as 3, 2, or 1) amino acid substitutions, and the V_{L} comprises the LC -CDR1 comprising the amino acid sequence of SEQ ID NO: 4, or a variant thereof comprising up to about 3 (such as 3, 2, or 1) amino acid substitutions, the LC -CDR2 comprising the amino acid sequence of SEQ ID NO: 5, , or a variant thereof comprising up to about 3 (such as 3, 2, or 1) amino acid substitutions, and the LC -CDR3 comprising the amino acid sequence of SEQ ID NO: 6, or a variant thereof comprising up to about 3 (such as 3, 2, or 1) amino acid substitutions; b) the V_{H} comprises the HC -CDR1 comprising the amino acid sequence of SEQ ID NO: 11, or a variant thereof comprising up to about 3 (such as 3, 2, or 1) amino acid substitutions, the HC -CDR2 comprising the amino acid sequence of SEQ ID NO: 12, or a variant thereof comprising up to about 3 (such as 3, 2, or 1) amino acid substitutions, and the HC -CDR3 comprising the amino acid sequence of SEQ ID NO: 13, or a variant thereof comprising up to about 3 (such as 3, 2, or 1) amino acid substitutions, and the V_{L} comprises the LC -CDR1 comprising the amino acid sequence of SEQ ID NO: 14, or a variant thereof comprising up to about 3 (such as 3, 2, or 1) amino acid substitutions, the LC -CDR2 comprising the amino acid sequence of SEQ ID NO: 15, or a variant thereof comprising up to about 3 (such as 3, 2, or 1) amino acid substitutions, and the LC -CDR3 comprising the amino acid sequence of SEQ ID NO: 16, or a variant thereof comprising up to about 3 (such as 3, 2, or 1) amino acid substitutions; c) the V_{H} comprises the HC -CDR1 comprising the amino acid sequence of SEQ ID NO: 21, or a variant thereof comprising up to about 3 (such as 3, 2, or 1) amino acid substitutions, the HC -CDR2 comprising the amino acid sequence of SEQ ID NO: 22, or a variant thereof comprising up to about 3 (such as 3, 2, or 1) amino acid substitutions, and the HC -CDR3 comprising the amino acid sequence of SEQ ID NO: 23, or a variant thereof comprising up to about 3 (such as 3, 2, or 1) amino acid substitutions, and the V_{L} comprises the LC -CDR1 comprising the amino acid sequence of SEQ ID NO: 24, or a variant thereof comprising up to about 3 (such as 3, 2, or 1) amino acid substitutions, the LC -CDR2 comprising the amino acid sequence of SEQ ID NO: 25, or a variant thereof comprising up to about 3 (such as 3, 2, or 1) amino acid substitutions, and the LC -CDR3 comprising the amino acid sequence of SEQ ID NO: 26, or a variant thereof comprising up to about 3 (such as 3, 2, or 1) amino acid substitutions; d) the V_{H} comprises the HC -CDR1 comprising the amino acid sequence of SEQ ID NO: 31, or a variant thereof comprising up to about 3 (such as 3, 2, or 1) amino acid substitutions, the HC -CDR2 comprising the amino acid sequence of SEQ ID NO: 32, or a variant thereof comprising up to about 3 (such as 3, 2, or 1) amino acid substitutions, and the HC -CDR3 comprising the amino acid sequence of SEQ ID NO: 33, or a variant thereof comprising up to about 3 (such as 3, 2, or 1) amino acid substitutions, and the V_{L} comprises the LC -CDR1 comprising the amino acid sequence of SEQ ID NO: 34, or a variant thereof comprising up to about 3 (such as 3, 2, or 1) amino acid substitutions, the LC -CDR2 comprising the amino acid sequence of SEQ ID NO: 35, or a variant thereof comprising up to about 3 (such as 3, 2, or 1) amino acid substitutions, and the LC -CDR3 comprising the amino acid sequence of SEQ ID NO: 36, or a variant thereof comprising up to about 3 (such as 3, 2, or 1) amino acid substitutions; e) the V_{H} comprises the HC -CDR1 comprising the amino acid sequence of SEQ ID NO: 41, or a variant thereof comprising up to about 3 (such as 3, 2, or 1) amino acid substitutions, the HC -CDR2 comprising the amino acid sequence of SEQ ID NO: 42, or a variant thereof comprising up to about 3 (such as 3, 2, or 1) amino acid substitutions, and the HC -CDR3 comprising the amino acid sequence of SEQ ID NO: 43, or a variant thereof comprising up to about 3 (such as 3, 2, or 1) amino acid substitutions, and the V_{L} comprises the LC -CDR1 comprising the amino acid sequence of SEQ ID NO: 44, or a variant thereof comprising up to about 3 (such as 3, 2, or 1) amino acid substitutions, the LC -CDR2 comprising the amino acid sequence of SEQ ID NO: 45, or a variant thereof comprising up to about 3 (such as 3, 2, or 1) amino acid substitutions, and the LC -CDR3 comprising the amino acid sequence of SEQ ID NO: 46, or a variant thereof comprising up to about 3 (such as 3, 2, or 1) amino acid substitutions; f) the V_{H} comprises the HC -CDR1 comprising the amino acid sequence of SEQ ID NO: 51, or a variant thereof comprising up to about 3 (such as 3, 2, or 1) amino acid substitutions, the HC -CDR2 comprising the amino acid sequence of SEQ ID NO: 52, or a variant thereof comprising up to about 3 (such as 3, 2, or 1) amino acid substitutions, and the HC -CDR3 comprising the amino acid sequence of SEQ ID NO: 53, or a variant thereof comprising up to about 3 (such as 3, 2, or 1) amino acid substitutions, and the V_{L} comprises the LC -CDR1 comprising the amino acid sequence of SEQ ID NO: 54, or a variant thereof comprising up to about 3 (such as 3, 2, or 1) amino acid substitutions, the LC -CDR2 comprising the amino acid sequence of SEQ ID NO: 55, or a variant thereof comprising up to about 3 (such as 3, 2, or 1) amino acid substitutions, and the LC -CDR3 comprising the amino acid sequence of SEQ ID NO: 56, or a variant thereof comprising up to about 3 (such as 3, 2, or 1) amino acid substitutions; g) the V_{H} comprises the HC -CDR1 comprising the amino acid sequence of SEQ ID NO: 61, or a variant thereof comprising up to about 3 (such as 3, 2, or 1) amino acid substitutions, the HC -CDR2 comprising the amino acid sequence of SEQ ID NO: 62, or a variant thereof comprising up to about 3 (such as 3, 2, or 1) amino acid substitutions, and the HC -CDR3 comprising the amino acid sequence of SEQ ID NO: 63, or a variant thereof comprising up to about 3 (such as 3, 2, or 1) amino acid substitutions, and the V_{L} comprises the LC -CDR1 comprising the amino acid sequence of SEQ ID NO: 64, or a variant thereof comprising up to about 3 (such as 3, 2, or 1) amino acid substitutions, the LC -CDR2 comprising the amino acid sequence of SEQ ID NO: 65, or a variant thereof comprising up to about 3 (such as 3, 2, or 1) amino acid substitutions, and the LC -CDR3 comprising the amino acid sequence of SEQ ID NO: 66, or a variant thereof comprising up to about 3 (such as 3, 2, or 1) amino acid substitutions; h) the V_{H} comprises the HC -CDR1 comprising the amino acid sequence of SEQ ID NO: 71, or a variant thereof comprising up to about 3 (such as 3, 2, or 1) amino acid substitutions, the HC -CDR2 comprising the amino acid sequence of SEQ ID NO: 72, or a variant thereof comprising up to about 3 (such as 3, 2, or 1) amino acid substitutions, and the HC -CDR3 comprising the amino acid sequence of SEQ ID NO: 73, or a variant thereof comprising up to about 3 (such as 3, 2, or 1) amino acid substitutions, and the V_{L} comprises the LC -CDR1 comprising the amino acid sequence of SEQ ID NO: 74, or a variant thereof comprising up to about 3 (such as 3, 2, or 1) amino acid substitutions, the LC -CDR2 comprising the amino acid sequence of SEQ ID NO: 75, or a variant thereof comprising up to about 3 (such as 3, 2, or 1) amino acid substitutions, and the LC -CDR3 comprising the amino acid sequence of SEQ ID NO: 76, or a variant thereof comprising up to about 3 (such as 3, 2, or 1) amino acid substitutions; i) the V_{H} comprises the HC -CDR1 comprising the amino acid sequence of SEQ ID NO: 81, or a variant thereof comprising up to about 3 (such as 3, 2, or 1) amino acid substitutions, the HC -CDR2 comprising the amino acid sequence of SEQ ID NO: 82, or a variant thereof comprising up to about 3 (such as 3, 2, or 1) amino acid substitutions, and the HC -CDR3 comprising the amino acid sequence of SEQ ID NO: 83, or a variant thereof comprising up to about 3 (such as 3, 2, or 1) amino acid substitutions, and the V_{L} comprises the LC -CDR1 comprising the amino acid sequence of SEQ ID NO: 84, or a variant thereof comprising up to about 3 (such as 3, 2, or 1) amino acid substitutions, the LC -CDR2 comprising the amino acid sequence of SEQ ID NO: 85, or a variant thereof comprising up to about 3 (such as 3, 2, or 1) amino acid substitutions, and the LC -CDR3 comprising the amino acid sequence of SEQ ID NO: 86, or a variant thereof comprising up to about 3 (such as 3, 2, or 1) amino acid substitutions; j) the V_{H} comprises the HC -CDR1 comprising the amino acid sequence of SEQ ID NO: 91, or a variant thereof comprising up to about 3 (such as 3, 2, or 1) amino acid substitutions, the HC -CDR2 comprising the amino acid sequence of SEQ ID NO: 92, or a variant thereof comprising up to about 3 (such as 3, 2, or 1) amino acid substitutions, and the HC -CDR3 comprising the amino acid sequence of SEQ ID NO: 93, or a variant thereof comprising up to about 3 (such as 3, 2, or 1) amino acid substitutions, and the V_{L} comprises the LC -CDR1 comprising the amino acid sequence of SEQ ID NO: 94, or a variant thereof comprising up to about 3 (such as 3, 2, or 1) amino acid substitutions, the LC -CDR2 comprising the amino acid sequence of SEQ ID NO: 95, or a variant thereof comprising up to about 3 (such as 3, 2, or 1) amino acid substitutions, and the LC -CDR3 comprising the amino acid sequence of SEQ ID NO: 96, or a variant thereof comprising up to about 3 (such as 3, 2, or 1) amino acid substitutions; k) the V_{H} comprises the HC -CDR1 comprising the amino acid sequence of SEQ ID NO: 101, or a variant thereof comprising up to about 3 (such as 3, 2, or 1) amino acid substitutions, the HC -CDR2 comprising the amino acid sequence of SEQ ID NO: 102, or a variant thereof comprising up to about 3 (such as 3, 2, or 1) amino acid substitutions, and the HC -CDR3 comprising the amino acid sequence of SEQ ID NO: 103, or a variant thereof comprising up to about 3 (such as 3, 2, or 1) amino acid substitutions, and the V_{L} comprises the LC -CDR1 comprising the amino acid sequence of SEQ ID NO: 104, or a variant thereof comprising up to about 3 (such as 3, 2, or 1) amino acid substitutions, the LC -CDR2 comprising the amino acid sequence of SEQ ID NO: 105, or a variant thereof comprising up to about 3 (such as 3, 2, or 1) amino acid substitutions, and the LC -CDR3 comprising the amino acid sequence of SEQ ID NO:106, or a variant thereof comprising up to about 3 (such as 3, 2, or 1) amino acid substitutions; l) the V_{H} comprises the HC -CDR1 comprising the amino acid sequence of SEQ ID NO: 111, or a variant thereof comprising up to about 3 (such as 3, 2, or 1) amino acid substitutions, the HC -CDR2 comprising the amino acid sequence of SEQ ID NO: 112, or a variant thereof comprising up to about 3 (such as 3, 2, or 1) amino acid substitutions, and the HC -CDR3 comprising the amino acid sequence of SEQ ID NO: 113, or a variant thereof comprising up to about 3 (such as 3, 2, or 1) amino acid substitutions, and the V_{L} comprises the LC -CDR1 comprising the amino acid sequence of SEQ ID NO: 114, or a variant thereof comprising up to about 3 (such as 3, 2, or 1) amino acid substitutions, the LC -CDR2 comprising the amino acid sequence of SEQ ID NO: 115, or a variant thereof comprising up to about 3 (such as 3, 2, or 1) amino acid substitutions, and the LC -CDR3 comprising the amino acid sequence of SEQ ID NO: 116, or a variant thereof comprising up to about 3 (such as 3, 2, or 1) amino acid substitutions; m) the V_{H} comprises the HC -CDR1 comprising the amino acid sequence of SEQ ID NO: 121, or a variant thereof comprising up to about 3 (such as 3, 2, or 1) amino acid substitutions, the HC -CDR2 comprising the amino acid sequence of SEQ ID NO: 122, or a variant thereof comprising up to about 3 (such as 3, 2, or 1) amino acid substitutions, and the HC -CDR3 comprising the amino acid sequence of SEQ ID NO: 123, or a variant thereof comprising up to about 3 (such as 3, 2, or 1) amino acid substitutions, and the V_{L} comprises the LC -CDR1 comprising the amino acid sequence of SEQ ID NO: 124, or a variant thereof comprising up to about 3 (such as 3, 2, or 1) amino acid substitutions, the LC -CDR2 comprising the amino acid sequence of SEQ ID NO: 125, or a variant thereof comprising up to about 3 (such as 3, 2, or 1) amino acid substitutions, and the LC -CDR3 comprising the amino acid sequence of SEQ ID NO: 126, or a variant thereof comprising up to about 3 (such as 3, 2, or 1) amino acid substitutions; n) the V_{H} comprises the HC -CDR1 comprising the amino acid sequence of SEQ ID NO: 131, or a variant thereof comprising up to about 3 (such as 3, 2, or 1) amino acid substitutions, the HC -CDR2 comprising the amino acid sequence of SEQ ID NO: 132, or a variant thereof comprising up to about 3 (such as 3, 2, or 1) amino acid substitutions, and the HC -CDR3 comprising the amino acid sequence of SEQ ID NO: 133, or a variant thereof comprising up to about 3 (such as 3, 2, or 1) amino acid substitutions, and the V_{L} comprises the LC -CDR1 comprising the amino acid sequence of SEQ ID NO: 134, or a variant thereof comprising up to about 3 (such as 3, 2, or 1) amino acid substitutions, the LC -CDR2 comprising the amino acid sequence of SEQ ID NO: 135, or a variant thereof comprising up to about 3 (such as 3, 2, or 1) amino acid substitutions, and the LC -CDR3 comprising the amino acid sequence of SEQ ID NO: 136, or a variant thereof comprising up to about 3 (such as 3, 2, or 1) amino acid substitutions; o) the V_{H} comprises the HC -CDR1 comprising the amino acid sequence of SEQ ID NO: 211, or a variant thereof comprising up to about 3 (such as 3, 2, or 1) amino acid substitutions, the HC -CDR2 comprising the amino acid sequence of SEQ ID NO: 212, or a variant thereof comprising up to about 3 (such as 3, 2, or 1) amino acid substitutions, and the HC -CDR3 comprising the amino acid sequence of SEQ ID NO: 213, or a variant thereof comprising up to about 3 (such as 3, 2, or 1) amino acid substitutions, and the V_{L} comprises the LC -CDR1 comprising the amino acid sequence of SEQ ID NO: 214, or a variant thereof comprising up to about 3 (such as 3, 2, or 1) amino acid substitutions, the LC -CDR2 comprising the amino acid sequence of SEQ ID NO: 215, or a variant thereof comprising up to about 3 (such as 3, 2, or 1) amino acid substitutions, and the LC -CDR3 comprising the amino acid sequence of SEQ ID NO: 216, or a variant thereof comprising up to about 3 (such as 3, 2, or 1) amino acid substitutions; and p) the V_{H} comprises the HC -CDR1 comprising the amino acid sequence of SEQ ID NO: 221, or a variant thereof comprising up to about 3 (such as 3, 2, or 1) amino acid substitutions, the HC -CDR2 comprising the amino acid sequence of SEQ ID NO: 222, or a variant thereof comprising up to about 3 (such as 3, 2, or 1) amino acid substitutions, and the HC -CDR3 comprising the amino acid sequence of SEQ ID NO: 223, or a variant thereof comprising up to about 3 (such as 3, 2, or 1) amino acid substitutions, and the V_{L} comprises the LC -CDR1 comprising the amino acid sequence of SEQ ID NO: 224, or a variant thereof comprising up to about 3 (such as 3, 2, or 1) amino acid substitutions, the LC -CDR2 comprising the amino acid sequence of SEQ ID NO: 225, or a variant thereof comprising up to about 3 (such as 3, 2, or 1) amino acid substitutions, and the LC -CDR3 comprising the amino acid sequence of SEQ ID NO: 226, or a variant thereof comprising up to about 3 (such as 3, 2, or 1) amino acid substitutions.

In some embodiments, there is provided an anti-CD137 construct (*e.g*., an anti-CD137 scFv) comprising an anti-CD137 antibody moiety that binds to CD137, comprising a heavy chain variable region (V_{H}) and a light chain variable region (V_{L}), wherein: a) the V_{H} comprises: i) an HC-CDR1 comprising an amino acid sequence of DTYIH or GFNIQDT, ii) an HC-CDR2 comprising an amino acid sequence of DPANGN, and iii) an HC-CDR3 comprising an amino acid sequence of GNLHYALMD; and b) the V_{L} comprises: i) an LC-CDR1 comprising an amino acid sequence of NTYLS, ii) an LC-CDR2 comprising an amino acid sequence of RVNRKV, and iii) an LC-CDR3 comprising an amino acid sequence of LQYLDFPY.

In some embodiments, there is provided an anti-CD137 construct (*e.g*., an anti-CD137 scFv) comprising an anti-CD137 antibody moiety that binds to CD137, comprising: a) an HC-CDR1, an HC-CDR2, and an HC-CDR3, respectively comprising the amino acid sequences of a CDR1, a CDR2, and a CDR3 within a V_{H} chain region having the sequence set forth in SEQ ID No: 7, 17, 27, 37, 47, 57, 67, 77, 87, 97, 107, 117, 127, 137, 217 and 227; and b) a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 8, 18, 28, 38, 48, 58, 68, 78, 88, 98, 108, 118, 128, 138, 218 and 228.

In some embodiments, there is provided an anti-CD137 construct (*e.g*., an anti-CD137 scFv) comprising an anti-CD137 antibody moiety that binds to CD137, comprising a) a V_{H} chain region having the sequence set forth in SEQ ID No: 7, 17, 27, 37, 47, 57, 67, 77, 87, 97, 107, 117, 127, 137, 217 and 227, or a variant thereof having at least about 80% (such as at least about any one of 80%, 85%, 90%, 95%, 96%, 97%, 98%, or 99%) sequence identity to SEQ ID NO: 7, 17, 27, 37, 47, 57, 67, 77, 87, 97, 107, 117, 127, 137, 217 and 227, and b) a V_{L} chain region having the sequence set forth in SEQ ID No: 8, 18, 28, 38, 48, 58, 68, 78, 88, 98, 108, 118, 128, 138, 218 and 228, or a variant thereof having at least about 80% (such as at least about any one of 80%, 85%, 90%, 95%, 96%, 97%, 98%, or 99%) sequence identity to SEQ ID NO: 8, 18, 28, 38, 48, 58, 68, 78, 88, 98, 108, 118, 128, 138, 218 and 228. In some embodiments, the V_{H} chain region and the V_{L} chain region are linked *via* a linker (*e.g.,* peptide linker).

In some embodiments, there is provided an anti-CD137 construct (*e.g.*, an anti-CD137 scFv) comprising an anti-CD137 antibody moiety that binds to CD137, comprising a V_{H} region having the sequence set forth in SEQ ID No: 7, 17, 27, 37, 47, 57, 67, 77, 87, 97, 107, 117, 127, 137, 217 and 227; and a V_{L} region having the sequence set forth in SEQ ID No: 8, 18, 28, 38, 48, 58, 68, 78, 88, 98, 108, 118, 128, 138, 218 and 228.

In some embodiments according to any one of the anti-CD 137 constructs described herein, the anti-CD 137 antibody moiety comprises (a) a heavy chain variable region comprising amino acids having the sequence set forth in SEQ ID NO: 7; and a light chain variable region comprising amino acids having the sequence set forth in SEQ ID NO: 8; (b) a heavy chain variable region comprising amino acids having the sequence set forth in SEQ ID NO: 17; and a light chain variable region comprising amino acids having the sequence set forth in SEQ ID NO: 18; (c) a heavy chain variable region comprising amino acids having the sequence set forth in SEQ ID NO: 27; and a light chain variable region comprising amino acids having the sequence set forth in SEQ ID NO: 28; (d) a heavy chain variable region comprising amino acids having the sequence set forth in SEQ ID NO: 37; and a light chain variable region comprising amino acids having the sequence set forth in SEQ ID NO: 38; (e) a heavy chain variable region comprising amino acids having the sequence set forth in SEQ ID NO: 47; and a light chain variable region comprising amino acids having the sequence set forth in SEQ ID NO: 48; (f) a heavy chain variable region comprising amino acids having the sequence set forth in SEQ ID NO: 57; and a light chain variable region comprising amino acids having the sequence set forth in SEQ ID NO: 58; (g) a heavy chain variable region comprising amino acids having the sequence set forth in SEQ ID NO: 67; and a light chain variable region comprising amino acids having the sequence set forth in SEQ ID NO: 68; (h) a heavy chain variable region comprising amino acids having the sequence set forth in SEQ ID NO: 77; and a light chain variable region comprising amino acids having the sequence set forth in SEQ ID NO: 78; (i) a heavy chain variable region comprising amino acids having the sequence set forth in SEQ ID NO: 87; and a light chain variable region comprising amino acids having the sequence set forth in SEQ ID NO: 88; (j) a heavy chain variable region comprising amino acids having the sequence set forth in SEQ ID NO: 97; and a light chain variable region comprising amino acids having the sequence set forth in SEQ ID NO: 98; (k) a heavy chain variable region comprising amino acids having the sequence set forth in SEQ ID NO: 107; and a light chain variable region comprising amino acids having the sequence set forth in SEQ ID NO: 108; (l) a heavy chain variable region comprising amino acids having the sequence set forth in SEQ ID NO: 117; and a light chain variable region comprising amino acids having the sequence set forth in SEQ ID NO: 118; (m) a heavy chain variable region comprising amino acids having the sequence set forth in SEQ ID NO: 127; and a light chain variable region comprising amino acids having the sequence set forth in SEQ ID NO: 128; or (n) a heavy chain variable region comprising amino acids having the sequence set forth in SEQ ID NO: 137; and a light chain variable region comprising amino acids having the sequence set forth in SEQ ID NO: 138; (o) a heavy chain variable region comprising amino acids having the sequence set forth in SEQ ID NO: 217; and a light chain variable region comprising amino acids having the sequence set forth in SEQ ID NO: 218; or (p) a heavy chain variable region comprising amino acids having the sequence set forth in SEQ ID NO: 227; and a light chain variable region comprising amino acids having the sequence set forth in SEQ ID NO: 228.

In some embodiments, there is provided an anti-CD137 construct (*e.g*., an anti-CD137 scFv) comprising an anti-CD137 antibody moiety that binds to CD137, comprising a heavy chain (HC) having the sequence set forth in SEQ ID No: 9, 19, 29, 39, 49, 59, 69, 79, 89, 99, 109, 119, 129, 139, 149, 219 and 229; and a light chain (LC) having the sequence set forth in SEQ ID No: 10, 20, 30, 40, 50, 60, 70, 80, 90, 100, 110, 120, 130, 140, 150, 220 and 230.

In some embodiments according to any one of the anti-CD 137 constructs described herein, the anti-CD 137 antibody moiety comprises a heavy chain comprising amino acids having the sequence set forth in SEQ ID NO: 9; and a light chain comprising amino acids having the sequence set forth in SEQ ID NO: 10. In some embodiments according to any one of the anti-CD137 constructs described herein, the anti-CD137 antibody moiety comprises a heavy chain comprising amino acids having the sequence set forth in SEQ ID NO: 19; and a light chain comprising amino acids having the sequence set forth in SEQ ID NO: 20. In some embodiments according to any one of the anti-CD137 constructs described herein, the anti-CD137 antibody moiety comprises a heavy chain comprising amino acids having the sequence set forth in SEQ ID NO: 29; and a light chain comprising amino acids having the sequence set forth in SEQ ID NO: 30. In some embodiments according to any one of the anti-CD137 constructs described herein, the anti-CD137 antibody moiety comprises a heavy chain comprising amino acids having the sequence set forth in SEQ ID NO: 39; and a light chain comprising amino acids having the sequence set forth in SEQ ID NO: 40. In some embodiments according to any one of the anti-CD137 constructs described herein, the anti-CD137 antibody moiety comprises a heavy chain comprising amino acids having the sequence set forth in SEQ ID NO: 49; and a light chain comprising amino acids having the sequence set forth in SEQ ID NO: 50. In some embodiments according to any one of the anti-CD137 constructs described herein, the anti-CD137 antibody moiety comprises a heavy chain comprising amino acids having the sequence set forth in SEQ ID NO: 59; and a light chain comprising amino acids having the sequence set forth in SEQ ID NO: 60. In some embodiments according to any one of the anti-CD137 constructs described herein, the anti-CD137 antibody moiety comprises a heavy chain comprising amino acids having the sequence set forth in SEQ ID NO: 69; and a light chain comprising amino acids having the sequence set forth in SEQ ID NO: 70. In some embodiments according to any one of the anti-CD137 constructs described herein, the anti-CD137 antibody moiety comprises a heavy chain comprising amino acids having the sequence set forth in SEQ ID NO: 79; and a light chain comprising amino acids having the sequence set forth in SEQ ID NO: 80. In some embodiments according to any one of the anti-CD137 constructs described herein, the anti-CD137 antibody moiety comprises a heavy chain comprising amino acids having the sequence set forth in SEQ ID NO: 89; and a light chain comprising amino acids having the sequence set forth in SEQ ID NO: 90. In some embodiments according to any one of the anti-CD137 constructs described herein, the anti-CD137 antibody moiety comprises a heavy chain comprising amino acids having the sequence set forth in SEQ ID NO: 99; and a light chain comprising amino acids having the sequence set forth in SEQ ID NO: 100. In some embodiments according to any one of the anti-CD137 constructs described herein, the anti-CD137 antibody moiety comprises a heavy chain comprising amino acids having the sequence set forth in SEQ ID NO: 109; and a light chain comprising amino acids having the sequence set forth in SEQ ID NO: 110. In some embodiments according to any one of the anti-CD137 constructs described herein, the anti-CD137 antibody moiety comprises a heavy chain comprising amino acids having the sequence set forth in SEQ ID NO: 119; and a light chain comprising amino acids having the sequence set forth in SEQ ID NO: 120. In some embodiments according to any one of the anti-CD137 constructs described herein, the anti-CD137 antibody moiety comprises a heavy chain comprising amino acids having the sequence set forth in SEQ ID NO: 129; and a light chain comprising amino acids having the sequence set forth in SEQ ID NO: 130. In some embodiments according to any one of the anti-CD137 constructs described herein, the anti-CD137 antibody moiety comprises a heavy chain comprising amino acids having the sequence set forth in SEQ ID NO: 139; and a light chain comprising amino acids having the sequence set forth in SEQ ID NO: 140. In some embodiments according to any one of the anti-CD137 constructs described herein, the anti-CD137 antibody moiety comprises a heavy chain comprising amino acids having the sequence set forth in SEQ ID NO: 149; and a light chain comprising amino acids having the sequence set forth in SEQ ID NO: 150. In some embodiments according to any one of the anti-CD137 constructs described herein, the anti-CD137 antibody moiety comprises a heavy chain comprising amino acids having the sequence set forth in SEQ ID NO: 219; and a light chain comprising amino acids having the sequence set forth in SEQ ID NO: 220. In some embodiments according to any one of the anti-CD137 constructs described herein, the anti-CD137 antibody moiety comprises a heavy chain comprising amino acids having the sequence set forth in SEQ ID NO: 229; and a light chain comprising amino acids having the sequence set forth in SEQ ID NO: 230.

Another aspect of the present application provides isolated anti-CD137 constructs comprising an antibody moiety that binds to CD137, comprising a heavy chain variable region (V_{H}) and a light chain variable region (V_{L}), wherein: a) the V_{H} comprises: i) an HC-CDR1 comprising an amino acid sequence of any one of SEQ ID NOs: 151-153, or a variant thereof comprising up to about 3 amino acid substitutions; ii) an HC-CDR2 comprising an amino acid sequence of any one of SEQ ID NOs: 154-156, or a variant thereof comprising up to about 3 amino acid substitutions; iii) an HC-CDR3 comprising an amino acid sequence of any one of SEQ ID NOs: 157-159, or a variant thereof comprising up to about 3 amino acid substitutions; and b) the V_{L} comprises: i) an LC-CDR1 comprising an amino acid sequence of any one of SEQ ID NOs: 160-163, or a variant thereof comprising up to about 3 amino acid substitutions; ii) an HC-CDR2 comprising an amino acid sequence of any one of SEQ ID NOs: 164-166, or a variant thereof comprising up to about 3 amino acid substitutions; iii) an HC-CDR3 comprising an amino acid sequence of any one of SEQ ID NOs: 167-169, or a variant thereof comprising up to about 3 amino acid substitutions.

In some embodiments, the V_{H} comprises the HC-CDR1 comprising the amino acid sequence of SEQ ID NO: 151, an HC-CDR2 comprising the amino acid sequence of SEQ ID NO: 154, and the HC-CDR3 comprising the amino acid sequence of SEQ ID NO: 157, and the V_{L} comprises the LC-CDR1 comprising the amino acid sequence of SEQ ID NO: 160, an LC-CDR2 comprising the amino acid sequence of SEQ ID NO: 164, and the LC-CDR3 comprising the amino acid sequence of SEQ ID NO: 167.

In some embodiments, the V_{H} comprises the HC-CDR1 comprising the amino acid sequence of SEQ ID NO: 151, an HC-CDR2 comprising the amino acid sequence of SEQ ID NO: 154, and the HC-CDR3 comprising the amino acid sequence of SEQ ID NO: 157, and the V_{L} comprises the LC-CDR1 comprising the amino acid sequence of SEQ ID NO: 162, an LC-CDR2 comprising the amino acid sequence of SEQ ID NO: 166, and the LC-CDR3 comprising the amino acid sequence of SEQ ID NO: 169.

In some embodiments, the V_{H} comprises the HC-CDR1 comprising the amino acid sequence of SEQ ID NO: 152, an HC-CDR2 comprising the amino acid sequence of SEQ ID NO: 155, and the HC-CDR3 comprising the amino acid sequence of SEQ ID NO: 158, and the V_{L} comprises the LC-CDR1 comprising the amino acid sequence of SEQ ID NO: 163, an LC-CDR2 comprising the amino acid sequence of SEQ ID NO: 166, and the LC-CDR3 comprising the amino acid sequence of SEQ ID NO: 169,.

In some embodiments, the V_{H} comprises the HC-CDR1 comprising the amino acid sequence of SEQ ID NO: 153, an HC-CDR2 comprising the amino acid sequence of SEQ ID NO: 156, and the HC-CDR3 comprising the amino acid sequence of SEQ ID NO: 159, and the V_{L} comprises the LC-CDR1 comprising the amino acid sequence of SEQ ID NO: 160, an LC-CDR2 comprising the amino acid sequence of SEQ ID NO: 164, and the LC-CDR3 comprising the amino acid sequence of SEQ ID NO: 167.

In some embodiments, the V_{H} comprises the HC-CDR1 comprising the amino acid sequence of SEQ ID NO: 153, an HC-CDR2 comprising the amino acid sequence of SEQ ID NO: 156, and the HC-CDR3 comprising the amino acid sequence of SEQ ID NO: 159, and the V_{L} comprises the LC-CDR1 comprising the amino acid sequence of SEQ ID NO: 161, an LC-CDR2 comprising the amino acid sequence of SEQ ID NO: 165, and the LC-CDR3 comprising the amino acid sequence of SEQ ID NO: 168.

In some embodiments according to any one of the anti-CD 137 constructs described herein, the anti-CD 137 antibody moiety comprises (a) a heavy chain variable region comprising amino acids having the sequence set forth in SEQ ID NO: 170; and a light chain variable region comprising amino acids having the sequence set forth in SEQ ID NO: 173; (b) a heavy chain variable region comprising amino acids having the sequence set forth in SEQ ID NO: 170; and a light chain variable region comprising amino acids having the sequence set forth in SEQ ID NO: 176; (c) a heavy chain variable region comprising amino acids having the sequence set forth in SEQ ID NO: 171; and a light chain variable region comprising amino acids having the sequence set forth in SEQ ID NO: 173; (d) a heavy chain variable region comprising amino acids having the sequence set forth in SEQ ID NO: 171; and a light chain variable region comprising amino acids having the sequence set forth in SEQ ID NO: 174; or (e) a heavy chain variable region comprising amino acids having the sequence set forth in SEQ ID NO: 172; and a light chain variable region comprising amino acids having the sequence set forth in SEQ ID NO: 175.

In some embodiments, the anti-CD137 construct comprises or is an antibody or antigen-binding fragment thereof selected from the group consisting of a full-length antibody, a multispecific antibody, a single-chain Fv (scFv), a Fab fragment, a Fab' fragment, a F(ab')2, an Fv fragment, a disulfide stabilized Fv fragment (dsFv), a (dsFv)₂, a V_{H}H, a Fv-Fc fusion, a scFv-Fc fusion, a scFv-Fv fusion, a diabody, a tribody, and a tetrabody. In some embodiments, the antibody or antigen-binding fragment is chimeric, human, partially humanized, fully humanized, or semi-synthetic. In some embodiments, the antibody or antigen-binding fragment thereof comprises an immunoglobulin isotype selected from the group consisting of an IgG, an IgM, an IgA, an IgD, and an IgE. In some embodiments, the antibody or antigen-binding fragment thereof has an isotype selected from the group consisting of an IgG1, an IgG2, an IgG3, or IgG4.

In some embodiments, the construct comprises a humanized anti-CD137 full-length antibody.

In some embodiments, the construct comprises a humanized anti-CD137 single chain Fv fragment.

In some embodiments, the construct binds to a human CD137. In some embodiments, the construct binds to a mammal CD137 (such as a monkey CD137). In some embodiments, the construct binds to both a human CD137 and a monkey CD137. In some embodiments, the construct does not bind to a mouse CD137.

As disclosed above, an anti-CD137 construct disclosed herein comprises an anti-CD137 antibody moiety that binds to CD137. In some embodiments, anti-CD137 antibody moiety binds to human and simian CD137.

In some embodiments, anti-CD137 antibody moiety comprised in an anti-CD137 construct disclosed herein is a CD137 agonist, wherein the binding of the antibody moiety to CD137 can enhance an immune signaling pathway mediated by CD137. In some embodiments, the binding of the antibody moiety to CD137 cannot enhance an immune signaling pathway mediated by CD137 without cross-linking and/or clustering of the antibody moiety/CD137 complex. In some embodiments, the binding of the antibody moiety to CD137 can activate an immune cell, e.g., a T cell and/or a NK cell. In some embodiments, the binding of the antibody moiety to CD137 cannot activate an immune cell, e.g., a T cell and/or a NK cell without cross-linking and/or clustering of the antibody moiety/CD 137 complex. In some embodiments, the cross-linking and/or clustering of the antibody moiety/CD137 complex can be mediated by a second moiety of the anti-CD137 construct disclosed herein. In some embodiments, the cross-linking and/or clustering of the antibody moiety/CD137 complex can be mediated by the binding of a Fc receptor to a Fc region of the anti-CD137 construct. In some embodiments, the cross-linking and/or clustering of the antibody moiety/CD137 complex can be mediated by the binding of a second antibody moiety of the anti-CD137 construct to a tumor associated antigen (TAA).

In some embodiments, the antibody moiety comprises an Fc fragment selected from the group consisting of Fc fragments from IgG, IgA, IgD, IgE, IgM, and any combinations and hybrids thereof. In some embodiments, the Fc fragment is derived from a human IgG. In some embodiments, the Fc fragment comprises the Fc region of human IgG1, IgG2, IgG3, IgG4, or a combination or hybrid IgG. In some embodiments, the Fc fragment is an IgG1 Fc fragment. In some embodiments, the Fc fragment comprises the CH2 and CH3 domains of IgG1. In some embodiments, the Fc fragment is an IgG2 Fc fragment. In some embodiments, the Fc fragment comprises the CH2 and CH3 domains of IgG2. In some embodiments, the Fc fragment is an IgG4 Fc fragment. In some embodiments, the Fc fragment comprises the CH2 and CH3 domains of IgG4. IgG4 Fc is known to exhibit less effector activity than IgG1 or IgG2 Fc, and thus may be desirable for some applications. In some embodiments, the Fc fragment is derived from of a mouse immunoglobulin.

In some embodiments, the antibody moiety comprises an Fc fragment. In some embodiments, the antibody moiety is an scFv fused to an Fc fragment. In some embodiments, the antibody moiety comprises a scFv fused to an Fc fragment *via* a peptide linker. In some embodiments, the Fc fragment is a human IgG1 Fc fragment. In some embodiments, the Fc fragment comprises one or more mutations to increase clearance or decrease half-life.

In some embodiments, the Fc fragment comprises an immunoglobulin IgG heavy chain constant region comprising a hinge region (starting at Cys226), an IgG CH2 domain and CH3 domain. The term "hinge region" or "hinge sequence" as used herein refers to the amino acid sequence located between the linker and the CH2 domain. In some embodiments, the fusion protein comprises an Fc fragment comprising a hinge region. In some embodiments, the Fc fragment of the fusion protein starts at the hinge region and extends to the C-terminus of the IgG heavy chain. In some embodiments, the fusion protein comprises an Fc fragment that does not comprise the hinge region.

In some embodiments, the IgG CH2 domain starts at Ala231. In some embodiments, the CH3 domain starts at Gly341. It is understood that the C-terminus Lys residue of human IgG can be optionally absent. It is also understood that conservative amino acid substitutions of the Fc region without affecting the desired structure and/or stability of Fc is contemplated within the scope of the invention.

In some embodiments, each chain of the Fc fragment is fused to the same antibody moiety. In some embodiments, the scFv-Fc comprises two identical scFvs described herein, each fused with one chain of the Fc fragment. In some embodiments, the scFv-Fc is a homodimer.

In some embodiments, the scFv-Fc comprises two different scFvs, each fused with one chain of the Fc fragment. In some embodiments, the scFv-Fc is a heterodimer. Heterodimerization of non-identical polypeptides in the scFv-Fc can be facilitated by methods known in the art, including without limitation, heterodimerization by the knob-into-hole technology. The structure and assembly method of the knob-into-hole technology can be found in, *e.g.,* US5,821,333, US7,642,228, US 201 1/0287009 and PCT/US2012/059810, hereby incorporated by reference in their entireties. This technology was developed by introducing a "knob" (or a protuberance) by replacing a small amino acid residue with a large one in the CH3 domain of one Fc and introducing a "hole" (or a cavity) in the CH3 domain of the other Fc by replacing one or more large amino acid residues with smaller ones. In some embodiments, one chain of the Fc fragment in the fusion protein comprises a knob, and the second chain of the Fc fragment comprises a hole.

The preferred residues for the formation of a knob are generally naturally occurring amino acid residues and are preferably selected from arginine (R), phenylalanine (F), tyrosine (Y) and tryptophan (W). Most preferred are tryptophan and tyrosine. In one embodiment, the original residue for the formation of the knob has a small side chain volume, such as alanine, asparagine, aspartic acid, glycine, serine, threonine or valine. Exemplary amino acid substitutions in the CH3 domain for forming the knob include without limitation the T366W, T366Y or F405W substitution.

The preferred residues for the formation of a hole are usually naturally occurring amino acid residues and are preferably selected from alanine (A), serine (S), threonine (T) and valine (V). In one embodiment, the original residue for the formation of the hole has a large side chain volume, such as tyrosine, arginine, phenylalanine or tryptophan. Exemplary amino acid substitutions in the CH3 domain for generating the hole include without limitation the T366S, L368A, F405A, Y407A, Y407T and Y407V substitutions. In certain embodiments, the knob comprises T366W substitution, and the hole comprises the T366S/L368A/Y407V substitutions. It is understood that other modifications to the Fc region known in the art that facilitate heterodimerization are also contemplated and encompassed by the instant application.

Other scFv-Fc variants (including variants of isolated anti-CD137 scFv-Fc, *e.g.,* a full-length anti-CD 137 antibody variants) comprising any of the variants described herein (*e.g.,* Fc variants, effector function variants, glycosylation variants, cysteine engineered variants), or combinations thereof, are contemplated.

### a) Antibody affinity

Binding specificity of the antibody moieties can be determined experimentally by methods known in the art. Such methods comprise, but are not limited to Western blots, ELISA-, RIA-, ECL-, IRMA-, EIA-, BIACORE^{™} -tests and peptide scans.

In some embodiments, the K_{D} of the binding between the antibody moiety and CD137 is about 10⁻⁷ M to about 10⁻¹² M, about 10⁻⁷ M to about 10⁻⁸ M, about 10⁻⁸ M to about 10⁻⁹ M, about 10⁻⁹ M to about 10⁻¹⁰ M, about 10⁻¹⁰ M to about 10⁻¹¹ M, about 10⁻¹¹ M to about 10⁻¹² M, about 10⁻⁷ M to about 10⁻¹² M, about 10⁻⁸ M to about 10⁻¹² M, about 10⁻⁹ M to about 10⁻¹² M, about 10⁻¹⁰ M to about 10⁻¹² M, about 10⁻⁷ M to about 10⁻¹¹ M, about 10⁻⁸ M to about 10⁻¹¹ M, about 10⁻⁹ M to about 10⁻¹¹ M, about 10⁻⁷ M to about 10⁻¹⁰ M, about 10⁻⁸ M to about 10⁻¹⁰ M, or about 10⁻⁷ M to about 10⁻⁹ M. In some embodiments, the K_{D} of the binding between the antibody moiety and CD137 is stronger than about any one of 10⁻⁷ M, 10⁻⁸ M, 10⁻⁹ M, 10⁻¹⁰ M, 10⁻¹¹ M, or 10⁻¹² M. In some embodiments, CD137 is human CD137.

In some embodiments, the Kₒₙ of the binding between the antibody moiety and CD137 is about 10³ M⁻¹s⁻¹ to about 10⁸ M⁻¹s⁻¹, about 10³ M⁻¹s⁻¹ to about 10⁴ M⁻¹s⁻¹, about 10⁴ M⁻¹s⁻¹ to about 10⁵ M⁻¹s⁻¹, about 10⁵ M⁻¹s⁻¹ to about 10⁶ M⁻¹s⁻¹, about 10⁶ M⁻¹s⁻¹ to about 10⁷ M⁻¹s⁻¹, or about 10⁷ M⁻¹s⁻¹ to about 10⁸ M⁻¹s⁻¹. In some embodiments, the Kₒₙ of the binding between the antibody moiety and CD137 is about 10³ M⁻¹s⁻¹ to about 10⁵ M⁻¹s⁻¹, about 10⁴ M⁻¹s⁻¹ to about 10⁶ M⁻¹s⁻¹, about 10⁵ M⁻¹s⁻¹ to about 10⁷ M⁻¹s⁻¹, about 10⁶ M⁻¹s⁻¹ to about 10⁸ M⁻¹s⁻¹, about 10⁴ M⁻¹s⁻¹ to about 10⁷ M⁻¹s⁻¹, or about 10⁵ M⁻¹s⁻¹ to about 10⁸ M⁻¹s⁻¹. In some embodiments, the Kₒₙ of the binding between the antibody moiety and CD137 is no more than about any one of 10³ M⁻¹s⁻¹, 10⁴ M⁻¹s⁻¹, 10⁵ M⁻¹s⁻¹, 10⁶ M⁻¹s⁻¹, 10⁷ M⁻¹s⁻¹ or 10⁸ M⁻¹s⁻¹. In some embodiments, CD137 is human CD137.

In some embodiments, the K_{off} of the binding between the antibody moiety and CD137 is about 1 s⁻¹ to about 10⁻⁶ s⁻¹, about 1 s⁻¹ to about 10⁻² s⁻¹, about 10⁻² s⁻¹ to about 10⁻³ s⁻¹, about 10⁻³ s⁻¹ to about 10⁻⁴ s⁻¹, about 10^{-4 1} to about 10⁻⁵ s⁻¹, about 10⁻⁵ s⁻¹ to about 10⁻⁶ s⁻¹, about 1 s⁻¹ to about 10⁻⁵ s⁻¹, about 10⁻² s⁻¹ to about 10⁻⁶ s⁻¹, about 10⁻³ s⁻¹ to about 10⁻⁶ s⁻¹, about 10⁻⁴ s⁻¹ to about 10⁻⁶ s⁻¹, about 10⁻² s⁻¹ to about 10⁻⁵ s⁻¹, or about 10⁻³ s⁻¹ to about 10⁻⁵ s⁻¹. In some embodiments, the K_{off} of the binding between the antibody moiety and CD137 is at least about any one of 1 s⁻¹, 10⁻² s⁻¹, 10⁻³ s⁻¹, 10⁻⁴ s⁻¹, 10⁻⁵ s⁻¹ or 10⁻⁶ s⁻¹. In some embodiments, CD137 is human CD137.

In some embodiments, the binding affinity of the anti-CD137 antibody moieties or anti-CD137 constructs are higher (for example, has a smaller Kd value) than an existing anti-CD137 antibody (e.g., anti-human CD137 antibody such as BMS-663513 (urelumab) or PF-05082566 (utomilumab)).

### b) Chimeric or humanized antibodies

In some embodiments, the antibody moiety is a chimeric antibody. Certain chimeric antibodies are described, *e.g.,* in U.S. Patent No. 4,816,567; and Morrison et al., Proc. Natl. Acad. Sci. USA, 81:6851-6855 (1984)). In some embodiments, a chimeric antibody comprises a non-human variable region (*e.g.,* a variable region derived from mouse) and a human constant region. In some embodiments, a chimeric antibody is a "class switched" antibody in which the class or subclass has been changed from that of the parent antibody. Chimeric antibodies include antigen-binding fragments thereof.

In some embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, *e.g.*, CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), *e.g.,* to restore or improve antibody specificity or affinity.

Humanized antibodies and methods of making them are reviewed, *e.g*., in Almagro and Fransson, Front. Biosci. 13:1619-1633 (2008), and are further described, *e.g.,* in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (*see, e.g.,* Sims et al. J. Immunol. 151:2296 (1993)); Framework regions derived from the consensus sequence of human antibodies of a particular subgroup of light or heavy chain variable regions (see, *e.g.,* Carter et al. Proc. Natl. Acad. Sci. USA, 89:4285 (1992); and Presta et al. J. Immunol., 151:2623 (1993)); human mature (somatically mutated) framework regions or human germline framework regions (see, *e.g.,* Almagro and Fransson, Front. Biosci. 13:1619-1633 (2008)); and framework regions derived from screening FR libraries (see, *e.g.,* Baca et al., J. Biol. Chem. 272:10678-10684 (1997) and Rosok et al., J. Biol. Chem. 271:22611-22618 (1996)).

### c) Human antibodies

In some embodiments, the antibody moiety is a human antibody (known as human domain antibody, or human DAb). Human antibodies can be produced using various techniques known in the art. Human antibodies are described generally in van Dijk and van de Winkel, Curr. Opin. Pharmacol. 5: 368-74 (2001), Lonberg, Curr. Opin. Immunol. 20:450-459 (2008), and Chen, Mol. Immunol. 47(4):912-21 (2010). Transgenic mice or rats capable of producing fully human single-domain antibodies (or DAb) are known in the art. See, *e*.*g*., US20090307787A1, U.S. Pat. No. 8,754,287, US20150289489A1, US20100122358A1, and WO2004049794.

Human antibodies (*e*.*g*., human DAbs) may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). *See also, e*.*g*., U.S. Patent Nos. 6,075,181 and 6,150,584 describing XENOMOUSE^{™} technology; U.S. Patent No. 5,770,429 describing HuMab^{®} technology; U.S. Patent No. 7,041,870 describing K-M MOUSE^{®} technology, and U.S. Patent Application Publication No. US 2007/0061900, describing VelociMouse^{®} technology). Human variable regions from intact antibodies generated by such animals may be further modified, *e.g.,* by combining with a different human constant region.

Human antibodies (e.g., human DAbs) can also be made by hybridoma-based methods. Human myeloma and mouse-human heteromyeloma cell lines for the production of human monoclonal antibodies have been described (*See*, *e*.*g*., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991)). Human antibodies generated *via* human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies (e.g., human DAbs) may also be generated by isolating Fv clone variable domain sequences selected from human-derived phage display libraries. Such variable domain sequences may then be combined with a desired human constant domain. Techniques for selecting human antibodies from antibody libraries are described below.

### d) Library-derived antibodies

The antibody moieties may be isolated by screening combinatorial libraries for antibodies with the desired activity or activities. For example, a variety of methods are known in the art for generating phage display libraries and screening such libraries for antibodies possessing the desired binding characteristics. Such methods are reviewed, *e*.*g*., in Hoogenboom et al. in Methods in Molecular Biology 178:1-37 (O'Brien et al., ed., Human Press, Totowa, NJ, 2001) and further described, *e.g.,* in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004). Methods for constructing single-domain antibody libraries have been described, for example, see U.S. Pat. NO. 7371849.

In certain phage display methods, repertoires of V_{H} and V_{L} genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically displays antibody fragments, either as scFv fragments or as Fab fragments. Libraries from immunized sources provide high-affinity antibodies to the immunogen without the requirement of constructing hybridomas. Alternatively, the naive repertoire can be cloned (*e.g.,* from human) to provide a single source of antibodies to a wide range of non-self and also self-antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### e) Substitution, insertion, deletion and variants

In some embodiments, antibody variants having one or more amino acid substitutions are provided. Sites of interest for substitutional mutagenesis include the HVRs (or CDRs) and FRs. Conservative substitutions are shown in Table 2 under the heading of "Preferred substitutions." More substantial changes are provided in Table 2 under the heading of "exemplary substitutions," and as further described below in reference to amino acid side chain classes. Amino acid substitutions may be introduced into an antibody of interest and the products screened for a desired activity, *e*.*g*., retained/improved antigen binding, decreased immunogenicity, or improved ADCC or CDC.

**Table 2. Amino acid substitutions**

| **Original** | **Exemplary Substitutions** | **Preferred** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties: (1) hydrophobic: Norleucine, Met, Ala, Val, Leu, Ile; (2) neutral hydrophilic: Cys, Ser, Thr, Asn, Gln; (3) acidic: Asp, Glu; (4) basic: His, Lys, Arg; (5) residues that influence chain orientation: Gly, Pro; and (6) aromatic: Trp, Tyr, Phe.

Non-conservative substitutions will entail exchanging a member of one of these classes for another class.

One type of substitutional variant involves substituting one or more hypervariable region residues of a parent antibody (*e*.*g*., a humanized or human antibody). Generally, the resulting variant(s) selected for further study will have modifications (*e*.*g*., improvements) in certain biological properties (*e*.*g*., increased affinity, reduced immunogenicity) relative to the parent antibody and/or will have substantially retained certain biological properties of the parent antibody. An exemplary substitutional variant is an affinity matured antibody, which may be conveniently generated, *e*.*g*., using phage display-based affinity maturation techniques such as those described herein. Briefly, one or more HVR (or CDRs) residues are mutated and the variant antibodies displayed on phage and screened for a particular biological activity (e.g. binding affinity).

Alterations *(e.g.,* substitutions) may be made in HVRs (or CDRs), *e.g.,* to improve antibody affinity. Such alterations may be made in HVR (or CDRs) "hotspots," *i.e.,* residues encoded by codons that undergo mutation at high frequency during the somatic maturation process *(see, e.g.,* Chowdhury, MethodsMol. Biol. 207:179-196 (2008)), and/or SDRs (a-CDRs), with the resulting variant VH or VL being tested for binding affinity. Affinity maturation by constructing and reselecting from secondary libraries has been described, *e*.*g*., in Hoogenboom et al. in Methods in Molecular Biology 178:1-37 (O'Brien et al., ed., Human Press, Totowa, NJ, (2001)). In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR (or CDRs) residues *(e.g.,* 4-6 residues at a time) are randomized. HVR (or CDRs) residues involved in antigen binding may be specifically identified, *e*.*g*., using alanine scanning mutagenesis or modeling. CDR-H3 and CDR-L3 in particular are often targeted.

In some embodiments, substitutions, insertions, or deletions may occur within one or more HVRs (or CDRs) so long as such alterations do not substantially reduce the ability of the antibody to bind antigen. For example, conservative alterations (*e*.*g*., conservative substitutions as provided herein) that do not substantially reduce binding affinity may be made in HVRs (or CDRs). Such alterations may be outside of HVR "hotspots" or CDRs. In some embodiments of the variant V_{H}H sequences provided above, each HVR (or CDRs) either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (*e.g.,* for ADEPT) or a polypeptide which increases the serum half-life of the antibody.

### f) Glycosylation variants

In some embodiments, the antibody moiety is altered to increase or decrease the extent to which the construct is glycosylated. Addition or deletion of glycosylation sites to an antibody may be conveniently accomplished by altering the amino acid sequence such that one or more glycosylation sites is created or removed.

Where the antibody moiety comprises an Fc region (*e*.*g*., scFv-Fc), the carbohydrate attached thereto may be altered. Native antibodies produced by mammalian cells typically comprise a branched, biantennary oligosaccharide that is generally attached by an N-linkage to Asn297 of the C_{H}2 domain of the Fc region. *See, e.g.,* Wright et al. TIBTECH 15:26-32 (1997). The oligosaccharide may include various carbohydrates, *e*.*g*., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in the antibody moiety may be made in order to create antibody variants with certain improved properties.

In some embodiments, the antibody moiety has a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (*e.g.,* complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ± 3 amino acids upstream or downstream of position 297, *i*.*e*., between positions 294 and 300, due to minor sequence variations in antibodies. Such fucosylation variants may have improved ADCC function. *See, e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Patent Application No. US 2003/0157108 A1, Presta, L; and WO 2004/056312 A1, Adams et al.*,* especially at Example 11), and knockout cell lines, such as alpha-1,6-fucosyltransferase gene, *FUT8,* knockout CHO cells *(see, e.g.,* Yamane-Ohnuki et al. Biotech. Bioeng. 87: 614 (2004); Kanda, Y. et al., Biotechnol. Bioeng., 94(4):680-688 (2006); and WO2003/085107).

In some embodiments, the antibody moiety has bisected oligosaccharides, *e*.*g*., in which a biantennary oligosaccharide attached to the Fc region of the antibody is bisected by GlcNAc. Such antibody variants may have reduced fucosylation and/or improved ADCC function. Examples of such antibody variants are described, *e*.*g*., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, *e.g.,* in WO 1997/30087 (Patel et al.); WO 1998/58964 (Raju, S.); and WO 1999/22764 (Raju, S.).

### g) Fc region variants

In some embodiments, one or more amino acid modifications may be introduced into the Fc region of the antibody moiety (*e*.*g*., scFv-Fc), thereby generating an Fc region variant. The Fc region variant may comprise a human Fc region sequence (*e.g.,* a human IgG1, IgG2, IgG3 or IgG4 Fc region) comprising an amino acid modification (*e*.*g*. a substitution) at one or more amino acid positions.

In some embodiments, the Fc fragment possesses some but not all effector functions, which make it a desirable candidate for applications in which the half-life of the antibody moiety *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 2 on page 464 of Ravetch and Kinet, Annu. Rev. Immunol. 9:457-492 (1991). Non-limiting examples *of in vitro* assays to assess ADCC activity of a molecule of interest is described in U.S. Patent No. 5,500,362 (s*ee*, *e*.*g*. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (*see* Bruggemann, M. et al., J. Exp. Med. 166:1351-1361 (1987)). Alternatively, non-radioactive assays methods may be employed (*see,* for example, ACTI^{™} non-radioactive cytotoxicity assay for flow cytometry (CellTechnology, Inc. Mountain View, CA; and CytoTox 96^{®} non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. *See, e.g.,* C1q and C3c binding ELISA in WO 2006/029879 and WO 2005/100402. To assess complement activation, a CDC assay may be performed (*see,* for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half-life determinations can also be performed using methods known in the art (*see, e.g.,* Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. *(See, e.g.,* U.S. Patent No. 6,737,056; WO 2004/056312, and Shields et al., J. Biol. Chem. 9(2): 6591-6604 (2001).)

In some embodiments, the Fc fragment is an IgG1 Fc fragment. In some embodiments, the IgG1 Fc fragment comprises a L234A mutation and/or a L235A mutation. In some embodiments, the Fc fragment is an IgG2 or IgG4 Fc fragment. In some embodiments, the Fc fragment is an IgG4 Fc fragment comprising a S228P, F234A, and/or a L235A mutation.

In some embodiments, the antibody moiety comprises an Fc region with one or more amino acid substitutions which improve ADCC, *e*.*g*., substitutions at positions 298, 333, and/or 334 of the Fc region (EU numbering of residues).

In some embodiments, alterations are made in the Fc region that result in altered (*i.e.,* either improved or diminished) C1q binding and/or Complement Dependent Cytotoxicity (CDC), *e.g.,* as described in US Patent No. 6,194,551, WO 99/51642, and Idusogie et al. J. Immunol. 164: 4178-4184 (2000).

In some embodiments, the antibody moiety (*e*.*g*., scFv-Fc) variant comprising a variant Fc region comprising one or more amino acid substitutions which alters half-life and/or changes binding to the neonatal Fc receptor (FcRn). Antibodies with increased halflives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which alters binding of the Fc region to FcRn. Such Fc variants include those with substitutions at one or more of Fc region residues, *e*.*g*., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### h) Cysteine engineered antibody variants

In some embodiments, it may be desirable to create cysteine engineered antibody moieties, *e*.*g*., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In some embodiments, any one or more of the following residues may be substituted with cysteine: A118 (EU numbering) of the heavy chain; and S400 (EU numbering) of the heavy chain Fc region. Cysteine engineered antibody moieties may be generated as described, *e*.*g*., in U.S. Patent No. 7,521,541.

### i) Antibody derivatives

In some embodiments, the antibody moiety described herein may be further modified to comprise additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (*e*.*g*., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in diagnosis under defined conditions, *etc.*

In some embodiments, the antibody moiety may be further modified to comprise one or more biologically active protein, polypeptides or fragments thereof. "Bioactive" or "biologically active", as used herein interchangeably, means showing biological activity in the body to carry out a specific function. For example, it may mean the combination with a particular biomolecule such as protein, DNA, *etc.,* and then promotion or inhibition of the activity of such biomolecule. In some embodiments, the bioactive protein or fragments thereof include proteins and polypeptides that are administered to patients as the active drug substance for prevention of or treatment of a disease or condition, as well as proteins and polypeptides that are used for diagnostic purposes, such as enzymes used in diagnostic tests or *in vitro* assays, as well as proteins and polypeptides that are administered to a patient to prevent a disease such as a vaccine.

### j) Anti-CD137 scFv

The anti-CD 137 constructs in some embodiments are scFvs (hereinafter referred to as "anti-CD137 scFv") comprising an anti-CD137 antibody moiety described herein. The anti-CD137-scFv can comprise any one of the anti-CD137 antibody moieties described herein (*see* "anti-CD137 antibody moiety" section). In some embodiments, the anti-CD137 scFv has the configuration of (from N-terminus to C-terminus): V_{L}(CD137)-L-V_{H}(CD137). In some emobidments, the anti-CD137 scFv has the configuration of (from N-terminus to C-terminus): V_{H}(CD137)-L-V_{L}(CD137), L is a linker (such as peptide linker).

In some embodiments, the anti-CD137 scFv is chimeric, human, partially humanized, fully humanized, or semi-synthetic.

In some embodiments, the anti-CD137 VL and anti-CD137 VH in the scFv is linked *via* a linker (*e.g.,* a peptide linker). In some embodiments, the linker comprises from about four to about fifteen amino acids. In some embodiments, the linker is a GS linker. In some embodiments, the linker comprises a sequence of any one of SEQ ID Nos: 183-210.

### k) Anti-CD137 fusion proteins

The anti-CD137 constructs in some embodiments comprise an anti-CD137 antibody moiety and a half-life extending moiety. In some embodiments, the half-life extending moiety is an Fc fragment. In some embodiments, the half-life extending moiety is an albumin binding moiety (*e*.*g*., an albumin binding antibody moiety).

In some embodiments, the half-life extending moiety is an Fc fragment (such as any of the Fc fragments or variants thereof described herein). The term "Fc region," "Fc domain" or "Fc" refers to a C-terminal non-antigen binding region of an immunoglobulin heavy chain that contains at least a portion of the constant region. The term includes native Fc regions and variant Fc regions. In some embodiments, a human IgG heavy chain Fc region extends from Cys226 to the carboxyl-terminus of the heavy chain. However, the C-terminal lysine (Lys447) of the Fc region may or may not be present, without affecting the structure or stability of the Fc region. Unless otherwise specified herein, numbering of amino acid residues in the IgG or Fc region is according to the EU numbering system for antibodies, also called the EU index, as described in Kabat et al., Sequences of Proteins of Immunological Interest, 5th Ed. Public Health Service, National Institutes of Health, Bethesda, MD, 1991.

In some embodiments, the Fc fragment is selected from the group consisting of Fc fragments from IgG, IgA, IgD, IgE, IgM, and any combinations and hybrids thereof. In some embodiments, the Fc fragment is selected from the group consisting of Fc fragments from IgG1, IgG2, IgG3, IgG4, and any combinations and hybrids thereof.

In some embodiments, the Fc fragment has a reduced effector function as compared to corresponding wildtype Fc fragment (such as at least about 30%, 40%, 50%, 60%, 70%, 80%, 85%, 90%, or 95% reduced effector function as measured by the level of antibody-dependent cellular cytotoxicity (ADCC)).

In some embodiments, the Fc fragment is an IgG1 Fc fragment. In some embodiments, the IgG1 Fc fragment comprises a L234A mutation and/or a L235A mutation. In some embodiments, the Fc fragment is an IgG2 or IgG4 Fc fragment. In some embodiments, the Fc fragment is an IgG4 Fc fragment comprising a S228P, F234A, and/or a L235A mutation.

In some embodiments, the anti-CD 137 antibody moiety and the half-life extending moiety is linked *via* a linker (such as any of the linkers described in the "Linkers" section).

In some embodiments, the anti-CD137 fusion protein further comprises a second antibody. In some embodiments, the second antibody binds to a tumor antigen (such as any one of the tumor antigen described herein).

### l) Multispecific anti-CD137 molecules

The anti-CD137 constructs in some embodiments comprise a multi-specific (e.g., bispecific) anti-CD137 molecule comprising an anti-CD137 antibody moiety according to any one of the anti-CD137 antibody moieties described herein, and a second binding moiety (such as a second antibody moiety) specifically recognizing a second antigen. In some embodiments, the multi-specific anti-CD137 molecule comprises an anti-CD137 antibody moiety and a second antibody moiety specifically recognizing a second antigen. In some embodiments, the second antigen is a tumor associated antigen.

In some embodiments, there is provided multispecific antibody comprising: a) a first antigen-binding moiety comprising a construct described herein; and b) a second antigen-binding moiety that binds to a second antigen that is not CD137. In some embodiments, the second antigen comprises a tumor associated antigen.

Multi-specific molecules are molecules that have binding specificities for at least two different antigens or epitopes (e.g., bispecific antibodies have binding specificities for two antigens or epitopes). Multi-specific molecules with more than two valences and/or specificities are also contemplated. For example, trispecific antibodies can be prepared (Tutt et al. J. Immunol. 147: 60 (1991)). It is to be appreciated that one of skill in the art could select appropriate features of individual multi-specific molecules described herein to combine with one another to form a multi-specific anti-CD 137 molecule of the invention.

"Tumor associated antigen" as described herein refers to, for example, any antigen that is expressed significantly higher (such as at least about 5%, 10%, 20%, 30%, 40%, 50%, 60%, or 70% higher) by tumor cells (*e.g.,* cancer cells) than by non-tumor cells (*e.g.,* noncancer cells).

Exemplary tumor associated antigens that can be recognized by the second antibody moiety described herein include, but are not limited to, alpha fetoprotein (AFP), CA15-3, CA27-29, CA19-9, CA-125, calretinin, carcinoembryonic antigen, CD34, CD99, CD117, chromogranin, cytokeratin, desmin, epithelial membrane protein (EMA), Factor VIII, CD31 FL1, glial fibrillary acidic protein (GFAP), gross cystic disease fluid protein (GCDFP-15), HMB-45, human chorionic gonadotropin (hCG), inhibin, keratin, CD45, a lymphocyte marker, MART-1 (Melan-A), Myo Dl, muscle-specific actin (MSA), neurofilament, neuronspecific enolase (NSE), placental alkaline phosphatase (PLAP), prostate-specific antigen, S100 protein, smooth muscle actin (SMA), synaptophysin, thyroglobulin, thyroid transcription factor- 1, tumor M2-PK, and vimentin.

In some embodiments, the tumor associated antigen is selected from the group consisting of Her-2, EGFR, PD-L1, c-Met, B Cell Maturation Antigen (BCMA), carbonic anhydrase IX (CA1X), carcinoembryonic antigen (CEA), CD5, CD7, CD10, CD19, CD20, CD22, CD30, CD33, CD34, CD38, CD41, CD44, CD49f, CD56, CD74, CD123, CD133, CD138, CD276 (B7H3), epithelial glycoprotein (EGP2), trophoblast cell-surface antigen 2 (TROP-2), epithelial glycoprotein- 40 (EGP-40), epithelial cell adhesion molecule (EpCAM), receptor tyrosine-protein kinases erb-B2,3,4, folate-binding protein (FBP), fetal acetylcholine receptor (AChR), folate receptor-a, Ganglioside G2 (GD2), Ganglioside G3 (GD3), human telomerase reverse transcriptase (hTERT), kinase insert domain receptor (KDR), Lewis A (CA 1.9.9), Lewis Y (LeY), Glypican-3 (GPC3), L1 cell adhesion molecule (L1CAM), Mucin 16 (Muc-16), Mucin 1 (Muc-1), NG2D ligands, oncofetal antigen (h5T4), prostate stem cell antigen (PSCA), prostate-specific membrane antigen (PSMA), tumor- associated glycoprotein 72 (TAG-72), Claudin18.2 (CLDN18.2), vascular endothelial growth factor R2 (VEGF- R2), Wilms tumor protein (WT-1), type 1 tyrosine-protein kinase transmembrane receptor (ROR1) and any combination thereof. In some embodiments, the tumor associated antigen is Her-2, EGFR, B7H3, c-Met, or PD-L1. In some embodiments, the tumor associated antigen is selected from the group consisting of Her-2, EGFR, B7H3, c-Met, or PD-L1.

### m) Tandem scFv

The multi-specific anti-CD137 molecule in some embodiments is a tandem scFv comprising a first scFv comprising an anti-CD137 antibody moiety specifically recognizing CD137 (referred to herein as "anti-CD 137 scFv") and a second scFv specifically recognizing a second antigen (also referred to herein as a "tandem scFv multi-specific anti-CD137 antibody"). In some embodiments, the tandem scFv multi-specific anti-CD137 antibody further comprises at least one (such as at least about any of 2, 3, 4, 5, or more) additional scFv.

In some embodiments, there is provided a tandem scFv multi-specific (e.g., bispecific) anti-CD137 antibody comprising a) a first scFv specifically recognizing CD137, and b) a second scFv specifically recognizing a second antigen (*e.g.,* a tumor associated antigen), wherein the tandem scFv multi-specific anti-CD137 antibody is a tandem di-scFv or a tandem tri-scFv. In some embodiments, the tandem scFv multi-specific anti-CD137 antibody is a tandem di-scFv. In some embodiments, the tandem scFv multi-specific anti-CD137 antibody is a bispecific T-cell engager. In some embodiments, the second scFv binds to a different CD137 epitope. In some embodiments, the second scFv specifically recognizes a second antigen that is not CD137. In some embodiments, the second scFv specifically recognizes a second antigen such as a tumor associated antigen. In some embodiments, the first anti-CD137 scFv is chimeric, human, partially humanized, fully humanized, or semi-synthetic. In some embodiments, the second scFv is chimeric, human, partially humanized, fully humanized, or semi-synthetic. In some embodiments, both the first and second scFvs are chimeric, human, partially humanized, fully humanized, or semi-synthetic. In some embodiments, the tandem scFv multi-specific anti-CD 137 antibody further comprises at least one (such as at least about any of 2, 3, 4, 5, or more) additional scFv. In some embodiments, the first anti-CD 137 scFv and the second scFv are connected by a linker (*e.g.,* peptide linker). In some embodiments, the linker comprises the amino acid sequence of (GGGGS)ₙ, wherein n is equal to 1, 2, 3, 4, 5, 6, 7, 8, 9, 10 or more. In some embodiments, the linker comprises the amino acid sequence of TSGGGGS. In some embodiments, the first anti-CD137 scFv is N-terminal to the second scFv. In some embodiments, the first anti-CD137 scFv is C-terminal to the second scFv. In some embodiments, the tandem scFv multi-specific (e.g., bispecific) anti-CD137 antibody further comprises a tag (*e.g.,* a peptide tag for purification purpose). In some embodiments, the tag is N-terminal to the tandem scFv multi-specific (*e*.*g*., bispecific) anti-CD137 antibody. In some embodiments, the tag is C-terminal to the tandem scFv multispecific (*e*.*g*., bispecific) anti-CD137 antibody. In some embodiments, the tag comprises the amino acid sequence of HHHHHH

In some embodiments, the tandem scFv multi-specific anti-CD137 antibody is a tandem di-scFv comprising two scFvs (referred to herein as "tandem di-scFv bispecific anti-CD137 antibody"). The tandem di-scFv bispecific anti-CD137 antibody can have V_{H} and V_{L} assembled in any configurations, such as the configurations listed below (from N-terminus to C-terminus), wherein X is the second antigen specifically bound by the second scFv, L1, L2, and L3 are optional linkers (such as peptide linkers). *See* "Linkers" section for all applicable linkers. In some embodiments, the linker (L1, L2, or L3) comprises the amino acid sequence of SRGGGGSGGGGSGGGGSLEMA. In some embodiments, the linker (L1, L2, or L3) is or comprises a (GGGGS)ₙ sequence, wherein n is equal to 1, 2, 3, 4, 5, 6, 7, 8, 9, 10 or more. In some embodiments, the linker (L1, L2, or L3) comprises the amino acid sequence of TSGGGGS. In some embodiments, the linker (L1, L2, or L3) comprises the amino acid sequence of GEGTSTGSGGSGGSGGAD.
V_{L}(CD137)-L1-V_{H}(CD137)-L2-V_{L}(X)-L3-V_{H}(X);
V_{L}(CD137)-L1-V_{H}(CD137)-L2-V_{H}(X)-L3-V_{L}(X);
V_{H}(CD137)-L1-V_{L}(CD137)-L2-V_{L}(X)-L3-V_{H}(X);
V_{H}(CD137)-L1-V_{L}(CD137)-L₂-V_{H}(X)-L3-V_{L}(X);
V_{L}(X)-L1-V_{H}(X)-L2-V_{L}(CD137)-L3-V_{H}(CD137);
V_{L}(X)-L1-V_{H}(X)-L2-V_{H}(CD137)-L3-V_{L}(CD137);
V_{H}(X)-L1-V_{L}(X)-L2-V_{L}(CD137)-L3-V_{H}(CD137);
V_{H}(X)-L1-V_{L}(X)-L2-V_{H}(CD137)-L3-V_{L}(CD137);
V_{L}(CD137)-L1-V_{H}(X)-L2-V_{L}(X)-L3-V_{H}(CD137);
V_{L}(CD137)-L1-V_{L}(X)-L2-V_{H}(X)-L3-V_{H}(CD137);
V_{H}(CD137)-L1-V_{H}(X)-L2-V_{L}(X)-L3-V_{L}(CD137);
V_{H}(CD137)-L1-V_{L}(X)-L2-V_{H}(X)-L3-V_{L}(CD137);
V_{L}(X)-L1-V_{H}(CD137)-L2-V_{L}(CD137)-L3-V_{H}(X);
V_{L}(X)-L1-V_{L}(CD137)-L2-V_{H}(CD137)-L3-V_{H}(X);
V_{H}(X)-L1-V_{H}(CD137)-L2-V_{L}(CD137)-L3-V_{L}(X); or
V_{H}(X)-L1-V_{L}(CD137)-L2-V_{H}(CD137)-L3-V_{L}(X).

### n) Linkers

In some embodiments, the anti-CD137 constructs described herein comprise one or more linkers between two moieties (e.g., the anti-CD137 antibody moiety and the half-life extending moiety, the anti-CD137 scFv and the full length antibody in the multispecific antibodies described herein). The length, the degree of flexibility and/or other properties of the linker(s) used in the multispecific antibodies may have some influence on properties, including but not limited to the affinity, specificity or avidity for one or more particular antigens or epitopes. For example, longer linkers may be selected to ensure that two adjacent domains do not sterically interfere with one another. In some embodiment, a linker (such as peptide linker) comprises flexible residues (such as glycine and serine) so that the adjacent domains are free to move relative to each other. For example, a glycine-serine doublet can be a suitable peptide linker, e.g., a linker comprising an amino acid sequence of any one of SEQ ID Nos: 183-210 (or any combination thereof) in the SEQUENCE TABLE. In some embodiments, the linker is a non-peptide linker. In some embodiments, the linker is a peptide linker. In some embodiments, the linker is a non-cleavable linker. In some embodiments, the linker is a cleavable linker.

Other linker considerations include the effect on physical or pharmacokinetic properties of the resulting compound, such as solubility, lipophilicity, hydrophilicity, hydrophobicity, stability (more or less stable as well as planned degradation), rigidity, flexibility, immunogenicity, modulation of antibody binding, the ability to be incorporated into a micelle or liposome, and the like.

Coupling of two moieties may be accomplished by any chemical reaction that will bind the two molecules so long as both components retain their respective activities, e.g., binding to CD137 and a second antigen in a multispecific antibody, respectively. This linkage can include many chemical mechanisms, for instance covalent binding, affinity binding, intercalation, coordinate binding and complexation. In some embodiments, the binding is covalent binding. Covalent binding can be achieved either by direct condensation of existing side chains or by the incorporation of external bridging molecules. Many bivalent or polyvalent linking agents may be useful in coupling protein molecules in this context. For example, representative coupling agents can include organic compounds such as thioesters, carbodiimides, succinimide esters, diisocyanates, glutaraldehyde, diazobenzenes and hexamethylene diamines. This listing is not intended to be exhaustive of the various classes of coupling agents known in the art but, rather, is exemplary of the more common coupling agents (*see* Killen and Lindstrom, Jour. Immun. 133:1335-2549 (1984); Jansen et al., Immunological Reviews 62:185-216 (1982); and Vitetta et al., Science 238:1098 (1987)).

Linkers can be applied in the present application are described in the literature *(see, for example,* Ramakrishnan, S. et al., Cancer Res. 44:201-208 (1984) describing use of MBS (M-maleimidobenzoyl-N-hydroxysuccinimide ester). In some embodiments, non-peptide linkers used herein include: (i) EDC (1-ethyl-3-(3-dimethylamino-propyl) carbodiimide hydrochloride; (ii) SMPT (4-succinimidyloxycarbonyl-alpha-methyl-alpha-(2-pridyl-dithio)-toluene (Pierce Chem. Co., Cat. (21558G); (iii) SPDP (succinimidyl-6 [3-(2-pyridyldithio) propionamido] hexanoate (Pierce Chem. Co., Cat #21651G); (iv) Sulfo-LC-SPDP (sulfosuccinimidyl 6 [3-(2-pyridyldithio)-propianamide] hexanoate (Pierce Chem. Co. Cat. #2165-G); and (v) sulfo-NHS (N-hydroxysulfo-succinimide: Pierce Chem. Co., Cat. #24510) conjugated to EDC.

The linkers described herein contain components that have different attributes, thus may lead to multispecific antibodies with differing physio-chemical properties. For example, sulfo-NHS esters of alkyl carboxylates are more stable than sulfo-NHS esters of aromatic carboxylates. NHS-ester containing linkers are less soluble than sulfo-NHS esters. Further, the linker SMPT contains a sterically hindered disulfide bond, and can form antibody fusion protein with increased stability. Disulfide linkages, are in general, less stable than other linkages because the disulfide linkage is cleaved *in vitro,* resulting in less antibody fusion protein available. Sulfo-NHS, in particular, can enhance the stability of carbodimide couplings. Carbodimide couplings (such as EDC) when used in conjunction with sulfo-NHS, forms esters that are more resistant to hydrolysis than the carbodimide coupling reaction alone.

The peptide linker may have a naturally occurring sequence, or a non-naturally occurring sequence. For example, a sequence derived from the hinge region of heavy chain only antibodies may be used as the linker. *See,* for example, WO1996/34103.

The peptide linker can be of any suitable length. In some embodiments, the peptide linker is at least about any of 1, 2, 3, 4, 5, 6, 7, 8, 9, 10, 11, 12, 13, 14, 15, 16, 17, 18, 19, 20, 25, 30, 35, 40, 50, 75, 100 or more amino acids long. In some embodiments, the peptide linker is no more than about any of 100, 75, 50, 40, 35, 30, 25, 20, 19, 18, 17, 16, 15, 14, 13, 12, 11, 10, 9, 8, 7, 6, 5 or fewer amino acids long. In some embodiments, the length of the peptide linker is any of about 1 amino acid to about 10 amino acids, about 1 amino acid to about 20 amino acids, about 1 amino acid to about 30 amino acids, about 5 amino acids to about 15 amino acids, about 10 amino acids to about 25 amino acids, about 5 amino acids to about 30 amino acids, about 10 amino acids to about 30 amino acids long, about 30 amino acids to about 50 amino acids, about 50 amino acids to about 100 amino acids, or about 1 amino acid to about 100 amino acids.

An essential technical feature of such peptide linker is that said peptide linker does not comprise any polymerization activity. The characteristics of a peptide linker, which comprise the absence of the promotion of secondary structures, are known in the art and described, *e.g.,* in Dall'Acqua et al. (Biochem. (1998) 37, 9266-9273), Cheadle et al. (Mol Immunol (1992) 29, 21-30) and Raag and Whitlow (FASEB (1995) 9(1), 73-80). A particularly preferred amino acid in context of the "peptide linker" is Gly. Furthermore, peptide linkers that also do not promote any secondary structures are preferred. The linkage of the domains to each other can be provided by, *e.g.,* genetic engineering. Methods for preparing fused and operatively linked multispecific single chain constructs and expressing them in mammalian cells or bacteria are well-known in the art (*e.g.* WO 99/54440, Ausubel, Current Protocols in Molecular Biology, Green Publishing Associates and Wiley Interscience, N. Y. 1989 and 1994 or Sambrook et al., Molecular Cloning: A Laboratory Manual, Cold Spring Harbor Laboratory Press, Cold Spring Harbor, N. Y., 2001).

The peptide linker can be a stable linker, which is not cleavable by proteases, especially by Matrix metalloproteinases (MMPs).

### o) Immunoconjugate

Provided herein also include immunoconjugates comprising any of the anti-CD137 construct (such as a multispecific antibody) described herein, linked to a therapeutic agent or a label. In some embodiments, the label is selected from the group consisting of a radioisotope, a fluorescent dye and an enzyme.

### p) Nucleic Acids

Nucleic acid molecules encoding the anti-CD137 constructs or anti-CD137 antibody moieties described herein are also contemplated. In some embodiments, there is provided a nucleic acid (or a set of nucleic acids) encoding a full-length anti-CD137 antibody. In some embodiments, there is provided a nucleic acid (or a set of nucleic acids) encoding an anti-CD137 scFv. In some embodiments, there is provided a nucleic acid (or a set of nucleic acids) encoding an anti-CD137 Fc fusion protein. In some embodiments, there is provided a nucleic acid (or a set of nucleic acids) encoding a multi-specific anti-CD137 molecule (*e.g.,* a multispecific anti-CD137 antibody or a multispecific anti-CD137 antibody), or polypeptide portion thereof. In some embodiments, the nucleic acid (or a set of nucleic acids) encoding the anti-CD137 construct described herein may further comprises a nucleic acid sequence encoding a peptide tag (such as protein purification tag, *e.g.,* His-tag, HA tag).

Also contemplated here are isolated host cell comprising an anti-CD137 construct, an isolated nucleic acid encoding the polypeptide components of the anti-CD137 construct, or a vector comprising a nucleic acid encoding the polypeptide components of the anti-CD137 construct described herein.

The present application also includes variants to these nucleic acid sequences. For example, the variants include nucleotide sequences that hybridize to the nucleic acid sequences encoding the anti-CD 137 constructs or anti-CD137 antibody moieties of the present application under at least moderately stringent hybridization conditions.

The present invention also provides vectors in which a nucleic acid of the present invention is inserted.

The nucleic acids of the present invention may also be used for nucleic acid immunization and gene therapy, using standard gene delivery protocols. Methods for gene delivery are known in the art. See, *e.g.,* U.S. Pat. Nos. 5,399,346, 5,580,859, 5,589,466, incorporated by reference herein in their entireties. In some embodiments, the invention provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al. (2001, Molecular Cloning: A Laboratory Manual, Cold Spring Harbor Laboratory, New York), and in other virology and molecular biology manuals. Viruses which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers (*see, e.g.,* WO 01/96584; WO 01/29058; and U.S. Pat. No. 6,326,193).

### IV. Methods of preparation

In some embodiments, there is provided a method of preparing an anti-CD137 construct or antibody moiety that binds to CD137 and a composition such as polynucleotide, nucleic acid construct, vector, host cell, or culture medium that is produced during the preparation of the anti-CD137 construct or antibody moiety. The anti-CD137 construct or antibody moiety or composition described herein may be prepared by a number of processes as generally described below and more specifically in the Examples.

### Antibody Expression and Production

The antibodies (including anti-CD137 monoclonal antibodies, anti-CD137 multispecific antibodies, and anti-CD137 antibody moieties) described herein can be prepared using any known methods in the art, including those described below and in the Examples.

### Monoclonal antibodies

Monoclonal antibodies are obtained from a population of substantially homogeneous antibodies, *i.e.,* the individual antibodies comprising the population are identical except for possible naturally occurring mutations and/or post-translational modifications (*e*.*g*., isomerizations, amidations) that may be present in minor amounts. Thus, the modifier "monoclonal" indicates the character of the antibody as not being a mixture of discrete antibodies. For example, the monoclonal antibodies may be made using the hybridoma method first described by Kohler et al., Nature, 256:495 (1975), or may be made by recombinant DNA methods (U.S. Pat. No. 4,816,567). In the hybridoma method, a mouse or other appropriate host animal, such as a hamster or a llama, is immunized as hereinabove described to elicit lymphocytes that produce or are capable of producing antibodies that will specifically bind the protein used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp. 59-103 (Academic Press, 1986). Also see Example 1 for immunization in Camels.

The immunizing agent will typically include the antigenic protein or a fusion variant thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell. Goding, Monoclonal Antibodies: Principles and Practice, Academic Press (1986), pp. 59-103.

Immortalized cell lines are usually transformed mammalian cells, particularly myeloma cells of rodent, bovine and human origin. Usually, rat or mouse myeloma cell lines are employed. The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which are substances that prevent the growth of HGPRT-deficient cells.

Preferred immortalized myeloma cells are those that fuse efficiently, support stable high-level production of antibody by the selected antibody-producing cells, and are sensitive to a medium such as HAT medium. Among these, preferred are murine myeloma lines, such as those derived from MOPC-21 and MPC-11 mouse tumors available from the Salk Institute Cell Distribution Center, San Diego, Calif. USA, and SP-2 cells (and derivatives thereof, *e.g.,* X63-Ag8-653) available from the American Type Culture Collection, Manassas, Va. USA. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunosorbent assay (ELISA).

The culture medium in which the hybridoma cells are cultured can be assayed for the presence of monoclonal antibodies directed against the desired antigen. Preferably, the binding affinity and specificity of the monoclonal antibody can be determined by immunoprecipitation or by an in vitro binding assay, such as radioimmunoassay (RIA) or enzyme-linked assay (ELISA). Such techniques and assays are known in the in art. For example, binding affinity may be determined by the Scatchard analysis of Munson *et al., Anal. Biochem.,* 107:220 (1980).

After hybridoma cells are identified that produce antibodies of the desired specificity, affinity, and/or activity, the clones may be subcloned by limiting dilution procedures and grown by standard methods (Goding, supra). Suitable culture media for this purpose include, for example, D-MEM or RPMI-1640 medium. In addition, the hybridoma cells may be grown in vivo as tumors in a mammal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

Monoclonal antibodies may also be made by recombinant DNA methods, such as those described in U.S. Pat. No. 4,816,567, and as described herein. DNA encoding the monoclonal antibodies is readily isolated and sequenced using conventional procedures (*e*.*g*., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese hamster ovary (CHO) cells, or myeloma cells that do not otherwise produce immunoglobulin protein, in order to synthesize monoclonal antibodies in such recombinant host cells. Review articles on recombinant expression in bacteria of DNA encoding the antibody include Skerra et al., Curr. Opinion in Immunol., 5:256-262 (1993) and Plückthun, Immunol. Revs. 130:151-188 (1992).

In a further embodiment, antibodies can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nucl. Acids Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light-chain constant domains in place of the homologous murine sequences (U.S. Pat. No. 4,816,567; Morrison, et al., Proc. Natl Acad. Sci. USA, 81:6851 (1984)), or by covalently joining to the immunoglobulin coding sequence all or part of the coding sequence for a non-immunoglobulin polypeptide. Typically, such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

The monoclonal antibodies described herein may by monovalent, the preparation of which is well known in the art. For example, one method involves recombinant expression of immunoglobulin light chain and a modified heavy chain. The heavy chain is truncated generally at any point in the Fc region so as to prevent heavy chain crosslinking. Alternatively, the relevant cysteine residues may be substituted with another amino acid residue or are deleted so as to prevent crosslinking. *In vitro* methods are also suitable for preparing monovalent antibodies. Digestion of antibodies to produce fragments thereof, particularly Fab fragments, can be accomplished using routine techniques known in the art.

Chimeric or hybrid antibodies also may be prepared *in vitro* using known methods in synthetic protein chemistry, including those involving crosslinking agents. For example, immunotoxins may be constructed using a disulfide-exchange reaction or by forming a thioether bond. Examples of suitable reagents for this purpose include iminothiolate and methyl-4-mercaptobutyrimidate.

Also, see the Examples for monoclonal antibody production.

### Multispecific antibodies

Also provided here are methods of preparing the multispecific antibodies (e.g., bispecific antibody) described herein. The multispecific antibodies can be prepared using any methods known in the art or as described herein (such as in Examples 1 and 3).

Methods of preparing multispecific antibodies of the present application include those described in WO 2008119353 (Genmab), WO 2011131746 (Genmab) and reported by van der Neut- Kolfschoten et al. (Science. 2007 Sep 14;317(5844) : 1554-7). Examples of other platforms useful for preparing multispecific antibodies include but are not limited to BiTE (Micromet), DART (MacroGenics), Fcab and Mab2 (F-star), Fc-engineered IgGl (Xencor) or DuoBody.

Traditional methods such as the hybrid hybridoma and chemical conjugation methods (Marvin and Zhu (2005) Acta Pharmacol Sin 26 : 649) can also be used. Co-expression in a host cell of two components (such as a heavy chain of anti-tumor associated antigen full length antibody and a light chain of anti-tumor associated antigen antibody fused to an anti-CD137 scFv) leads to a mixture of possible antibody products in addition to the desired multispecific antibody, which can then be isolated by, e.g., affinity chromatography or similar methods.

### Nucleic Acid Molecules Encoding antibody moieties

In some embodiments, there is provided a polynucleotide encoding any one of the anti-CD137 constructs or antibody moieties described herein. In some embodiments, there is provided a polynucleotide prepared using any one of the methods as described herein. In some embodiments, a nucleic acid molecule comprises a polynucleotide that encodes a heavy chain or a light chain of an antibody moiety (e.g., anti-CD137 antibody moiety). In some embodiments, a nucleic acid molecule comprises both a polynucleotide that encodes a heavy chain and a polynucleotide that encodes a light chain, of an antibody moiety (e.g., anti-CD137 antibody moiety). In some embodiments, a first nucleic acid molecule comprises a first polynucleotide that encodes a heavy chain and a second nucleic acid molecule comprises a second polynucleotide that encodes a light chain. In some embodiments, a nucleic acid molecule encoding an scFv (*e*.*g*., anti-CD137 scFv) is provided.

In some such embodiments, the heavy chain and the light chain are expressed from one nucleic acid molecule, or from two separate nucleic acid molecules, as two separate polypeptides. In some embodiments, such as when an antibody is an scFv, a single polynucleotide encodes a single polypeptide comprising both a heavy chain and a light chain linked together.

In some embodiments, a polynucleotide encoding a heavy chain or light chain of an antibody moiety (*e*.*g*., anti-CD137 antibody moiety) comprises a nucleotide sequence that encodes a leader sequence, which, when translated, is located at the N terminus of the heavy chain or light chain. As discussed above, the leader sequence may be the native heavy or light chain leader sequence, or may be another heterologous leader sequence.

In some embodiments, the polynucleotide is a DNA. In some embodiments, the polynucleotide is an RNA. In some embodiments, the RNA is a mRNA.

Nucleic acid molecules may be constructed using recombinant DNA techniques conventional in the art. In some embodiments, a nucleic acid molecule is an expression vector that is suitable for expression in a selected host cell.

### Nucleic acid construct

In some embodiments, there is provided a nucleic acid construct comprising any one of the polynucleotides described herein. In some embodiments, there is provided a nucleic acid construct prepared using any method described herein.

In some embodiments, the nucleic acid construct further comprises a promoter operably linked to the polynucleotide. In some embodiments, the polynucleotide corresponds to a gene, wherein the promoter is a wild-type promoter for the gene.

### Vectors

In some embodiments, there is provided a vector comprising any polynucleotides that encode the heavy chains and/or light chains of any one of the antibody moieties described herein (*e*.*g*., anti-CD137 antibody moieties) or nucleic acid construct described herein. In some embodiments, there is provided a vector prepared using any method described herein. Vectors comprising polynucleotides that encode any of anti-CD137 constructs such as antibodies, scFvs, fusion proteins or other forms of constructs described herein (*e*.*g*., anti-CD137 scFv) are also provided. Such vectors include, but are not limited to, DNA vectors, phage vectors, viral vectors, retroviral vectors, *etc.* In some embodiments, a vector comprises a first polynucleotide sequence encoding a heavy chain and a second polynucleotide sequence encoding a light chain. In some embodiments, the heavy chain and light chain are expressed from the vector as two separate polypeptides. In some embodiments, the heavy chain and light chain are expressed as part of a single polypeptide, such as, for example, when the antibody is an scFv.

In some embodiments, a first vector comprises a polynucleotide that encodes a heavy chain and a second vector comprises a polynucleotide that encodes a light chain. In some embodiments, the first vector and second vector are transfected into host cells in similar amounts (such as similar molar amounts or similar mass amounts). In some embodiments, a mole- or mass-ratio of between 5:1 and 1:5 of the first vector and the second vector is transfected into host cells. In some embodiments, a mass ratio of between 1:1 and 1:5 for the vector encoding the heavy chain and the vector encoding the light chain is used. In some embodiments, a mass ratio of 1:2 for the vector encoding the heavy chain and the vector encoding the light chain is used.

In some embodiments, a vector is selected that is optimized for expression of polypeptides in CHO or CHO-derived cells, or in NSO cells. Exemplary such vectors are described, *e.g.,* in Running Deer et al., Biotechnol. Prog. 20:880-889 (2004).

### Host Cells

In some embodiments, there is provided a host cell comprising any poplypeptide, nucleic acid construct and/or vector described herein. In some embodiments, there is provided a host cell prepared using any method described herein. In some embodiments, the host cell is capable of producing any of antibody moieties described herein under a fermentation condition.

In some embodiments, the antibody moieties described herein (*e*.*g*., anti-CD137 antibody moieties) may be expressed in prokaryotic cells, such as bacterial cells; or in eukaryotic cells, such as fungal cells (such as yeast), plant cells, insect cells, and mammalian cells. Such expression may be carried out, for example, according to procedures known in the art. Exemplary eukaryotic cells that may be used to express polypeptides include, but are not limited to, COS cells, including COS 7 cells; 293 cells, including 293-6E cells; CHO cells, including CHO-S, DG44. Lec13 CHO cells, and FUT8 CHO cells; PER.C6^{®} cells (Crucell); and NSO cells. In some embodiments, the antibody moieties described herein (*e*.*g*., anti-CD137 antibody moieties) may be expressed in yeast. See, *e.g.,* U.S. Publication No. US 2006/0270045 A1. In some embodiments, a particular eukaryotic host cell is selected based on its ability to make desired post-translational modifications to the heavy chains and/or light chains of the antibody moiety. For example, in some embodiments, CHO cells produce polypeptides that have a higher level of sialylation than the same polypeptide produced in 293 cells.

Introduction of one or more nucleic acids into a desired host cell may be accomplished by any method, including but not limited to, calcium phosphate transfection, DEAE-dextran mediated transfection, cationic lipid-mediated transfection, electroporation, transduction, infection, *etc.* Non-limiting exemplary methods are described, *e.g.,* in Sambrook et al., Molecular Cloning, A Laboratory Manual, 3rd ed. Cold Spring Harbor Laboratory Press (2001). Nucleic acids may be transiently or stably transfected in the desired host cells, according to any suitable method.

The invention also provides host cells comprising any of the polynucleotides or vectors described herein. In some embodiments, the invention provides a host cell comprising an anti-CD137 antibody. Any host cells capable of over-expressing heterologous DNAs can be used for the purpose of isolating the genes encoding the antibody, polypeptide or protein of interest. Non-limiting examples of mammalian host cells include but not limited to COS, HeLa, and CHO cells. See also PCT Publication No. WO 87/04462. Suitable non-mammalian host cells include prokaryotes (such as *E. coli* or *B. subtillis*) and yeast (such as *S. cerevisae, S. pombe;* or *K. lactis*).

In some embodiments, the antibody moiety is produced in a cell-free system. Non-limiting exemplary cell-free systems are described, *e.g.,* in Sitaraman et al., Methods Mol. Biol. 498: 229-44 (2009); Spirin, Trends Biotechnol. 22: 538-45 (2004); Endo et al., Biotechnol. Adv. 21: 695-713 (2003).

### Culture medium

In some embodiments, there is provided a culture medium comprising any antibody moiety, polynucleotide, nucleic acid construct, vector, and/or host cell described herein. In some embodiments, there is provided a culture medium prepared using any method described herein.

In some embodiments, the medium comprises hypoxanthine, aminopterin, and/or thymidine (e.g., HAT medium). In some embodiments, the medium does not comprise serum. In some embodiments, the medium comprises serum. In some embodiments, the medium is a D-MEM or RPMI-1640 medium.

### Purification of antibody moieties

The anti-CD137 constructs (*e*.*g*., anti-CD137 monospecific or multispecific antibodies) may be purified by any suitable method. Such methods include, but are not limited to, the use of affinity matrices or hydrophobic interaction chromatography. Suitable affinity ligands include the ROR1 ECD and ligands that bind antibody constant regions. For example, a Protein A, Protein G, Protein A/G, or an antibody affinity column may be used to bind the constant region and to purify an anti-CD137 construct comprising an Fc fragment. Hydrophobic interactive chromatography, for example, a butyl or phenyl column, may also suitable for purifying some polypeptides such as antibodies. Ion exchange chromatography (*e.g.* anion exchange chromatography and/or cation exchange chromatography) may also suitable for purifying some polypeptides such as antibodies. Mixed-mode chromatography *(*e.g. reversed phase/anion exchange, reversed phase/cation exchange, hydrophilic interaction/anion exchange, hydrophilic interaction/cation exchange, *etc*.) may also suitable for purifying some polypeptides such as antibodies. Many methods of purifying polypeptides are known in the art.

### V. Methods of modulating a cell composition

Any anti-CD137 constructs as described herein (including any multispecfic antibody) can be used in methods of modulating a cell composition (e.g., T cell composition). The methods comprise contacting the cell composition with the anti-CD137 construct. In some embodiments, contacting or at least portion of the contacting is carried out *ex vivo.* In some embodiments, contacting or at least portion of the contacting is carried out *in vivo.*

### Contacting

In some embodiments, the contacting is carried out in the presence of an agent. In some embodiments, the agent binds to CD3 (*e.g.,* an anti-CD3 antibody). In some embodiments, the agent that binds to CD28 (*e.g.,* an anti-CD28 antibody). In some embodiments, the agent comprises both an agent that binds to CD3 and an agent that binds to CD28. In some embodiments, the agent is a cytokine (*e.g.,* IL-2, IFNγ). In some embodiments, the agent comprises one or more agents (such as one, two three, four or five agents) selected from an agent that binds to CD3, an agent that binds to CD28, IL-2, TNFalpha, and IFNγ. In some embodiments, the agent comprises an agent that binds to CD3 (*e.g.,* an anti-CD3 antibody), IL-2 and IFNγ.

In some embodiments, the concentration of the agent (*e.g.,* an anti-CD3 antibody) is at least about 0.01 µg/ml, 0.02 µg/ml, 0.03 µg/ml, 0.05µg/ml, 0.075 µg/ml, 0.1 µg/ml, 0.125 µg/ml, 0.25 µg/ml, 0.5 µg/ml, or 1 µg/ml.

In some embodiments, the contacting is carried out for at least about 1 hours, 2 hours, 4 hours, 8 hours, or overnight. In some embodiments, the contacting is carried out for at least about 1 day, 2 days or 3 days. In some embodiments, the contacting is carried out for less than about 24 hours, 12 hours, or 8 hours. In some embodiments, the contacting is carried out for less than about 14 days, 10 days, 7 days, 5 days, or 3 days. In some embodiments, the contacting is carried out for about 0-48 hours, 1-24 hours, 2-20 hours, 4-16 hours, or 8-12 hours.

In some embodiments, the contacting is carried out at a temperature of about 0-20 °C. In some embodiments, the contacting is carried out at a temperature of about 2-8°C.

### Cell composition

In some embodiments, the cell composition comprises immune cells (*e*.*g*., human immune cells). In some embodiments, the immune cells comprise T cells (*e.g.,* enriched T cells, *e.g.,* the cells in the composition have at least 50%, 60%, 70%, 80%, 90% or 95% T cells). In some embodiments, the T cells are enriched CD4+ T cells (*e.g.,* the T cells in the composition have at least 50%, 60%, 70%, 80%, 90% or 95% CD4+ T cells). In some embodiments, the T cells are enriched CD8+ T cells (*e.g.,* the T cells in the composition have at least 50%, 60%, 70%, 80%, 90% or 95% CD8+ T cells). In some embodiments, the T cells comprise regulatory T cells (Treg cells). For example the T cells comprise at least 2.5%, 5%, 7.5%, 10%, 15%, or 20% regulatory T cells. In some embodiments, the T cells are engineered T cells comprising a recombinant receptor (such as a chimeric antigen receptor). In some embodiments, the immune cells comprise NK cells (*e.g.,* enriched NK cells, *e.g.,* the cells in the composition have at least 50%, 60%, 70%, 80%, 90% or 95% NK cells). In some embodiments, the cells in the composition comprise cytokine-induced killer (CIK) cells. In some embodiments, the cells comprise any one or more types of immune cells such as B cells, dendritic cells or macrophages.

In some embodiments, the cells were pretreated or simultaneously treated with an agent. In some embodiments, the agent binds to CD3 (*e.g.,* an anti-CD3 antibody). In some embodiments, the agent that binds to CD28 (*e.g.,* an anti-CD28 antibody). In some embodiments, the agent comprises both an agent that binds to CD3 and an agent that binds to CD28. In some embodiments, the agent is a cytokine (*e.g.,* IL-2, IFNγ). In some embodiments, the agent comprises one or more agents (such as one, two three, four or five agents) selected from an agent that binds to CD3, an agent that binds to CD28, IL-2, TNFalpha, and IFNγ.

In some embodiments, the concentration of the agent (*e.g.,* an anti-CD3 antibody) is at least about 0.01 µg/ml, 0.02 µg/ml, 0.03 µg/ml, 0.05µg/ml, 0.075 µg/ml, 0.1 µg/ml, 0.125 µg/ml, 0.25 µg/ml, 0.5 µg/ml, or 1 µg/ml.

In some embodiments, the cells in the composition are to be administered to an individual following the contacting.

### VI. Methods of Treatments or Modulating an immune response in an individual

Also provided here are methods of treating a disease or condition in an individual or modulating an immune response in an individual. The methods comprise administering any anti-CD137 construct described herein into individuals (*e*.*g*., mammals such as humans).

In some embodiments, there is provided a method of treating a disease or condition or modulating an immune response in an individual, comprising administering to the individual an effective amount of an anti-CD137 construct disclosed herein. In some embodiments, the construct is any of the multispecific antibodies as described herein.

In some embodiments, the disease or condition is a cancer. In some embodiments, the cancer is selected from the group consisting of melanoma, glioblastoma, ovarian cancer, lung cancer (*e.g.,* NSCLC), oropharyngeal cancer, colorectal cancer, breast cancer, head and neck cancer, or leukemia (*e*.*g*., AML). In some embodiments, the individual is a human. In some embodiments, the antibody moiety is chimeric or humanized. In some embodiments, the antibody moiety is a full-length antibody. In some embodiments, the antibody moiety has an isotype selected from the group consisting of an IgG (*e.g.,* IgG1, IgG2, IgG3, or IgG4), an IgM, an IgA, an IgD, and an IgE. In some embodiments, the effective amount of the anti-CD137 construct is about 0.005 µg/kg to about 5g/kg of total body weight of the individual. In some embodiments, the antibody agent is administered intravenously, intraperitoneally, intramuscularly, subcutaneously, or orally.

In some embodiments, the individual is a mammal (*e*.*g*., human, non-human primate, rat, mouse, cow, horse, pig, sheep, goat, dog, cat, *etc*.). In some embodiments, the individual is a human. In some embodiments, the individual is a clinical patient, a clinical trial volunteer, an experimental animal, *etc.* In some embodiments, the individual is younger than about 60 years old (including for example younger than about any of 50, 40, 30, 25, 20, 15, or 10 years old). In some embodiments, the individual is older than about 60 years old (including for example older than about any of 70, 80, 90, or 100 years old). In some embodiments, the individual is diagnosed with or genetically prone to one or more of the diseases or disorders described herein (such as a cancer, an autoimmune disease or transplantation). In some embodiments, the individual has one or more risk factors associated with one or more diseases or disorders described herein.

### Modulating immune response

In some embodiments, the modulating of immune response comprises modulating a cell population in the individual. In some embodiments, the cell population is a T cell population. In some embodiments, the cell population is dendritic cells. In some embodiments, the cell population is macrophages. In some embodiments, the cell population is B cells. In some embodiments, the cell population is NK cells. In some population, the cell population is effector T cells and/or memory T cells. In some embodiments, the cell population is effector/memory T cells defined by a phenotype of CD44high CD62L low.

In some embodiments, the modulating comprises promoting proliferation of a cell population. In some embodiments, the proliferation of the cell population after the administration of the anti-CD137 construct is increased by at least about 20%, 30%, 40%, 50%,, 60%, 70%, 80%, or 90% as compared to the proliferation of the reference cells after the administration of a control construct (*e*.*g*., an antibody that does not bind to anti-CD137). In some embodiments, the proliferation of the cell population after the administration of the anti-CD137 construct is increased by at least about 1-fold, 1.2-fold, 1.5 fold, 1.7-fold, 2-fold, 2.5-fold, 3-fold, 3.5-fold, 4-fold, 4.5-fold, 5-fold, 5.5-fold, or 6-fold as compared to the proliferation of the reference cells after the administration of a control construct (*e*.*g*., an antibody that does not bind to CD137).

### Use of anti-CD137 multispecific antibody

In some embodiments, there is provided a method of treating a disease or condition (*e.g.,* cancer) in an individual, comprising administering to the individual an effective amount of a multispecific antibody disclosed herein.

In some embodiments, there is provided a method of treating a tumor or cancer, comprising administering to the individual an effective amount of a multispecific antibody disclosed herein.

### Disease or condition

The anti-CD137 constructs described herein can be used for treating any disease or condition. In some embodiments, the disease or condition is an infection (such as a bacterial infection or viral infection). In some embodiments, the disease or condition is an autoimmune disorder. In some embodiments, the disease or condition is a cancer. In some embodiments, the disease or condition is transplantation.

In some embodiments, the anti-CD137 construct is used in a method for treating a cancer. Cancers that may be treated using any of the methods described herein include tumors that are not vascularized, or not yet substantially vascularized, as well as vascularized tumors. Types of cancers to be treated with the anti-CD 137 constructs as described in this application include, but are not limited to, carcinoma, blastoma, sarcoma, benign and malignant tumors, and malignancies *e*.*g*., sarcomas, carcinomas, and melanomas. Adult tumors/cancers and pediatric tumors/cancers are also included. In some embodiments, the cancer is a solid tumor.

In various embodiments, the cancer is early stage cancer, non-metastatic cancer, primary cancer, advanced cancer, locally advanced cancer, metastatic cancer, cancer in remission, recurrent cancer, cancer in an adjuvant setting, cancer in a neoadjuvant setting, or cancer substantially refractory to a therapy.

Examples of cancers that may be treated by the methods of this application include, but are not limited to, anal cancer, astrocytoma (*e*.*g*., cerebellar and cerebral), basal cell carcinoma, bladder cancer, bone cancer, (osteosarcoma and malignant fibrous histiocytoma), brain tumor (*e.g.,* glioma, brain stem glioma, cerebellar or cerebral astrocytoma (*e.g.,* astrocytoma, malignant glioma, medulloblastoma, and glioblastoma), breast cancer, central nervous system lymphoma, cervical cancer, colon cancer, colorectal cancer, endometrial cancer (*e.g.,* uterine cancer), esophageal cancer, eye cancer *(e.g.,* intraocular melanoma and retinoblastoma), gastric (stomach) cancer, gastrointestinal stromal tumor (GIST), head and neck cancer, hepatocellular (liver) cancer (*e*.*g*., hepatic carcinoma and heptoma), leukemia, liver cancer, lung cancer (*e*.*g*., small cell lung cancer, non-small cell lung cancer, adenocarcinoma of the lung, and squamous carcinoma of the lung), lymphoid neoplasm (*e*.*g*., lymphoma), medulloblastoma, melanoma, mesothelioma, myelodysplastic syndromes, nasopharyngeal cancer, neuroblastoma, ovarian cancer, pancreatic cancer, parathyroid cancer, cancer of the peritoneal, pituitary tumor, lymphoma, rectal cancer, renal cancer, renal pelvis and ureter cancer (transitional cell cancer), rhabdomyosarcoma, skin cancer (*e.g.,* nonmelanoma (*e*.*g*., squamous cell carcinoma), melanoma, and Merkel cell carcinoma), small intestine cancer, squamous cell cancer, testicular cancer, thyroid cancer, tuberous sclerosis, and post-transplant lymphoproliferative disorder (PTLD).

In some embodiments, the cancer is selected from the group consisting of melanoma, glioblastoma, ovarian cancer, lung cancer (*e.g.,* NSCLC), oropharyngeal cancer, colorectal cancer, breast cancer, head and neck cancer, or leukemia (e.g., AML).

### Method of Administering the Anti-CD137 Construct

The dose of the anti-CD137 construct (such as anti-CD 137 monospecific or multispecific antibodies) used for treating a disease or disorder as described herein administered into the individual may vary with the particular anti-CD137 construct (such as anti-CD137 monospecific or multispecific antibodies), the mode of administration, and the type of disease or condition being treated. In some embodiments, the type of disease or condition is a cancer. In some embodiments, the effective amount of the anti-CD137 construct (such as anti-CD137 monospecific or multispecific antibodies) is an amount that is effective to result in an objective response (such as a partial response or a complete response). In some embodiments, the effective amount of the anti-CD137 construct (such as anti-CD137 monospecific or multispecific antibodies) is an amount that is sufficient to result in a complete response in the individual. In some embodiments, the effective amount of the anti-CD137 construct (such as anti-CD137 monospecific or multispecific antibodies) is an amount that is sufficient to result in a partial response in the individual. In some embodiments, the effective amount of anti-CD137 construct (such as anti-CD137 monospecific or multispecific antibodies) is an amount that is sufficient to produce an overall response rate of more than about any of 20%, 25%, 30%, 35%, 40%, 45%, 50%, 55%, 60%, 64%, 65%, 70%, 75%, 80%, 85%, or 90% among a population of individuals treated with the anti-CD137 construct (such as anti-CD137 monospecific or multispecific antibodies). Responses of an individual to the treatment of the methods described herein can be determined, for example, based on RECIST levels.

In some embodiments, the effective amount of the anti-CD137 construct (such as anti-CD137 monospecific or multispecific antibodies) is an amount that is sufficient to prolong progress-free survival of the individual. In some embodiments, the effective amount of the anti-CD137 construct (such as anti-CD137 monospecific or multispecific antibodies) is an amount that is sufficient to prolong overall survival of the individual. In some embodiments, the effective amount of the anti-CD137 construct (such as anti-CD137 monospecific or multispecific antibodies) is an amount that is sufficient to produce clinical benefit of more than about any of 50%, 60%, 70%, or 77% among a population of individuals treated with the anti-CD137 construct (such as anti-CD137 monospecific or multispecific antibodies).

In some embodiments, the effective amount of the anti-CD137 construct (such as anti-CD137 monospecific or multispecific antibodies) alone or in combination with a second, third, and/or fourth agent, is an amount sufficient to decrease the size of a tumor, decrease the number of cancer cells, or decrease the growth rate of a tumor by at least about any of 10%, 20%, 30%, 40%, 50%, 60%, 70%, 80%, 90%, 95% or 100% compared to the corresponding tumor size, number of cancer cells, or tumor growth rate in the same subject prior to treatment or compared to the corresponding activity in other subjects not receiving the treatment (e.g., receiving a placebo treatment). Standard methods can be used to measure the magnitude of this effect, such as *in vitro* assays with purified enzyme, cell-based assays, animal models, or human testing.

In some embodiments, the effective amount of the anti-CD137 construct (such as anti-CD137 monospecific or multispecific antibodies) is an amount that is below the level that induces a toxicological effect (*i.e.,* an effect above a clinically acceptable level of toxicity) or is at a level where a potential side effect can be controlled or tolerated when the composition is administered to the individual.

In some embodiments, the effective amount of the anti-CD137 construct (such as anti-CD137 monospecific or multispecific antibodies) is an amount that is close to a maximum tolerated dose (MTD) of the composition following the same dosing regimen. In some embodiments, the effective amount of the anti-CD137 construct (such as anti-CD137 monospecific or multispecific antibodies) is more than about any of 80%, 90%, 95%, or 98% of the MTD.

In some embodiments, the effective amount of the anti-CD137 construct (such as anti-CD137 monospecific or multispecific antibodies) is an amount that slows or inhibits the progression of the disease or condition (for example, by at least about 5%, 10%, 15%, 20%, 30%, 40%, 50%) as compared to that of the individual not receiving the treatment. In some embodiments, the disease or condition is an autoimmune disease. In some embodiments, the disease or condition is an infection.

In some embodiments, the effective amount of the anti-CD137 construct (such as anti-CD137 monospecific or multispecific antibodies) is an amount that reduces the side effects (auto-immune response) of a condition (*e*.*g*., transplantation) (for example, by at least about 5%, 10%, 15%, 20%, 30%, 40%, or 50%) as compared to that of the individual not receiving the treatment.

In some embodiments of any of the above aspects, the effective amount of an anti-CD137 construct (such as anti-CD137 monospecific or multispecific antibodies) is in the range of about 0.001 µg/kg to about 100mg/kg of total body weight, for example, about 0.005 µg/kg to about 50 mg/kg, about 0.01 µg/kg to about 10 mg/kg, or about 0.01 µg/kg to about 1 mg/kg.

In some embodiments, the treatment comprises more than one administration of the anti-CD137 constructs (such as about two, three, four, five, six, seven, eight, night, or ten administrations of anti-CD137 constructs). In some embodiments, two administrations are carried out within about a week. In some embodiments, a second administration is carried out at least about 1, 2, 3, 4, 5, 6, or 7 days after the completion of the first administration. In some embodiments, a second administration is carried out about 1-14 days, 1-10 days, 1-7 days, 2-6 days, or 3-5 days after the completion of the first administration. In some embodiments, the anti-CD137 construct is administered about 1-3 times a week (such as about once a week, about twice a week, or about three times a week).

The anti-CD137 construct can be administered to an individual (such as human) *via* various routes, including, for example, intravenous, intra-arterial, intraperitoneal, intrapulmonary, oral, inhalation, intravesicular, intramuscular, intra-tracheal, subcutaneous, intraocular, intrathecal, transmucosal, and transdermal. In some embodiments, the anti-CD137 construct is included in a pharmaceutical composition while administered into the individual. In some embodiments, sustained continuous release formulation of the composition may be used. In some embodiments, the composition is administered intravenously. In some embodiments, the composition is administered intraperitoneally. In some embodiments, the composition is administered intravenously. In some embodiments, the composition is administered intraperitoneally. In some embodiments, the composition is administered intramuscularly. In some embodiments, the composition is administered subcutaneously. In some embodiments, the composition is administered intravenously. In some embodiments, the composition is administered orally.

### Combination therapy

This application also provides methods of administering an anti-CD137 construct into an individual for treating a disease or condition (such as cancer), wherein the method further comprises administering a second agent or therapy. In some embodiments, the second agent or therapy is a standard or commonly used agent or therapy for treating the disease or condition. In some embodiments, the second agent or therapy comprises a chemotherapeutic agent. In some embodiments, the second agent or therapy comprises a surgery. In some embodiments, the second agent or therapy comprises a radiation therapy. In some embodiments, the second agent or therapy comprises an immunotherapy. In some embodiments, the second agent or therapy comprises a hormonal therapy. In some embodiments, the second agent or therapy comprises an angiogenesis inhibitor. In some embodiments, the second agent or therapy comprises a tyrosine kinase inhibitor. In some embodiments, the second agent or therapy comprises an infectious agent.

In some embodiments, the anti-CD137 construct is administered simultaneously with the second agent or therapy. In some embodiments, the anti-CD137 construct is administered concurrently with the second agent or therapy. In some embodiments, the anti-CD137 construct is administered sequentially with the second agent or therapy. In some embodiments, the anti-CD137 construct is administered in the same unit dosage form as the second agent or therapy. In some embodiment, the anti-CD137 construct is administered in a different unit dosage form from the second agent or therapy.

In some embodiments, the second agent or therapy is an agent that binds to HER-2 (e.g., trastuzumab or trastuzumab emtansine). In some embodiments, the second agent or therapy is an agent that binds to EGFR. In some embodiments, the second agent or therapy targets PD-L1 or PD-1 (*e.g.,* an anti-PD-1 antibody). In some embodiments, the second agent is an agent that targets CTLA-4 (*e.g.,* an anti-CTLA-4 antibody). In some embodiments, the second agent comprises T cells (*e.g.,* CAR T cells). In some embodiments, the second agent comprises a cytokine. In some embodiments, the second agent or therapy comprises carboplatin, paclitaxel, and/or radiotherapy. In some embodiments, the second agent or therapy comprises a vaccine, such as a HPV vaccine. In some embodiments, the second agent or therapy comprises an EGFR inhibitor (*e*.*g*., cetuximab). In some embodiments, the second agent or therapy comprises an antineoplastic enzyme inhibitor (*e*.*g*., irinotecan). See Table 3 below for exemplary combination therapies.

**Table 3. Exemplary combination therapies**

| Second agent/therapy | Indication |
|---|---|
| Agent targeting PD-L1 or PD-1 (*e.g.,* anti-PD-Ll or PD-1 antibody) | NSCLC, glioblastoma (*e*.*g*., recurrent glioblastoma), AML |
| Agent targeting HER-2 (*e.g.,* anti- | Breast cancer (*e*.*g*., HER-2 breast |
| HER-2 antibody) | cancer) |
| Agent targeting CTLA-4 (*e*.*g*., anti-CTLA-4 antibody) | Melanoma |
| Immunotherapy (*e*.*g*., T cells or cytokines) | Ovarian cancer |
| Radiotherapy | NSCLC |
| Carboplatin + paclitaxel + radiotherapy | NSCLC |
| HPV vaccine | HPV-positive oropharyngeal cancer |
| EGFR inhibitor (*e*.*g*., anti-EGFR antibody) + antineoplastic enzyme inhibitor (*e*.*g*., irinotecan) | Colorectal cancer (*e*.*g*., advanced colorectal cancer) |

### VII. Compositions, Kits and Articles of manufacture

Also provided herein are compositions (such as formulations) comprising any one of the anti-CD137 construct or anti-CD 137 antibody moiety described herein, nucleic acid encoding the antibody moieties, vector comprising the nucleic acid encoding the antibody moieties, or host cells comprising the nucleic acid or vector.

Suitable formulations of the anti-CD137 construct described herein can be obtained by mixing the anti-CD 137 construct or anti-CD 137 antibody moiety having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propylparaben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as olyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e*.*g*. Zn-protein complexes); and/or non-ionic surfactants such as TWEEN^{™}, PLURONICS^{™} or polyethylene glycol (PEG). Lyophilized formulations adapted for subcutaneous administration are described in WO97/04801. Such lyophilized formulations may be reconstituted with a suitable diluent to a high protein concentration and the reconstituted formulation may be administered subcutaneously to the individual to be imaged, diagnosed, or treated herein.

The formulations to be used for *in vivo* administration must be sterile. This is readily accomplished by, *e*.*g*., filtration through sterile filtration membranes.

Also provided are kits comprising any one of the anti-CD137 construct or anti-CD137 antibody moiety described herein. The kits may be useful for any of the methods of modulating cell composition or treatment described herein.

In some embodiments, there is provided a kit comprising an anti-CD137 construct binding to CD137.

In some embodiments, the kit further comprises a device capable of delivering the anti-CD137 construct into an individual. One type of device, for applications such as parenteral delivery, is a syringe that is used to inject the composition into the body of a subject. Inhalation devices may also be used for certain applications.

In some embodiments, the kit further comprises a therapeutic agent for treating a disease or condition, *e*.*g*., cancer, infectious disease, autoimmune disease, or transplantation.

The kits of the present application are in suitable packaging. Suitable packaging includes, but is not limited to, *via*ls*,* bottles, jars, flexible packaging (*e.g.,* sealed Mylar or plastic bags), and the like. Kits may optionally provide additional components such as buffers and interpretative information.

The present application thus also provides articles of manufacture. The article of manufacture can comprise a container and a label or package insert on or associated with the container. Suitable containers include *via*ls (such as sealed *via*ls), bottles, jars, flexible packaging, and the like. Generally, the container holds a composition, and may have a sterile access port (for example the container may be an intravenous solution bag or a *via*l having a stopper pierceable by a hypodermic injection needle). The label or package insert indicates that the composition is used for imaging, diagnosing, or treating a particular condition in an individual. The label or package insert will further comprise instructions for administering the composition to the individual and for imaging the individual. The label may indicate directions for reconstitution and/or use. The container holding the composition may be a multi-use *via*l*,* which allows for repeat administrations (*e.g.* from 2-6 administrations) of the reconstituted formulation. Package insert refers to instructions customarily included in commercial packages of diagnostic products that contain information about the indications, usage, dosage, administration, contraindications and/or warnings concerning the use of such diagnostic products. Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The kits or article of manufacture may include multiple unit doses of the compositions and instructions for use, packaged in quantities sufficient for storage and use in pharmacies, for example, hospital pharmacies and compounding pharmacies.

Those skilled in the art will recognize that several embodiments are possible within the scope and spirit of this invention. The invention will now be described in greater detail by reference to the following non-limiting examples. The following examples further illustrate the invention but, of course, should not be construed as in any way limiting its scope.

### SEQUENCE LISTING

| **SEQ ID NO** | **GENE NAME** | **AMINO ACID SEQUENCE** |
|---|---|---|
| 1. | Clone 3 VH CDR1 | DTYIH |
| 2. | Clone 3 VH CDR2 | RIDPANGFTEYAQKFQG |
| 3. | Clone 3 VH CDR3 | GNLHYALMDY |
| 4. | Clone 3 VL CDR1 | KASQAINTYLS |
| 5. | Clone 3 VL CDR2 | RANRMVD |
| 6. | Clone 3 VL CDR3 | LQSDDFPYT |
| 7. | Clone 3 VH | QVQLVQSGAEVKKPGASVKVSCKASGFNPKDTYIHWVRQA PGQGLEWMGRIDPANGFTEYAQKFQGRVTMTRDTSTSTVY MELSSLRSEDTAVYYCTTGNLHYALMDYWGQGTSVTVSS |
| 8. | Clone 3 VL | QVQLVQSGAEVKKPGASVKVSCKASGFNPKDTYIHWVRQA PGQGLEWMGRIDPANGFTEYAQKFQGRVTMTRDTSTSTVY MELSSLRSEDTAVYYCTTGNLHYALMDYWGQGTSVTVSS DIQMTQSPSSVSASVGDRVTITCKASQAINTYLSWYQQKPG KAPKLLIYRANRMVDGVPSRFSGSGSGTDFTLTISSLQHEDF ATYYCLQSDDFPYTFGGGTKLEIKRTV QVQLVQSGAEVKKPGASVKVSCKASGFNPKDTYIHWVRQA PGQGLEWMGRIDPANGFTEYAQKFQGRVTMTRDTSTSTVY MELSSLRSEDTAVYYCTTGNLHYALMDYWGQGTSVTVSSA |
| 9. | Clone 3 HC | QVQLVQSGAEVKKPGASVKVSCKASGFNPKDTYIHWVRQA PGQGLEWMGRIDPANGFTEYAQKFQGRVTMTRDTSTSTVY MELSSLRSEDTAVYYCTTGNLHYALMDYWGQGTSVTVSSA |
| | | STKGPSVFPLAPCSRSTSESTAALGCLVKDYFPEPVTVSWNS GALTSGVHTFPAVLQSSGLYSLSSVVTVPSSNFGTQTYTCNV DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGL PAPIEKTISKTKGQPREPQVYTLPPSREEMTKNQVSLTCLVK GFYPSDIAVEWESNGQPENNYKTTPPMLDSDGSFFLYSKLT VDKSRWQQGNVFSCSVMHEALHNHYTQKSLSLSPGK DIQMTQSPSSVSASVGDRVTITCKASQAINTYLSWYQQKPG KAPKLLIYRANRMVDGVPSRFSGSGSGTDFTLTISSLQHEDF ATYYCLQSDDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 10. | Clone 3 LC | DIQMTQSPSSVSASVGDRVTITCKASQAINTYLSWYQQKPG KAPKLLIYRANRMVDGVPSRFSGSGSGTDFTLTISSLQHEDF ATYYCLQSDDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 11. | Clone 9 VH CDR1 | DTYIH |
| 12. | Clone 9 VH CDR2 | KIDPANGNTEYAQKFQG |
| 13. | Clone 9 VH CDR3 | GNLHYALMDY |
| 14. | Clone 9 VL CDR1 | KASQAIATYLS |
| 15. | Clone 9 VL CDR2 | RANRMYD |
| 16. | Clone 9 VL CDR3 | LQFDDFPYT |
| 17. | Clone 9 VH | QVQLVQSGAEVKKPGASVKVSCKASGFNLKDTYIHWVRQA PGQGLEWMGKIDPANGNTEYAQKFQGRVTMTRDTSTSTVY MELSSLRSEDTAVYYCTTGNLHYALMDYWGQGTSVTVSS |
| 18. | Clone 9 VL | DIQMTQSPSSVSASVGDRVTITCKASQAIATYLSWYQQKPG KAPKLLIYRANRMYDGVPSRFSGSGSGTDFTLTISSLQPEDF ATYYCLQFDDFPYTFGGGTKLEIKRTV |
| 19. | Clone 9 HC | QVQLVQSGAEVKKPGASVKVSCKASGFNLKDTYIHWVRQA PGQGLEWMGKIDPANGNTEYAQKFQGRVTMTRDTSTSTVY MELSSLRSEDTAVYYCTTGNLHYALMDYWGQGTSVTVSS DIQMTQSPSSVSASVGDRVTITCKASQAIATYLSWYQQKPG KAPKLLIYRANRMYDGVPSRFSGSGSGTDFTLTISSLQPEDF ATYYCLQFDDFPYTFGGGTKLEIKRTV QVQLVQSGAEVKKPGASVKVSCKASGFNLKDTYIHWVRQA PGQGLEWMGKIDPANGNTEYAQKFQGRVTMTRDTSTSTVY MELSSLRSEDTAVYYCTTGNLHYALMDYWGQGTSVTVSSA STKGPSVFPLAPCSRSTSESTAALGCLVKDYFPEPVTVSWNS GALTSGVHTFPAVLQSSGLYSLSSVVTVPSSNFGTQTYTCNV DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGL PAPIEKTISKTKGQPREPQVYTLPPSREEMTKNQVSLTCLVK GFYPSDIAVEWESNGQPENNYKTTPPMLDSDGSFFLYSKLT VDKSRWQQGNVFSCSVMHEALHNHYTQKSLSLSPGK DIQMTQSPSSVSASVGDRVTITCKASQAIATYLSWYQQKPG KAPKLLIYRANRMYDGVPSRFSGSGSGTDFTLTISSLQPEDF ATYYCLQFDDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT ESLNRGEC |
| 20. | Clone 9 LC | DIQMTQSPSSVSASVGDRVTITCKASQAIATYLSWYQQKPG KAPKLLIYRANRMYDGVPSRFSGSGSGTDFTLTISSLQPEDF ATYYCLQFDDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT ESLNRGEC |
| 21. | Clone 23 | DTYIH |
| | VH CDR1 | |
| 22. | Clone 23 VH CDR2 | KIDPANGQTEYAQKFQG |
| 23. | Clone 23 VH CDR3 | GNLHY ALMDY |
| 24. | Clone 23 VL CDR1 | KASQAINTYLS |
| 25. | Clone 23 VL CDR2 | RANRMVG |
| 26. | Clone 23 VL CDR3 | LQYRDFPYT |
| 27. | Clone 23 VH | QVQLVQSGAEVKKPGASVKVSCKASGFNIKDTYIHWVRQA PGQGLEWMGKIDPANGQTEYAQKFQGRVTMTRDTSTSTVY MELSSLRSEDTAVYYCTTGNLHYALMDYWGQGTSVTVSS |
| 28. | Clone 23 VL | DIQMTQSPSSVSASVGDRVTITCKASQAINTYLSWYQQKPG KAPKLLIYRANRMVGGVPSRFSGSGSGTDFTLTISSLQPEDF ATYYCLQYRDFPYTFGGGTKLEIKRTV |
| 29. | Clone 23 HC | QVQLVQSGAEVKKPGASVKVSCKASGFNIKDTYIHWVRQA PGQGLEWMGKIDPANGQTEYAQKFQGRVTMTRDTSTSTVY MELSSLRSEDTAVYYCTTGNLHYALMDYWGQGTSVTVSSA STKGPSVFPLAPCSRSTSESTAALGCLVKDYFPEPVTVSWNS GALTSGVHTFPAVLQSSGLYSLSSVVTVPSSNFGTQTYTCNV DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGL PAPIEKTISKTKGQPREPQVYTLPPSREEMTKNQVSLTCLVK GFYPSDIAVEWESNGQPENNYKTTPPMLDSDGSFFLYSKLT VDKSRWQQGNVFSCSVMHEALHNHYTQKSLSLSPGK |
| 30. | Clone 23 LC | DIQMTQSPSSVSASVGDRVTITCKASQAINTYLSWYQQKPG KAPKLLIYRANRMVGGVPSRFSGSGSGTDFTLTISSLQPEDF ATYYCLQYRDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 31. | Clone 25 VH CDR1 | DTYIH |
| 32. | Clone 25 VH CDR2 | RIDPANGNSEYAQKFQG |
| 33. | Clone 25 VH CDR3 | GNLHYGLMDY |
| 34. | Clone 25 VL CDR1 | KASQAINTYLS |
| 35. | Clone 25 VL CDR2 | RDNRKVD |
| 36. | Clone 25 VL CDR3 | LQYQDFPYK |
| 37. | Clone 25 VH | QVQLVQSGAEVKKPGASVKVSCKASGFNIQDTYIHWVRQA PGQGLEWMGRIDPANGNSEYAQKFQGRVTMTRDTSTSTVY MELSSLRSEDTAVYYCTTGNLHYGLMDYWGQGTSVTVSS |
| 38. | Clone 25 VL | DIQMTQSPSSVSASVGDRVTITCKASQAINTYLSWYQQKPG KAPKLLIYRDNRKVDGVPSRFSGSGSGTDFTLTISSLQPEDF ATYYCLQYQDFPYKFGGGTKLEIKRTV |
| 39. | Clone 25 HC | QVQLVQSGAEVKKPGASVKVSCKASGFNIQDTYIHWVRQA PGQGLEWMGRIDPANGNSEYAQKFQGRVTMTRDTSTSTVY MELSSLRSEDTAVYYCTTGNLHYGLMDYWGQGTSVTVSSA STKGPSVFPLAPCSRSTSESTAALGCLVKDYFPEPVTVSWNS GALTSGVHTFPAVLQSSGLYSLSSVVTVPSSNFGTQTYTCNV DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGL PAPIEKTISKTKGQPREPQVYTLPPSREEMTKNQVSLTCLVK GFYPSDIAVEWESNGQPENNYKTTPPMLDSDGSFFLYSKLT VDKSRWQQGNVFSCSVMHEALHNHYTQKSLSLSPGK |
| 40. | Clone 25 LC | DIQMTQSPSSVSASVGDRVTITCKASQAINTYLSWYQQKPG KAPKLLIYRDNRKVDGVPSRFSGSGSGTDFTLTISSLQPEDF ATYYCLQYQDFPYKFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 41. | Clone 33 VH CDR1 | DTYIQ |
| 42. | Clone 33 VH CDR2 | KIDPANGNTMYAQKFQG |
| 43. | Clone 33 VH CDR3 | GNLHY ALMDY |
| 44. | Clone 33 VL CDR1 | KASQTINTYLS |
| 45. | Clone 33 VL CDR2 | RANRMPD |
| 46. | Clone 33 VL CDR3 | LQYDDFPYT |
| 47. | Clone 33 VH | QVQLVQSGAEVKKPGASVKVSCKASGFNIKDTYIQWVRQA PGQGLEWMGKIDPANGNTMYAQKFQGRVTMTRDTSTSTV YMELSSLRSEDTAVYYCTTGNLHYALMDYWGQGTSVTVSS |
| 48. | Clone 33 VL | DIQMTQSPSSVSASVGDRVTITCKASQTINTYLSWYQQKPG KAPKLLIYRANRMPDGVPSRFSGSGSGTDFTLTISSLQPEDF ATYYCLQYDDFPYTFGGGTKLEIKRTV |
| 49. | Clone 33 HC | QVQLVQSGAEVKKPGASVKVSCKASGFNIKDTYIQWVRQA PGQGLEWMGKIDPANGNTMYAQKFQGRVTMTRDTSTSTV YMELSSLRSEDTAVYYCTTGNLHYALMDYWGQGTSVTVSS ASTKGPSVFPLAPCSRSTSESTAALGCLVKDYFPEPVTVSWN SGALTSGVHTFPAVLQSSGLYSLSSVVTVPSSNFGTQTYTCN VDHKPSNTKVDKTVERKCCVECPPCPAPPVAGPSVFLFPPKP KDTLMISRTPEVTCVVVDVSHEDPEVQFNWYVDGVEVHNA KTKPREEQFNSTFRVVSVLTVVHQDWLNGKEYKCKVSNKG LPAPIEKTISKTKGQPREPQVYTLPPSREEMTKNQVSLTCLV KGFYPSDIAVEWESNGQPENNYKTTPPMLDSDGSFFLYSKL TVDKSRWQQGNVFSCSVMHEALHNHYTQKSLSLSPGK |
| 50. | Clone 33 LC | DIQMTQSPSSVSASVGDRVTITCKASQTINTYLSWYQQKPG |
| | | KAPKLLIYRANRMPDGVPSRFSGSGSGTDFTLTISSLQPEDF ATYYCLQYDDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 51. | Clone 35 VH CDR1 | DTYIH |
| 52. | Clone 35 VH CDR2 | RIDPANGYTEYAQKFQG |
| 53. | Clone 35 VH CDR3 | GNLHY ALMDY |
| 54. | Clone 35 VL CDR1 | KASQAIATYLS |
| 55. | Clone 35 VL CDR2 | RANRMVD |
| 56. | Clone 35 VL CDR3 | LQFQDFPYT |
| 57. | Clone 35 VH | QVQLVQSGAEVKKPGASVKVSCKASGFNINDTYIHWVRQA PGQGLEWMGRIDPANGYTEYAQKFQGRVTMTRDTSTSTVY MELSSLRSEDTAVYYCTTGNLHYALMDYWGQGTSVTVSS |
| 58. | Clone 35 VL | DIQMTQSPSSVSASVGDRVTITCKASQAIATYLSWYQQKPG KAPKLLIYRANRMVDGVPSRFSGSGSGTDFTLTISSLQPEDF ATYYCLQFQDFPYTFGGGTKLEIKRTV |
| 59. | Clone 35 HC | QVQLVQSGAEVKKPGASVKVSCKASGFNINDTYIHWVRQA PGQGLEWMGRIDPANGYTEYAQKFQGRVTMTRDTSTSTVY MELSSLRSEDTAVYYCTTGNLHYALMDYWGQGTSVTVSSA STKGPSVFPLAPCSRSTSESTAALGCLVKDYFPEPVTVSWNS GALTSGVHTFPAVLQSSGLYSLSSVVTVPSSNFGTQTYTCNV DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGL PAPIEKTISKTKGQPREPQVYTLPPSREEMTKNQVSLTCLVK GFYPSDIAVEWESNGQPENNYKTTPPMLDSDGSFFLYSKLT VDKSRWQQGNVFSCSVMHEALHNHYTQKSLSLSPGK |
| 60. | Clone 35 LC | DIQMTQSPSSVSASVGDRVTITCKASQAIATYLSWYQQKPG KAPKLLIYRANRMVDGVPSRFSGSGSGTDFTLTISSLQPEDF ATYYCLQFQDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 61. | Clone 5 VH CDR1 | DTYIH |
| 62. | Clone 5 VH CDR2 | KIDP ANGNTEY AQKFQG |
| 63. | Clone 5 VH CDR3 | GNLHYGLMDY |
| 64. | Clone 5 VL CDR1 | KASQAINTYHS |
| 65. | Clone 5 VL CDR2 | RVNRMVD |
| 66. | Clone 5 VL CDR3 | LQYLDFPYT |
| 67. | Clone 5 VH | QVQLVQSGAEVKKPGASVKVSCKASGFNIRDTYIHWVRQA PGQGLEWMGKIDPANGNTEYAQKFQGRVTMTRDTSTSTVY MELSSLRSEDTAVYYCTTGNLHYGLMDYWGQGTSVTVSS |
| 68. | Clone 5 VL | DIQMTQSPSSVSASVGDRVTITCKASQAINTYHSWYQQKPG KAPKLLIYRVNRMVDGVPSRFSGSGSGTDFTLTISSLQPEDF ATYYCLQYLDFPYTFGGGTKLEIKRTV |
| 69. | Clone 5 HC | QVQLVQSGAEVKKPGASVKVSCKASGFNIRDTYIHWVRQA PGQGLEWMGKIDPANGNTEYAQKFQGRVTMTRDTSTSTVY MELSSLRSEDTAVYYCTTGNLHYGLMDYWGQGTSVTVSSA STKGPSVFPLAPCSRSTSESTAALGCLVKDYFPEPVTVSWNS GALTSGVHTFPAVLQSSGLYSLSSVVTVPSSNFGTQTYTCNV DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGL PAPIEKTISKTKGQPREPQVYTLPPSREEMTKNQVSLTCLVK GFYPSDIAVEWESNGQPENNYKTTPPMLDSDGSFFLYSKLT VDKSRWQQGNVFSCSVMHEALHNHYTQKSLSLSPGK |
| 70. | Clone 5 LC | DIQMTQSPSSVSASVGDRVTITCKASQAINTYHSWYQQKPG KAPKLLIYRVNRMVDGVPSRFSGSGSGTDFTLTISSLQPEDF ATYYCLQYLDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 71. | Clone 6 VH CDR1 | DTYIH |
| 72. | Clone 6 VH CDR2 | KIDPANGLTEYAQKFQG |
| 73. | Clone 6 VH CDR3 | GNLHYALMDY |
| 74. | Clone 6 VL CDR1 | KASQAIATYLS |
| 75. | Clone 6 VL CDR2 | RANRMGD |
| 76. | Clone 6 VL CDR3 | LQYLDFPYT |
| 77. | Clone 6 VH | QVQLVQSGAEVKKPGASVKVSCKASGFNICDTYIHWVRQA PGQGLEWMGKIDPANGLTEYAQKFQGRVTMTRDTSTSTVY MELSSLRSEDTAVYYCTTGNLHYALMDYWGQGTSVTVSS |
| 78. | Clone 6 VL | DIQMTQSPSSVSASVGDRVTITCKASQAIATYLSWYQQKPG KAPKLLIYRANRMGDGVPSRFSGSGSGTDFTLTISSLLPEDF ATYYCLQYLDFPYTFGGGTKLEIKRTV |
| 79. | Clone 6 HC | QVQLVQSGAEVKKPGASVKVSCKASGFNICDTYIHWVRQA PGQGLEWMGKIDPANGLTEYAQKFQGRVTMTRDTSTSTVY MELSSLRSEDTAVYYCTTGNLHYALMDYWGQGTSVTVSSA STKGPSVFPLAPCSRSTSESTAALGCLVKDYFPEPVTVSWNS GALTSGVHTFPAVLQSSGLYSLSSVVTVPSSNFGTQTYTCNV |
| | | DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGL PAPIEKTISKTKGQPREPQVYTLPPSREEMTKNQVSLTCLVK GFYPSDIAVEWESNGQPENNYKTTPPMLDSDGSFFLYSKLT VDKSRWQQGNVFSCSVMHEALHNHYTQKSLSLSPGK |
| 80. | Clone 6 LC | DIQMTQSPSSVSASVGDRVTITCKASQAIATYLSWYQQKPG KAPKLLIYRANRMGDGVPSRFSGSGSGTDFTLTISSLLPEDF ATYYCLQYLDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 81. | Clone 17 VH CDR1 | DTYIH |
| 82. | Clone 17 VH CDR2 | KIDPANGQTEYAQKFQG |
| 83. | Clone 17 VH CDR3 | GNLHYALMDY |
| 84. | Clone 17 VL CDR1 | KASQATNTYLS |
| 85. | Clone 17 VL CDR2 | RVNRVVD |
| 86. | Clone 17 VL CDR3 | LQYRDFPYT |
| 87. | Clone 17 VH | QVQLVQSGAEVKKPGASVKVSCKASGFCIKDTYIHWVRQA PGQGLEWMGKIDPANGQTEYAQKFQGRVTMTRDTSTSTVY MELSSLRSEDTAVYYCTTGNLHYALMDYWGQGTSVTVSS |
| 88. | Clone 17 VL | DIQMTQSPSSVSASVGDRVTITCKASQATNTYLSWYQQKPG KAPKLLIYRVNRVVDGVPSRFSGSGSGTDFTLTISSLQPEDF ATYYCLQYRDFPYTFGGGTKLEIKRTV |
| 89. | Clone 17 HC | QVQLVQSGAEVKKPGASVKVSCKASGFCIKDTYIHWVRQA PGQGLEWMGKIDPANGQTEYAQKFQGRVTMTRDTSTSTVY MELSSLRSEDTAVYYCTTGNLHYALMDYWGQGTSVTVSSA STKGPSVFPLAPCSRSTSESTAALGCLVKDYFPEPVTVSWNS GALTSGVHTFPAVLQSSGLYSLSSVVTVPSSNFGTQTYTCNV DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGL PAPIEKTISKTKGQPREPQVYTLPPSREEMTKNQVSLTCLVK GFYPSDIAVEWESNGQPENNYKTTPPMLDSDGSFFLYSKLT VDKSRWQQGNVFSCSVMHEALHNHYTQKSLSLSPGK |
| 90. | Clone 17 LC | DIQMTQSPSSVSASVGDRVTITCKASQATNTYLSWYQQKPG KAPKLLIYRVNRVVDGVPSRFSGSGSGTDFTLTISSLQPEDF ATYYCLQYRDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 91. | Clone 18 VH CDR1 | DTYIH |
| 92. | Clone 18 VH CDR2 | KIDP ANGMTEY AQKFQG |
| 93. | Clone 18 VH CDR3 | GNLHY ALMDY |
| 94. | Clone 18 VL CDR1 | KASQAPNTYLS |
| 95. | Clone 18 VL CDR2 | RANRMVD |
| 96. | Clone 18 VL CDR3 | LQYEDFPYT |
| 97. | Clone 18 VH | QVQLVQSGAEVKKPGASVKVSCQASGFNIKDTYIHWVRQA PGQGLGWMGKIDPANGMTEYAQKFQGRVTVTRDTSTSTVY MELSSLRSEDTAVYYCTTGNLHYALMDYWGQGTSVTVSS |
| 98. | Clone 18 VL | DIQMTQSPSSVSASVGDRVTITCKASQAPNTYLSWYQQKPG KAPKLLIYRANRMVDGVPSRFSGSGSGTDFTLTISSLQPEDF ATYYCLQYEDFPYTFGGGTKLEIKRTV |
| 99. | Clone 18 HC | QVQLVQSGAEVKKPGASVKVSCQASGFNIKDTYIHWVRQA PGQGLGWMGKIDPANGMTEYAQKFQGRVTVTRDTSTSTVY MELSSLRSEDTAVYYCTTGNLHYALMDYWGQGTSVTVSSA STKGPSVFPLAPCSRSTSESTAALGCLVKDYFPEPVTVSWNS GALTSGVHTFPAVLQSSGLYSLSSVVTVPSSNFGTQTYTCNV DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGL PAPIEKTISKTKGQPREPQVYTLPPSREEMTKNQVSLTCLVK GFYPSDIAVEWESNGQPENNYKTTPPMLDSDGSFFLYSKLT VDKSRWQQGNVFSCSVMHEALHNHYTQKSLSLSPGK |
| 100. | Clone 18 LC | DIQMTQSPSSVSASVGDRVTITCKASQAPNTYLSWYQQKPG KAPKLLIYRANRMVDGVPSRFSGSGSGTDFTLTISSLQPEDF ATYYCLQYEDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 101. | Clone 19 VH CDR1 | DTYIH |
| 102. | Clone 19 VH CDR2 | KIDPANGATEYAQKFQG |
| 103. | Clone 19 VH CDR3 | GNLHYGLMDY |
| 104. | Clone 19 VL CDR1 | KASQAINTYLS |
| 105. | Clone 19 VL CDR2 | RANRMGD |
| 106. | Clone 19 VL CDR3 | LQSDDFPYT |
| 107. | Clone 19 VH | QVQLVQSGAEVKKPGASVKVSCKASGFNMKDTYIHWVRQ APGQGLEWMGKIDPANGATEYAQKFQGRVTMTRDTSTSTV YMELSSLRSEDTAVYYCTTGNLHYGLMDYWGQGTSVTVSS |
| 108. | Clone 19 VL | DIQMTQSPSSVSASVGDRVTITCKASQAINTYLSWYQQKPG KAPKLLIYRANRMGDGVPSRFSGSGSGTDFTLTISSLQPEDF |
| | | ATYYCLQSDDFPYTFGGGTKLEIKRTV |
| 109. | Clone 19 HC | QVQLVQSGAEVKKPGASVKVSCKASGFNMKDTYIHWVRQ APGQGLEWMGKIDPANGATEYAQKFQGRVTMTRDTSTSTV YMELSSLRSEDTAVYYCTTGNLHYGLMDYWGQGTSVTVSS ASTKGPSVFPLAPCSRSTSESTAALGCLVKDYFPEPVTVSWN SGALTSGVHTFPAVLQSSGLYSLSSVVTVPSSNFGTQTYTCN VDHKPSNTKVDKTVERKCCVECPPCPAPPVAGPSVFLFPPKP KDTLMISRTPEVTCVVVDVSHEDPEVQFNWYVDGVEVHNA KTKPREEQFNSTFRVVSVLTVVHQDWLNGKEYKCKVSNKG LPAPIEKTISKTKGQPREPQVYTLPPSREEMTKNQVSLTCLV KGFYPSDIAVEWESNGQPENNYKTTPPMLDSDGSFFLYSKL TVDKSRWQQGNVFSCSVMHEALHNHYTQKSLSLSPGK |
| 110. | Clone 19 LC | DIQMTQSPSSVSASVGDRVTITCKASQAINTYLSWYQQKPG KAPKLLIYRANRMGDGVPSRFSGSGSGTDFTLTISSLQPEDF ATYYCLQSDDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 111. | Clone 20 VH CDR1 | DTYIH |
| 112. | Clone 20 VH CDR2 | KIDPANGDTEYAQKFQG |
| 113. | Clone 20 VH CDR3 | GNLHYALMDY |
| 114. | Clone 20 VL CDR1 | KASQAINTYLS |
| 115. | Clone 20 VL CDR2 | RANRAVD |
| 116. | Clone 20 VL CDR3 | LQYLDFPYT |
| 117. | Clone 20 VH | QVQLVQSGAEVKKPGASVKVSCKASGFNMKDTYIHWVRQ APGQGLEWMGKIDPANGDTEYAQKFQGRVTMTRDTSTSTV YMELSSLRSEDTAVYYCTTGNLHYALMDYWGQGTSVTVSS |
| 118. | Clone 20 VL | DIQMTQSPSSVSASVGDRVTITCKASQAINTYLSWYQQKPG KAPKLLIYRANRAVDGVPSRFSGSGSGTDFTLTISSLQPEDF ATYYCLQYLDFPYTFGGGTKLEIKRTV |
| 119. | Clone 20 HC | QVQLVQSGAEVKKPGASVKVSCKASGFNMKDTYIHWVRQ APGQGLEWMGKIDPANGDTEYAQKFQGRVTMTRDTSTSTV YMELSSLRSEDTAVYYCTTGNLHYALMDYWGQGTSVTVSS ASTKGPSVFPLAPCSRSTSESTAALGCLVKDYFPEPVTVSWN SGALTSGVHTFPAVLQSSGLYSLSSVVTVPSSNFGTQTYTCN VDHKPSNTKVDKTVERKCCVECPPCPAPPVAGPSVFLFPPKP KDTLMISRTPEVTCVVVDVSHEDPEVQFNWYVDGVEVHNA KTKPREEQFNSTFRVVSVLTVVHQDWLNGKEYKCKVSNKG LPAPIEKTISKTKGQPREPQVYTLPPSREEMTKNQVSLTCLV KGFYPSDIAVEWESNGQPENNYKTTPPMLDSDGSFFLYSKL TVDKSRWQQGNVFSCSVMHEALHNHYTQKSLSLSPGK |
| 120. | Clone 20 LC | DIQMTQSPSSVSASVGDRVTITCKASQAINTYLSWYQQKPG KAPKLLIYRANRAVDGVPSRFSGSGSGTDFTLTISSLQPEDF ATYYCLQYLDFPYTFGGGTKLEIKRTVAAPSVFIFPPSDEQL |
| | | KSGTASVVCLLNNFYPREAKVQWKVDNALQSGNSQESVTE QDSKDSTYSLSSTLTLSKADYEKHKVYACEVTHQGLSSPVT KSFNRGEC |
| 121. | Clone 2-9 VH CDR1 | DTYIH |
| 122. | Clone 2-9 VH CDR2 | RIDPANGNSEYAQKFQG |
| 123. | Clone 2-9 VH CDR3 | GNLHYALMDY |
| 124. | Clone 2-9 VL CDR1 | KASQPINTYLS |
| 125. | Clone 2-9 VL CDR2 | RVNRKVD |
| 126. | Clone 2-9 VL CDR3 | LQYLDFPYT |
| 127. | Clone 2-9 VH | QVQLVQSGAEVKKPGASVKASCKASGFNIQDTYIHWVRQA PGQGLEWMGRIDPANGNSEYAQKFQGRVTMTRDTSTSTVY MELSSLRSEDTAVYYCTTGNLHYALMDYWGQGTSVTVSS |
| 128. | Clone 2-9 VL | DIQMTQSPSSVSASVGDRVTITCKASQPINTYLSWYQQKPG KAPKLLIYRVNRKVDGVPSRFSGSGSGTDFTLTISSLQPEDF ATYYCLQYLDFPYTFGGGTKLEIKRTV |
| 129. | Clone 2-9 HC | QVQLVQSGAEVKKPGASVKASCKASGFNIQDTYIHWVRQA PGQGLEWMGRIDPANGNSEYAQKFQGRVTMTRDTSTSTVY MELSSLRSEDTAVYYCTTGNLHYALMDYWGQGTSVTVSSA STKGPSVFPLAPCSRSTSESTAALGCLVKDYFPEPVTVSWNS GALTSGVHTFPAVLQSSGLYSLSSVVTVPSSNFGTQTYTCNV DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGL PAPIEKTISKTKGQPREPQVYTLPPSREEMTKNQVSLTCLVK GFYPSDIAVEWESNGQPENNYKTTPPMLDSDGSFFLYSKLT VDKSRWQQGNVFSCSVMHEALHNHYTQKSLSLSPGK |
| 130. | Clone 2-9 LC | DIQMTQSPSSVSASVGDRVTITCKASQPINTYLSWYQQKPG KAPKLLIYRVNRKVDGVPSRFSGSGSGTDFTLTISSLQPEDF ATYYCLQYLDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 131. | Clone 2-11 VH CDR1 | DTYIH |
| 132. | Clone 2-11 VH CDR2 | KIDPANGGTEYAQKFQG |
| 133. | Clone 2-11 VH CDR3 | GNLHYALMDY |
| 134. | Clone 2-11 VL CDR1 | KASQPINTYLS |
| 135. | Clone 2-11 VL CDR2 | RVNRKVD |
| 136. | Clone 2-11 VL CDR3 | LQYVDFPYT |
| 137. | Clone 2-11 VH | QVQLVQSGAEVKKPGASVKVSCKASGFNIKDTYIHWVRQA PGQGLEWMGKIDPANGGTEYAQKFQGRVTMTRDTSTSTVY MELSSLRSEDTAVYYCTTGNLHYALMDYWGQGTSVTVSS |
| 138. | Clone 2-11 VL | DIQMTQSPSSVSASVGDRVTITCKASQPINTYLSWYQQKPG KAPKLLIYRVNRKVDGVPSRFSGSGSGTDFTLTISSLQPEDF ATYYCLQYVDFPYTFGGGTKLEIKRTV |
| 139. | Clone 2-11 HC | QVQLVQSGAEVKKPGASVKVSCKASGFNIKDTYIHWVRQA PGQGLEWMGKIDPANGGTEYAQKFQGRVTMTRDTSTSTVY MELSSLRSEDTAVYYCTTGNLHYALMDYWGQGTSVTVSSA STKGPSVFPLAPCSRSTSESTAALGCLVKDYFPEPVTVSWNS GALTSGVHTFPAVLQSSGLYSLSSVVTVPSSNFGTQTYTCNV DHKPSNTKVDKTVERKCCVECPPCPAPPVAGPSVFLFPPKPK DTLMISRTPEVTCVVVDVSHEDPEVQFNWYVDGVEVHNAK TKPREEQFNSTFRVVSVLTVVHQDWLNGKEYKCKVSNKGL PAPIEKTISKTKGQPREPQVYTLPPSREEMTKNQVSLTCLVK GFYPSDIAVEWESNGQPENNYKTTPPMLDSDGSFFLYSKLT VDKSRWQQGNVFSCSVMHEALHNHYTQKSLSLSPGK |
| 140. | Clone 2-11 LC | DIQMTQSPSSVSASVGDRVTITCKASQPINTYLSWYQQKPG KAPKLLIYRVNRKVDGVPSRFSGSGSGTDFTLTISSLQPEDF ATYYCLQYVDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 141. | Consensus VH CDR1 | DTYIH |
| 142. | Consensus VH CDR2 | XIDPANGXTEYAQKFQG |
| 143. | Consensus VH CDR3 | GNLHYXLMDY |
| 144. | Consensus VL CDR1 | KASQXXXTYXS |
| 145. | Consensus VL CDR2 | RXNRXXD |
| 146. | Consensus VL CDR3 | LQYXDFPYT |
| 147. | Consensus VH | QVQLVQSGAEVKKPGASVKVSCKASGFXXXDTYIHWVRQ APGQGLEWMGXIDPANGXTEYAQKFQGRVTMTRDTSTSTV YMELSSLRSEDTAVYYCTTGNLHYXLMDYWGQGTSVTVSS |
| 148. | Consensus VL | DIQMTQSPSSVSASVGDRVTITCKASQXXXTYXSWYQQKPG KAPKLLIYRXNRXXDGVPSRFSGSGSGTDFTLTISSLQPEDF ATYYCLQYXDFPYTFGGGTKLEIKRTV |
| 149. | Consensus HC | QVQLVQSGAEVKKPGASVKVSCKASGFXXXDTYIHWVRQ APGQGLEWMGKIDPANGXTEYAQKFQGRVTMTRDTSTSTV YMELSSLRSEDTAVYYCTTGNLHYXLMDYWGQGTSVTVSS ASTKGPSVFPLAPCSRSTSESTAALGCLVKDYFPEPVTVSWN SGALTSGVHTFPAVLQSSGLYSLSSVVTVPSSNFGTQTYTCN VDHKPSNTKVDKTVERKCCVECPPCPAPPVAGPSVFLFPPKP KDTLMISRTPEVTCVVVDVSHEDPEVQFNWYVDGVEVHNA KTKPREEQFNSTFRVVSVLTVVHQDWLNGKEYKCKVSNKG |
| | | LPAPIEKTISKTKGQPREPQVYTLPPSREEMTKNQVSLTCLV KGFYPSDIAVEWESNGQPENNYKTTPPMLDSDGSFFLYSKL TVDKSRWOOGNVFSCSVMHEALHNHYTOKSLSLSPGK |
| 150. | Consensus LC | DIQMTQSPSSVSASVGDRVTITCKASQXXXTYXSWYQQKPG KAPKLLIYRXNRXXDGVPSRFSGSGSGTDFTLTISSLQPEDF ATYYCLQYXDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |
| 151. | 29.39/44.2 1 VH CDR1 | DTYIH |
| 152. | 3.10 VH CDR1 | TYDVS |
| 153. | 30.19/6.62 VH CDR1 | DYSIH |
| 154. | 29.39/44.2 1 VH CDR2 | KIDPANGNTEYDLNFQG |
| 155. | 3.10 VH CDR2 | VIWTGGGTNYNSTFMS |
| 156. | 30.19/6.62 VH CDR2 | WMNTETGEPAYADDFKG |
| 157. | 29.39/44.2 1 VH CDR3 | GNLHYYLMDY |
| 158. | 3.10 VH CDR3 | VDY |
| 159. | 30.19/6.62 VH CDR3 | SSYGYFDY |
| 160. | 29.39/30.1 9 VL CDR1 | KASQAINTYLS |
| 161. | 6.62 VL CDR1 | RSSQTLEHINGNTYLE |
| 162. | 44.21 VL CDR1 | RSSQSLVHSNGNTYLH |
| 163. | 3.10 VL CDR1 | RSSQSLVHNNGNTYLH |
| 164. | 29.39/30.1 9 VL CDR2 | RANRMVD |
| 165. | 6.62 VL CDR2 | KVSDRFS |
| 166. | 44.21/3.10 VL CDR2 | KVSNRFS |
| 167. | 29.39/30.1 9 VL CDR3 | LQYDDFPYT |
| 168. | 6.62 VL CDR3 | FQGSHFPLT |
| 169. | 44.21/3.10 VL CDR3 | SQNAHVPWT |
| 170. | 29.39/44.2 1 VH | EVKLMESGAELVKPGASVKLSCTASGFNIKDTYIHWVKQRP EQGLEWIGKIDPANGNTEYDLNFQGRATITADTSSNTAYLQ LSSLTSEDTAVYYCTTGNLHYYLMDYWGQGTSVTSLE |
| 171. | 30.19/6.62 VH | QIQLVQSGPELKKPGETVKISCKASGYSFTDYSIHWVKQAPG KGLKWMGWMNTETGEPAYADDFKGRFAFSLETAASTAYL QINHLRDEDTATYFCTRSSYGYFDYWGQGTTLTVSS |
| 172. | 3.10 VH | EVQLVESGPGLVAPSQSLSITCTVSGFSLTTYDVSWIRQPPG KGLEWLGVIWTGGGTNYNSTFMSRLTISKDNSKNQVFLKM NSLQTDDTATYYCVRVDYWGQGTTLTVSR |
| 173. | 29.39/30.1 9 VL | DIVMTQSPSSMYASLGERVTITCKASQAINTYLSWFQQKPG KSPKTLIYRANRMVDGVPSRFSGSGSGQDYSLTISSLDYED MGIYYCLQYDDFPYTFGGGTKLEIKR |
| 174. | 6.62 VL | DVVMTQTPLSLPVSLGDQAAISCRSSQTLEHINGNTYLEWY LQKPGQSPKLLIYKVSDRFSGVPDRFSGSGSGTGFTLKISRVE AEDLGVYYCFQGSHFPLTFGGGTKLELK |
| 175. | 3.10 VL | DVVMTQTPLSLPVSLGGHASISCRSSQSLVHNNGNTYLHWF LQKPGQSPKLLIYKVSNRFSGVPDRFSGSGSGTDFTLKISRVE AEDLGVYFCSQNAHVPWTFGGGTKLEIKR |
| 176. | 44.21 VL | DIVMTQTPLSLPVSLGDHASISCRSSQSLVHSNGNTYLHWFL QKPGQSPKLLIYKVSNRFSGVPDRFSGSGSGTDFTLKISRVE AEDLGVYFCSQNAHVPWTFGGGTKRKSNE |
| 177. | Consensus humanized 29.39 VH CDR1 | GFX₁X₂X₃DTYI X₄ |
| | | wherein Xi=N or C; X₂=I, P, L or M; X₃=K, N, R, C or Q; X₄=H or Q. |
| 178. | Consensus humanized 29.39 VH CDR2 | X₁IDPANGX₂X₃X₄ |
| | | wherein X₁=K or R; X₂=N, G, F, Y, A, D, L, M or Q; X₃=S or T; X₄=E or M. |
| 179. | Consensus humanized 29.39 VH CDR3 | GNLHYX₁LMD |
| | | wherein X₁=Y, A or G. |
| 180. | Consensus humanized 29.39 VL CDR1 | KASQX₁X₂X₃TYX₄S |
| | | wherein X₁=A, P, or T; X₂=I, T, or P; X₃=N or A; X₄=L, G or H. |
| 181. | Consensus humanized 29.39 VL CDR2 | RX₁NRX₂X₃X₄ |
| | | wherein X₁=A, Y, V, or D; X₂=M, K, V, or A; X₃=V, P, Y or G; X₄=D or G. |
| 182. | Consensus humanized 29.39 VL CDR3 | LQX₁X₂DFPYX₃ |
| | | wherein X₁=Y, S, or F; X₂=D, V, L, R, E or Q; X₃=T or K. |
| 183. | Exemplary linker | GGGGSGGGGSGGGGS |
| 184. | Exemplary linker | GGGSG |
| 185. | Exemplary linker | GGGSGGGGSG |
| 186. | Exemplary linker | GSGGSGGSGGSG |
| 187. | Exemplary linker | GGSGGGSG |
| 188. | Exemplary linker | GGSGGGSGGGSG |
| 189. | Exemplary linker | GSGGSG |
| 190. | Exemplary linker | GSGGSGGSG |
| 191. | Exemplary linker | GSGSGSG |
| 192. | Exemplary linker | GGGGSGGGGSGGGGSGGG |
| 193. | Exemplary linker | GGGGSGGGGSGGGGSGGGGSGGGGSGGGGS |
| 194. | Exemplary linker | PAPAP |
| 195. | Exemplary linker | PAPAPPAPAPPAPAP |
| 196. | Exemplary linker | IKRTVAA |
| 197. | Exemplary linker | VSSASTK |
| 198. | Exemplary linker | AEAAAKA |
| 199. | Exemplary linker | AEAAAKEAAAKA |
| 200. | Exemplary linker | GRPGS GRPGS |
| 201. | Exemplary linker | GRPGS GRPGS GRPGS GRPGS |
| 202. | Exemplary linker | GRGGS GRGGS |
| 203. | Exemplary linker | GRGGS GRGGS GRGGS GRGGS |
| 204. | Exemplary linker | GKPGS GKPGS |
| 205. | Exemplary linker | GKPGS GKPGS GKPGS GKPGS |
| 206. | Exemplary linker | GEPGS GEPGS |
| 207. | Exemplary linker | GEGGS GEGGS GEGGS GEGGS |
| 208. | Exemplary linker | GDPGS GDPGS |
| 209. | Exemplary linker | GDPGS GDPGS GDPGS GDPGS |
| 210. | Exemplary linker | GGGGSGGGGSGGGGSGGGGS |
| 211. | Clone 2-9- 1 VH CDR1 | DTYIH |
| 212. | Clone 2-9- 1 VH CDR2 | RIDPASGNSEYAQKFQG |
| 213. | Clone 2-9- 1 VH CDR3 | GNLHYALMDY |
| 214. | Clone 2-9- 1 VL CDR1 | KASQPINTYLS |
| 215. | Clone 2-9- 1 VL CDR2 | RVNRKVD |
| 216. | Clone 2-9- 1 VL CDR3 | LQYLDFPYT |
| 217. | Clone 2-9-1 VH | QVQLVQSGAEVKKPGASVKASCKASGFNIQDTYIHWVRQA PGQGLEWMGRIDPASGNSEYAQKFQGRVTMTRDTSTSTVY MELSSLRSEDTAVYYCTTGNLHYALMDYWGQGTSVTVSS |
| 218. | Clone 2-9-1 VL | DIQMTQSPSSVSASVGDRVTITCKASQPINTYLSWYQQKPG KAPKLLIYRVNRKVDGVPSRFSGSGSGTDFTLTISSLQPEDF ATYYCLQYLDFPYTFGGGTKLEIKRTV |
| 219. | Clone 2-9-1 HC | QVQLVQSGAEVKKPGASVKASCKASGFNIQDTYIHWVRQA PGQGLEWMGRIDPASGNSEYAQKFQGRVTMTRDTSTSTVY MELSSLRSEDTAVYYCTTGNLHYALMDYWGQGTSVTVSSA STKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCDKTHTCPPCPAPELLGGPSVFLFP PKPKDTLMISRTPEVTCVVVDVEHEDPEVKFNWYVDGVEV HNAKTKPREEQYNSTYRVVSVLTVLHQDWLNGKEYKCKV SNKAFPAPIEKTISKAKGQPREPQVYTLPPSREEMTKNQVSL TCLVKGFYPSDIAVEWESNGQPENNYKTTPPVLDSDGSFFL YSKLTVDKSRWQQGNVFSCSVMHEALHNHYTQKSLSLSPG K |
| 220. | Clone 2-9-1 LC | DIQMTQSPSSVSASVGDRVTITCKASQPINTYLSWYQQKPG KAPKLLIYRVNRKVDGVPSRFSGSGSGTDFTLTISSLQPEDF ATYYCLQYLDFPYTFGGGTKLEIKRTVAAPSVFIFPPSDEQL |
| | | KSGTASVVCLLNNFYPREAKVQWKVDNALQSGNSQESVTE QDSKDSTYSLSSTLTLSKADYEKHKVYACEVTHQGLSSPVT KSFNRGEC |
| 221. | Clone 2-9- 2 VH CDR1 | DTYIH |
| 222. | Clone 2-9- 2 VH CDR2 | RIDPASGNSEYAQKFQG |
| 223. | Clone 2-9- 2 VH CDR3 | GNLHYALMDY |
| 224. | Clone 2-9- 2 VL CDR1 | KASQPINTYLS |
| 225. | Clone 2-9- 2 VL CDR2 | RVNRKVD |
| 226. | Clone 2-9- 2 VL CDR3 | LQYLDFPYT |
| 227. | Clone 2-9-2 VH | QVQLVQSGAEVKKPGASVKVSCKASGFNIQDTYIHWVRQA PGQGLEWMGRIDPASGNSEYAQKFQGRVTMTRDTSTSTVY MELSSLRSEDTAVYYCTTGNLHYALMDYWGQGTSVTVSS |
| 228. | Clone 2-9-2 VL | DIQMTQSPSSVSASVGDRVTITCKASQPINTYLSWYQQKPG KAPKLLIYRVNRKVDGVPSRFSGSGSGTDFTLTISSLQPEDF ATYYCLQYLDFPYTFGGGTKLEIKRTV |
| 229. | Clone 2-9-2 HC | QVQLVQSGAEVKKPGASVKVSCKASGFNIQDTYIHWVRQA PGQGLEWMGRIDPASGNSEYAQKFQGRVTMTRDTSTSTVY MELSSLRSEDTAVYYCTTGNLHYALMDYWGQGTSVTVSSA STKGPSVFPLAPCSRSTSESTAALGCLVKDYFPEPVTVSWNS GALTSGVHTFPAVLQSSGLYSLSSVVTVPSSSLGTKTYTCNV DHKPSNTKVDKRVESKYGPPCPPCPAPEFLGGPSVFLFPPKP KDTLMISRTPEVTCVVVDVSQEDPEVQFNWYVDGVEVHNA KTKPREEQFNSTYRVVSVLTVLHQDWLNGKEYKCKVSNKG LPSSIEKTISKAKGQPREPQVYTLPPSQEEMTKNQVSLTCLV KGFYPSDIAVEWESNGQPENNYKTTPPVLDSDGSFFLYSRLT VDKSRWQEGNVFSCSVMHEALHNHYTQKSLSLSLGK |
| 230. | Clone 2-9-2 LC | DIQMTQSPSSVSASVGDRVTITCKASQPINTYLSWYQQKPG KAPKLLIYRVNRKVDGVPSRFSGSGSGTDFTLTISSLQPEDF ATYYCLQYLDFPYTFGGGTKLEIKRTVAAPSVFIFPPSDEQL KSGTASVVCLLNNFYPREAKVQWKVDNALQSGNSQESVTE QDSKDSTYSLSSTLTLSKADYEKHKVYACEVTHQGLSSPVT KSFNRGEC |

### EXAMPLES

The examples below are intended to be purely exemplary of the application and should therefore not be considered to limit the application in any way. The following examples and detailed description are offered by way of illustration and not by way of limitation.

### Example 1: Generation of anti-CD137 antibodies

### A. Generating anti-CD137 antibodies from mouse hybridomas

Fully human monoclonal antibodies to the human CD137 (4-1BB) receptor were generated in the BALB/c mice. BALB/c mice were immunized with the extracellular domain of human CD137 in RIBI adjuvant (Ribi Immunochemical). Prior to fusion, mice were boosted intravenously (i.v.) with the same amount of antigen. Lymph nodes from immunized mice with adequate titers of antibodies to huCD137 were fused to mouse myeloma cells following standard procedures.

Hybridoma screening. Detection of binding to huCD137 by ELISA: To identify hybridomas secreting anti-human CD137 antibodies, ELISA plates (Corning) were coated with human CD137-Fc fusion protein at 1 µg/ml in PBS overnight at 4° C. The plates were then washed 3 times with PBS with 0.5% Tween-20 (PBS-T), and subsequently blocked with PBS-T plus 5% milk, for 60 min at room temperature. Thirty microliters of supernatants diluted 1:3 in PBS were added to the plates and incubated for 1-2 hour at ambient temperature. Afterwards, the plates were washed as before, and binding of antibodies was detected through Horseradish peroxidase (HRP)-conjugated goat F(ab')₂ anti-human IgG. The plates were developed with TMB and read at 450 nm. The amino acid sequences of top anti-CD137 hybridoma antibodies (clone# 29.39, 44.21, 3.10, 30.19 and 6.62) identified from the screening are shown in the SEQUENCE TABLE (SEQ ID NOS: 151-176) and Table 4.

**Table 4. Hybridoma anti-CD137 antibody sequence table**

| Clone Name | HC-CDR1 (SEQ ID NO) | HC-CDR2 (SEQ ID NO) | HC-CDR3 (SEQ ID NO) | LC-CDR1 (SEQ ID NO) | LC-CDR2 (SEQ ID NO) | LC-CDR3 (SEQ ID NO) |
|---|---|---|---|---|---|---|
| 29.39 | 151 | 154 | 157 | 160 | 164 | 167 |
| 44.21 | 151 | 154 | 157 | 162 | 166 | 169 |
| 3.10 | 152 | 155 | 158 | 163 | 166 | 169 |
| 30.19 | 153 | 156 | 159 | 160 | 164 | 167 |
| 6.62 | 153 | 156 | 159 | 161 | 165 | 168 |

### B. Humanization of anti-CD 137 antibodies

One representative clone 29.39 was selected for humanization of the framework. Briefly, Igblast was performed using the sequences of the clone to search database of human germline genes. Ideal germline sequences were selected, and mutations of framework sequences were made to change the framework sequences from mouse sequences to human sequences. For heavy chain, human germline IGHV1-46^{∗}01 was used, and the following mutations were made on framework: E1Q, K3Q, M5V, E6Q, L11V, 20V, T23K, K38R, R40A, E42G, I48M, D60A, L61Q, N62K, A67V, I69M, A71R, S75T, N76S, A78V, L80M, Q81E, T83R, S111V, L112S, and E113S. For light chain, human germline IGKV1-12^{∗}01 was used, and the following mutations were made on framework: V3Q, M11V, Y12S, L15V, E17D, F36Y, S43A, T46L, Q69T, Y71F, S72T, D79Q, Y80P, M83F, G84A, and I85T. The constructs were cloned into expression vectors, and antibody proteins were produced by SS320 cells.

### C. Affinity maturation, selection & modification

Affinity maturation was performed for the humanized 29.39 clone. Primers for making single mutation of amino acid for each CDR region were designed. A library of mutations was prepared using assembly PCR and cloned into phagemid vectors. Library quality was measured by transformation of TG1 cells and DNA sequencing of clones. Phage production was carried out using helper phages, and phage panning was performed using streptavidin-coupled Dynabeads coated with biotinylated human-CD 137 ECD or cynomolgus-CD137 ECD. After three round of panning, elution of panning products was used to infect SS320 cells, and colonies were picked and cultured in 2YT medium with IPTG. Binding affinities of Fab in supernatants were examined by ELISA assays. Positive clones against human- & cynomolgus-CD137 were selected. The top 14 binders (Clone# 3, 9, 23, 25, 33, 35, 5, 6, 17, 18, 19, 20, 2-9 and 2-11) and their CDRs (Kabat), VHs, VLs, heavy chains (HCs) and light chains (LCs) are shown in the SEQUENCE TABLE (SEQ ID NOS: 1-140). Consensus sequences of these CDRs (Kabat with modification), VHs, VLs, heavy chains (HCs) and light chains (LCs) are shown in the SEQUENCE TABLE (SEQ ID NOS: 141-150). Furthermore, subclones of 2-9 were generated, which comprises different IgG isotypes. Their CDRs (Kabat), VHs, VLs, heavy chains (HCs) and light chains (LCs) are shown in the SEQUENCE TABLE (SEQ ID NOS: 211-230).

### Example 2: Characterization of exemplary anti-CD137 antibodies

### A. Affinity assessment

Biolayer interferometry was used to measure the association and dissociation constants for the binding kinetics of the anti-CD137 antibodies. The binding kinetics was measured on a FortieBio Octet Red 96 at 30°C and analyzed with the FortieBio Data Analysis 9.0 software. Anti-human- IgG Fc (AHC) sensor was used to capture the anti-CD137 antibodies or control antibodies. A kinetic buffer only well was set as a reference well for subtraction during data processing. Data were fitted with a 1:1 Langmuir model for association and dissociation using Rmax linked global fitting for each antibody-antigen binding. Human CD137-his is a recombinant CD137 antigen with a poly histidine tag fused to C-terminus of human CD137 antigen (Sino Biological, 10041-H08H). Cyno CD137-his is a recombinant CD137 antigen with a poly histidine tag fused to C-terminus of cynomolgus CD137 antigen (Sino Biological, 90847-K02H-100).

As shown in FIGS. 1A-1B and Table 5 below, anti-CD137 clone 2-9 IgG2 effectively binds to human CD137 and cynomolgus monkey CD137 with a KD of 0.933 nM and 2.76 nM respectively. Assays using mouse CD137 did not show effective binding (data not shown).

**Table 5. Binding affinities of exemplary anti-CD137 monoclonal antibody**

| Samples | Antigen | Kd (M) | Kon (1/Ms) | Koff (1/s) |
|---|---|---|---|---|
| Anti-CD 137 2-9 IgG2 | Human CD137-his | 9.33E-10 | 9.31E+04 | 8.69E-04 |
| Anti-CD 137 2-9 IgG2 | Cyno CD137-his | 2.76E-09 | 2.58E+05 | 7.12E-04 |

FIGS. 1C-1D and Table 6 below show the binding results of another round of Octect binding assay for anti-CD137 clones, where 2-9 IgG2 and 2-9-1 IgG1 SELF effectively bind to human CD137 with a KD of 5.50 nM and 6.55 nM, respectively. The results demonstrated that clones 2-9 IgG2 and 2-9-1 IgG1 SELF had similar binding affinity to human CD137.

**Table 6. Binding affinities of exemplary anti-CD137 monoclonal antibody**

| Samples | Antigen | Kd (M) | Kon (1/Ms) | Koff (1/s) |
|---|---|---|---|---|
| 2-9 IgG2 | Human CD137-his | 5.50E-09 | 5.71E+04 | 3.14E-04 |
| 2-9-1 IgG1 SELF | Human CD137-his | 6.55E-09 | 5.20E+04 | 3 .40E-04 |

The Octet binding assay was also performed to compare the binding affinity of clones 2-9-1 IgG1 SELF and 2-9-2 IgG4. As shown in FIGS. 1E-1F and Table 7 below, anti-CD137 clones 2-9-1 IgG1 SELF and 2-9-2 IgG4 effectively binds to human CD137 with a KD of 11.1 nM and 12.9 nM, respectively. The results demonstrated that clones 2-9-1 IgG1 SELF and 2-9-2 IgG4 had similar binding affinity to human CD137.

**Table 7. Binding affinities of exemplary anti-CD137 monoclonal antibody**

| Samples | Antigen | Kd (M) | Kon (1/Ms) | Koff (1/s) |
|---|---|---|---|---|
| 2-9-1 IgG1 SELF | Human CD137-his | 1.11E-08 | 5.41E+04 | 6.00E-04 |
| 2-9-2 IgG4 | Human CD137-his | 1.29E-08 | 6.22E+04 | 8.01E-04 |

### B. CD137 reporter assay

Currently, there are several existing anti-CD137 antibodies in clinical ongoing. However, these antibodies are either associated with safety concerns or lack of efficacy. For example, urelumab, a strong agonist antibody to CD137 in Phase II clinical trial, is very effective in activating CD137, but causes severe liver toxicity at a low dose (≥ 1 mg/kg) due to its ability to activate CD137 signaling without crosslinking or clustering of the antibody/antigen complex. (Segal NH, et al. Clin Cancer Res. 2017 Apr 15;23(8): 1929-1936). Another existing anti-CD137 antibody, Utomilumab (PF-05082566), a weak agonist antibody to CD137 also in Phase II clinical trial, was shown to be safe at up to 10mg/kg but demonstrated limited efficacy in clinical trial. (Tolcher AW et al. Clin Cancer Res. 2017 Sep 15;23(18):5349-5357). Based on these findings, the next-generation of anti-CD137 monoclonal antibody should achieve high CD137 activation only specifically at tumor site. To select anti-CD137 antibodies that exhibit low toxicity and high efficacy, two reference antibodies were used for comparison. Reference Ab 1 was synthesized in-house based on the Urelumab sequences disclosed in U.S. Patent No. 7,288,638. Reference Ab 2 was synthesized in-house based on the Utomilumab sequences disclosed in International Publication No. WO 2012/032433.

The CD137 agonist activity of the anti-CD137 monospecific antibodies described in Example 1 was first evaluated in reporter assays. The NF-κB reporter cell line was established by stably transfected with CD137 in 293T cells and NF-κB Luciferase reporter gene. The FcγRIIB 293F cells was established by stably transfected with FcγRIIB in 293F cells. In this assay, FcγRIIB transfected 293F cells were used to mimic the monocytes and macrophage, which express high level of FcyRIIB, an inhibitory Fcγ receptor , and which are concentrated in tumor microenvironment but not peripheral tissues. The NF-κB reporter and CD137 double transfected reporter cell line 293T was used to mimic the T cells, which express CD137 on its surface and express NF-κB for downstream signaling. Therefore, co-culture of the CD137/NF-κB reporter cells with the FcγRIIB 293F cells mimics T cells in tumor microenvironment, whereas CD137/NF-κB reporter cells cultured alone mimics T cells in peripheral tissues.

The two reference antibodies used in this reporter system, reference antibody 1 represents Urelumab, which showed strong activity but also liver toxicity. Reference antibody 2 represents Utomilumab, which showed weak activity but no liver toxicity. The two references were used here to evaluate the activities as well as the potential safety profiles of the anti-CD137 clones.

Culture condition followed ATCC recommended medium and condition. FcγRIIB 293F cells was seeded into a 96-well plate for overnight. 60,000 cells per well per 100 ul medium were used for seeding. In certain experiments, mock 293F cells were seeded in 96 well plate in order to see the antibody effect without FcγRIIB cross-linking. On the second day, antibodies were added at a series of concentrations for 20 min at 37 °C. 120,000 293T reporter cells were then added into to the FcγRIIB 293T cells in DMEM medium (HyClone, Cat No. 16777-200). Negative controls were provided in the form of samples without antibody treatment and/or without cross-linking or effector cells. Five hours later, luciferase activity was measured using Bright-Glo^{™} Luciferase Assay System (Promega, Cat No. E2610) following manufacturer protocol.

The results showed a dose-dependent luciferase activity in the presence of anti-CD137 monoclonal antibodies. FIG. 2A shows CD137/NF-κB reporter cells alone treated with the antibodies, mimicking the CD137 activation in peripheral tissues, where inhibitory receptor FcγRIIB is not highly expressed and cannot bind to the Fc regions of the antibodies to facilitate cross-linking/CD137 clustering.

FIG 2B shows CD137/NF-κB reporter cells cultured with FcγRIIB 293F cells and treated with the antibodies, mimicking the CD137 activation in tumor site where inhibitory receptor FcγRIIB is highly expressed.

Reference Ab 1, a strong CD137 agonist, showed NF-κB activation with or without FcγRIIB mediated cross-linking/CD137 clustering, indicating that this reference antibody can activate CD137 signaling in peripheral tissues and cause peripheral toxicity. Reference antibody 2, a weak CD137 agonist, showed NF-κB activation only with FcγRIIB mediated cross-linking/CD137 clustering, consistent with this reference antibody's better safety profile in clinical trial. In FIG 2A, the anti-CD137 mAbs disclosed here behaved similarly to Reference antibody 2, indicating relatively safe clinical profile compared to Reference Ab 1. In FIG 2B, the anti-CD137 mAbs showed stronger immune activation compared to Reference Ab 2, indicating better therapeutic efficacy compared to Reference Ab 2. In particular, clone 2-9 showed almost no activity without FCγRIIB mediated cross-linking/ CD137 clustering while exhibited a superior activity with FcγRIIB mediated cross-linking/ CD137 clustering.

### C. CD137 reporter assay with anti-CD137 antibodies with Fc modifications

Recent publications showed that human IgG hinge and Fc can impact therapeutic antibody functions, especially for agonistic antibodies. For example, enhanced FcγIIB binding through Fc mutations can potentiate agonistic antibody function. It appeared that in vivo FcγRIIB binding through Fc region can mediate CD137 clustering, thus induce strong downstream signaling in target cells. (White AL et al. Cancer Immunol Immunother. 2013 May;62(5):941-8). Publications also highlighted that the conformation and flexibility of IgG2 Fc hinge region may affect agonistic activity for immune-costimulatory antibodies, such as anti-CD40 antibodies and anti-CD137 antibodies (White AL et al. Curr Top Microbiol Immunol. 2014;382:355-72). Based on those findings, it was hypothesized that anti-CD137 mAb IgG1 and IgG2 with Fc mutations to enhance FcγRIIB binding may improve efficacy compared to other isotypes, such as IgG2 wildtype. In addition, with higher expression of FcγRIIB in tumor site, this Fc engineered CD137 monoclonal antibody may keep low peripheral toxicity while induce higher efficacy at the tumor site.

Accordingly, the CD137 agonist activity of the anti-CD137 monoclonal antibodies with Fc mutations to enhance FcγRIIB binding (S267E/L328F) (Chu SY et al. Mol Immunol. 2008 Sep;45(15):3926-33) were evaluated in the reporter assay and compared to parental IgG2 wildtype antibody.

Additionally, IgG4 isoform is known to have reduced effector binding, which may reduce peripheral toxicity while induce antitumor efficacy at the tumor site. Accordingly, the anti-CD137 monoclonal antibodies were also tested in IgG4 isoform.

The results showed a dose-dependent luciferase activity in the presence of anti-CD137 monoclonal antibodies. As shown in FIG. 3A, all 2-9 clones (with or without Fc mutation) showed no activity without FCγRIIB mediated cross-linking/CD137 clustering, indicating safe clinical profile. FIG. 3B shows NF-κB reporter signal with FcγRIIB mediated cross-linking/CD137 clustering, mimicking the CD137 activation in tumor site where inhibitory receptor FcγRIIB is highly expressed. Clone 2-9 IgG1 SELF and IgG2 SELF showed higher CD137 activation compared to IgG2 wildtype parental antibody, indicating enhanced CD137 activation through enhanced FCγRIIB binding. FIG 3C shows NF-κB reporter signal comparing 2-9-1 IgG1 SELF and 2-9-2 IgG4 with FcγRIIB mediated cross-linking/CD137 clustering. Both clones showed high CD137 activation, and 2-9-1 IgG1 SELF showed higher activity compared to 2-9-2 IgG4.

### D. Cytokine release assay

Human peripheral blood mononuclear cells (PBMCs) were then used to test the cytokine release effects of the anti-CD137 mAbs. PBMCs contain CD137 positive T cells and NK cells, which are the target cells of the anti-CD137 monoclonal antibodies, and the FcγRIIB expressing monocytes and macrophages were also present in the PBMC. Once T cells were activated by anti-CD137 antibodies, anti-tumor cytokines such as IFN-γ or IL-2 were secreted as an indication for anti-tumor efficacy. The two reference antibodies were used in this reporter system as described above to evaluate the activities of the anti-CD137 clones.

Human PBMCs were isolated using Ficoll-Paque^{®} PLUS (Cat. No. 17-1440-02, GE Healthcare). Anti-CD3 antibody (OKT3) 1ug/ml and serial dilution of anti-CD137 mAbs were added into PBMCs in Gibco RPMI medium 1640 (Cat. No. A1049101, Thermo Fischer). 48 hour later, media were collected, and IFNγ and IL2 levels were determined using ELISA method (Human IFN-γ ELISA MAX^{™} Deluxe, Cat. No. 430106, BioLegend; Human IL2 ELISA MAX^{™} Deluxe, Cat. No. 421601, BioLegend).

As shown in FIG. 4A, a dose-dependent secretion of IFNγ by the effector cells (*i.e.*, PBMCs) was observed in the presence of anti-CD137 antibodies. As expected, reference antibody 1, a strong agonist, induced higher IFN-γ secretion compared to reference antibody 2, a weak agonist. Furthermore, clone 2-9 with different Fc regions, such as 2-9-1 IgG1 SELF, 2-9 IgG2 wildtype and 2-9-1 IgG2 SELF all showed significant higher IFN-γ maximum release compared to the two reference antibodies. The experiments were repeated three times with different donors, and similar results were observed.

As shown in FIG. 4B, a dose-dependent secretion of IL2 by the effector cells (i.e., PBMCs) was observed in the presence of anti-CD137 antibodies. As expected, reference antibody 1, a strong agonist, induced IL-2 secretion. Furthermore, clone 2-9 with different Fc regions, i.e., 2-9-1 IgG1 SELF and 2-9-2 IgG4, both showed significantly higher IL2 maximum release compared to reference antibody 1.

In conclusion, the 2-9 mAbs induced cytokine production in a dose-dependent manner, and induced higher IFN-γ and IL2 levels compared to the two reference antibodies. As IFN-γ and IL2 are important antitumor cytokines, the data indicate that clone 2-9 antibodies, including Fc mutations and wild type, have enhanced antitumor efficacy compared to the two reference antibodies.

### E. In vivo antitumor efficacy study

The 2-9 variants were further tested in vivo in a Biocytogen facility to evaluate their antitumor efficacy. Using h-CD137(a.k.a. 4-1BB) Knock-In C57BL/6 mice and MC38 murine colon cancer model, efficacies of 2-9 IgG2 wt, 2-9-1 IgG2 SELF and 2-9-1 IgG1 SELF were studied and compared to vehicle control. MC38 tumor cells were implanted one week before treatment. Treatment started when tumor volume reached approximately 100 mm³, where drugs were dosed intraperitoneally twice a week for 4 weeks at 10, 1, 0.3 mg/kg for 2-9-1-IgG1 SELF antibody, or 1 mg/kg for both 2-9-1-IgG2 SELF and 2-9-IgG2 wt antibodies. On day 24 post-treatment, tumor volumes in multiple mice of the control group reached tumor size limits (2000 mm³) for termination of the procedure. Other groups continued treatment and tumor measurement until day 28. Day 28 post-treatment was the data end point for the analyses. The treatment with 2-9 IgG2 wt, 2-9-1 IgG1 SELF and 2-9-1 IgG2 SELF significantly reduced tumor growth compared to vehicle control as shown in FIG. 5A and Table 8. Comparing the treatment with 2-9 IgG2 wt, 2-9-1 IgG1 SELF and 2-9-1 IgG2 SELF using the same dosage regime (1 mg/kg), the number of animals that reached tumor volume below 100 mm³ out of the total number of animals in each group (N=8) is 2/8, 5/8 and 5/8, respectively. Body weight changes were not significant between the groups throughout the study (FIG. 5B), indicating the treatments were well tolerated.

**Table 8. In vivo antitumor efficacy of exemplary anti-CD137 antibodies**

| **Groups** | **Test articles** | **Tumor volume (mm³)^{a}** | | **TGI (%)** | **P^{b}** |
|---|---|---|---|---|---|
| | | **Before treatment** | **Day 24 post treatment** | | |
| G1 | Vehicle | 117±4 | 2006±240 | - | - |
| G2 | 2-9-1 IGG1 SELF (10 mg/kg) | 117±5 | 46±16 | 103.8 | ^{∗∗∗}<0.001 |
| G3 | 2-9-1 IgG1 SELF (1 mg/kg) | 118±5 | 155±72 | 98.0 | ^{∗∗∗}<0.001 |
| G4 | 2-9-1 IgG1 SELF (0.3 m g /k g) | 118±5 | 340±125 | 88.2 | ^{∗∗∗}<0.001 |
| G5 | 2-9-1 IgG2 SELF (1 mg/kg) | 118±4 | 94±42 | 101.3 | ^{∗∗∗}<0.001 |
| G6 | 2-9 IgG2 WT (1 mg/kg) | 118±6 | 371±109 | 86.6 | ^{∗∗∗}<0.001 |

| | | | | | |
|---|---|---|---|---|---|
| ^{a}: Mean ± SEM; ^{b}: T-test on tumor volume of each treatment group versus Vehicle control on day 24 post-treatment. | | | | | |

Furthermore, Biocytogen previously tested a utomilumab analog in the MC38 murine colon cancer model using h-CD137(a.k.a. 4-1BB) Knock-In C57BL/6 mice under conditions similar to the above study for 2-9 variants. As shown in FIG. 6A, the utomilumab analog was tested using two dosage regimens, 1 mg/kg and 10 mg/kg. Compared to an IgG control antibody, the 1 mg/kg treatment group resulted in a Tumor Growth Inhibition (TGI) of 60.0%, and the10 mg/kg treatment group resulted in a TGI of 85.4% (the results can also be found at www.biocytogen.com). Utomilumab is known to have a relatively safe toxicity profile, and FIG. 6B shows that the utomilumab treatment did not change mouse body weight significant.

As this study was conducted under conditions similar to the study of the 2-9 variants, the data of the 2-9 variants and the utomilumab analog are comparable. In particular, the 2-9 variants resulted in a higher TGI compared to the TGIs of the utomilumab analog treatment regardless of the dosage regimes (FIG. 5A, 6A and Table 8). When compared at the same dosage level of 10 mg/kg, the TGI of 2-9-1 IgG1 SELF (103.8%) is significantly higher that the TGI of the utomilumab analog (85.4%). These results indicate that each of the 2-9 variants is more efficacious in vivo compared to the utomilumab analog.

In addition to the various embodiments depicted and claimed, the disclosed subject matter is also directed to other embodiments having other combinations of the features disclosed and claimed herein. As such, the particular features presented herein can be combined with each other in other manners within the scope of the disclosed subject matter such that the disclosed subject matter includes any suitable combination of the features disclosed herein. The foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the compositions and methods of the disclosed subject matter without departing from the spirit or scope of the disclosed subject matter. Thus, it is intended that the disclosed subject matter include modifications and variations that are within the scope of the appended claims and their equivalents.

Various publications, patents and patent applications are cited herein, the contents of which are hereby incorporated by reference in their entireties.

## Claims

1. An isolated anti-CD137 construct comprising an antibody moiety that binds to CD137 comprising a heavy chain variable region (V_{H}) and a light chain variable region (V_{L}), wherein:
a) the V_{H} comprises:
i) an HC-CDR1 comprising an amino acid sequence of GFX₁X₂X₃DTYIX₄ (SEQ ID NO: 177), wherein X₁=N or C; X₂=I, P, L, or M; X₃=K, N, R, C or Q; X₄=H or Q,
ii) an HC-CDR2 comprising an amino acid sequence of X₁IDPANGX₂X₃X₄ (SEQ ID NO:178), wherein X₁=K or R; X₂=N, G, F, Y, A, D, L, M, or Q; X₃=S or T; X₄=E or M, and
iii) an HC-CDR3 comprising an amino acid sequence of GNLHYX₁LMD (SEQ ID NO: 179), wherein X₁=Y, A, or G; and
b) the V_{L} comprises:
i) an LC-CDR1 comprising an amino acid sequence of KASQX₁X₂X₃TYX₄S (SEQ ID NO: 180), wherein X₁=A, P or T; X₂=I, T or P; X₃=N or A; X₄=L, G or H,
ii) an LC-CDR2 comprising an amino acid sequence of RX₁NRX₂X₃X₄ (SEQ ID NO: 181), wherein X₁=A, Y, V or D; X₂=M, K, V or A; X₃=V, P, Y or G; X₄=D or G, and
iii) an LC-CDR3 comprising an amino acid sequence of LQX₁X₂DFPYX₃ (SEQ ID NO: 182), wherein X₁=Y, S or F; X₂=D, V, L, R, E or Q; X₃=T or K.

2. The anti-CD137 construct of claim 1, wherein:
a) the HC-CDR1 comprises an amino acid sequence of any one of SEQ ID NOs: 1, 11, 21, 31, 41, 51, 61, 71, 81, 91, 101, 111, 121, 131, 141, 211 and 221, or a variant thereof comprising up to about 3 amino acid substitutions;
b) the HC-CDR2 comprises an amino acid sequence of any one of SEQ ID NOs: 2, 12, 22, 32, 42, 52, 62, 72, 82, 92, 102, 112, 122, 132, 142, 212 and 222, or a variant thereof comprising up to about 3 amino acid substitutions;
c) the HC-CDR3 comprises an amino acid sequence of any one of SEQ ID NOs: 3, 13, 23, 33, 43, 53, 63, 73, 83, 93, 103, 113, 123, 133, 143, 213 and 223, or a variant thereof comprising up to about 3 amino acid substitutions;
d) the LC-CDR1 comprises an amino acid sequence of any one of SEQ ID NOs: 4, 14, 24, 34, 44, 54, 64, 74, 84, 94, 104, 114, 124, 134, 144, 214 and 224, or a variant thereof comprising up to about 3 amino acid substitutions;
e) the LC-CDR2 comprises an amino acid sequence of any one of SEQ ID NOs: 5, 15, 25, 35, 45, 55, 65, 75, 85, 95, 105, 115, 125, 135, 145, 215 and 225, or a variant thereof comprising up to about 3 amino acid substitutions; and
f) the LC-CDR3 comprises an amino acid sequence of any one of SEQ ID NOs: 6, 16, 26, 36, 46, 56, 66, 76, 86, 96, 106, 116, 126, 136, 146, 216 and 226, or a variant thereof comprising up to about 3 amino acid substitutions.

3. An anti-CD137 construct comprising an anti-CD137 antibody moiety that cross-competes for binding to CD137 with a reference anti-CD137 construct comprising a heavy chain variable region (V_{H}) comprising HC-CDR1, HC-CDR2 and HC-CDR3 domains and a light chain variable region (V_{L}) comprising LC-CDR1, LC-CDR2 and LC-CDR3 domains that are selected from the group consisting of:
a) the V_{H} comprises the HC -CDR1 comprising the amino acid sequence of SEQ ID NO: 1, the HC -CDR2 comprising the amino acid sequence of SEQ ID NO: 2, and the HC - CDR3 comprising the amino acid sequence of SEQ ID NO: 3, and the V_{L} comprises the LC - CDR1 comprising the amino acid sequence of SEQ ID NO: 4, the LC -CDR2 comprising the amino acid sequence of SEQ ID NO: 5, and the LC -CDR3 comprising the amino acid sequence of SEQ ID NO: 6;
b) the V_{H} comprises the HC -CDR1 comprising the amino acid sequence of SEQ ID NO: 11, the HC -CDR2 comprising the amino acid sequence of SEQ ID NO: 12, and the HC -CDR3 comprising the amino acid sequence of SEQ ID NO: 13, and the V_{L} comprises the LC -CDR1 comprising the amino acid sequence of SEQ ID NO: 14, the LC -CDR2 comprising the amino acid sequence of SEQ ID NO: 15, and the LC -CDR3 comprising the amino acid sequence of SEQ ID NO: 16;
c) the V_{H} comprises the HC -CDR1 comprising the amino acid sequence of SEQ ID NO: 21, the HC -CDR2 comprising the amino acid sequence of SEQ ID NO: 22, and the HC -CDR3 comprising the amino acid sequence of SEQ ID NO: 23, and the V_{L} comprises the LC -CDR1 comprising the amino acid sequence of SEQ ID NO: 24, the LC -CDR2 comprising the amino acid sequence of SEQ ID NO: 25, and the LC -CDR3 comprising the amino acid sequence of SEQ ID NO: 26;
d) the V_{H} comprises the HC -CDR1 comprising the amino acid sequence of SEQ ID NO: 31, the HC -CDR2 comprising the amino acid sequence of SEQ ID NO: 32, and the HC -CDR3 comprising the amino acid sequence of SEQ ID NO: 33, and the V_{L} comprises the LC -CDR1 comprising the amino acid sequence of SEQ ID NO: 34, the LC -CDR2 comprising the amino acid sequence of SEQ ID NO: 35, and the LC -CDR3 comprising the amino acid sequence of SEQ ID NO: 36;
e) the V_{H} comprises the HC -CDR1 comprising the amino acid sequence of SEQ ID NO: 41, the HC -CDR2 comprising the amino acid sequence of SEQ ID NO: 42, and the HC -CDR3 comprising the amino acid sequence of SEQ ID NO: 43, and the V_{L} comprises the LC -CDR1 comprising the amino acid sequence of SEQ ID NO: 44, the LC -CDR2 comprising the amino acid sequence of SEQ ID NO: 45, and the LC -CDR3 comprising the amino acid sequence of SEQ ID NO: 46;
f) the V_{H} comprises the HC -CDR1 comprising the amino acid sequence of SEQ ID NO: 51, the HC -CDR2 comprising the amino acid sequence of SEQ ID NO: 52, and the HC -CDR3 comprising the amino acid sequence of SEQ ID NO: 53, and the V_{L} comprises the LC -CDR1 comprising the amino acid sequence of SEQ ID NO: 54, the LC -CDR2 comprising the amino acid sequence of SEQ ID NO: 55, and the LC -CDR3 comprising the amino acid sequence of SEQ ID NO: 56;
g) the V_{H} comprises the HC -CDR1 comprising the amino acid sequence of SEQ ID NO: 61, the HC -CDR2 comprising the amino acid sequence of SEQ ID NO: 62, and the HC -CDR3 comprising the amino acid sequence of SEQ ID NO: 63, and the V_{L} comprises the LC -CDR1 comprising the amino acid sequence of SEQ ID NO: 64, the LC -CDR2 comprising the amino acid sequence of SEQ ID NO: 65, and the LC -CDR3 comprising the amino acid sequence of SEQ ID NO: 66;
h) the V_{H} comprises the HC -CDR1 comprising the amino acid sequence of SEQ ID NO: 71, the HC -CDR2 comprising the amino acid sequence of SEQ ID NO: 72, and the HC -CDR3 comprising the amino acid sequence of SEQ ID NO: 73, and the V_{L} comprises the LC -CDR1 comprising the amino acid sequence of SEQ ID NO: 74, the LC -CDR2 comprising the amino acid sequence of SEQ ID NO: 75, and the LC -CDR3 comprising the amino acid sequence of SEQ ID NO: 76;
i) the V_{H} comprises the HC -CDR1 comprising the amino acid sequence of SEQ ID NO: 81, the HC -CDR2 comprising the amino acid sequence of SEQ ID NO: 82, and the HC -CDR3 comprising the amino acid sequence of SEQ ID NO: 83, and the V_{L} comprises the LC -CDR1 comprising the amino acid sequence of SEQ ID NO: 84, the LC -CDR2 comprising the amino acid sequence of SEQ ID NO: 85, and the LC -CDR3 comprising the amino acid sequence of SEQ ID NO: 86;
j) the V_{H} comprises the HC -CDR1 comprising the amino acid sequence of SEQ ID NO: 91, the HC -CDR2 comprising the amino acid sequence of SEQ ID NO: 92, and the HC -CDR3 comprising the amino acid sequence of SEQ ID NO: 93, and the V_{L} comprises the LC -CDR1 comprising the amino acid sequence of SEQ ID NO: 94, the LC -CDR2 comprising the amino acid sequence of SEQ ID NO: 95, and the LC -CDR3 comprising the amino acid sequence of SEQ ID NO: 96;
k) the V_{H} comprises the HC -CDR1 comprising the amino acid sequence of SEQ ID NO: 101, the HC -CDR2 comprising the amino acid sequence of SEQ ID NO: 102, and the HC -CDR3 comprising the amino acid sequence of SEQ ID NO: 103, and the V_{L} comprises the LC -CDR1 comprising the amino acid sequence of SEQ ID NO: 104, the LC -CDR2 comprising the amino acid sequence of SEQ ID NO: 105, and the LC -CDR3 comprising the amino acid sequence of SEQ ID NO:106;
l) the V_{H} comprises the HC -CDR1 comprising the amino acid sequence of SEQ ID NO: 111, the HC -CDR2 comprising the amino acid sequence of SEQ ID NO: 112, and the HC -CDR3 comprising the amino acid sequence of SEQ ID NO: 113, and the V_{L} comprises the LC -CDR1 comprising the amino acid sequence of SEQ ID NO: 114, the LC -CDR2 comprising the amino acid sequence of SEQ ID NO: 115, and the LC -CDR3 comprising the amino acid sequence of SEQ ID NO: 116;
m) the V_{H} comprises the HC -CDR1 comprising the amino acid sequence of SEQ ID NO: 121, the HC -CDR2 comprising the amino acid sequence of SEQ ID NO: 122, and the HC -CDR3 comprising the amino acid sequence of SEQ ID NO: 123, and the V_{L} comprises the LC -CDR1 comprising the amino acid sequence of SEQ ID NO: 124, the LC -CDR2 comprising the amino acid sequence of SEQ ID NO: 125, and the LC -CDR3 comprising the amino acid sequence of SEQ ID NO: 126;
n) the V_{H} comprises the HC -CDR1 comprising the amino acid sequence of SEQ ID NO: 131, the HC -CDR2 comprising the amino acid sequence of SEQ ID NO: 132, and the HC -CDR3 comprising the amino acid sequence of SEQ ID NO: 133, and the V_{L} comprises the LC -CDR1 comprising the amino acid sequence of SEQ ID NO: 134, the LC -CDR2 comprising the amino acid sequence of SEQ ID NO: 135, and the LC -CDR3 comprising the amino acid sequence of SEQ ID NO: 136;
o) the V_{H} comprises the HC -CDR1 comprising the amino acid sequence of SEQ ID NO: 211, the HC -CDR2 comprising the amino acid sequence of SEQ ID NO: 212, and the HC -CDR3 comprising the amino acid sequence of SEQ ID NO: 213, and the V_{L} comprises the LC -CDR1 comprising the amino acid sequence of SEQ ID NO: 214, the LC -CDR2 comprising the amino acid sequence of SEQ ID NO: 215, and the LC -CDR3 comprising the amino acid sequence of SEQ ID NO: 216; and
p) the V_{H} comprises the HC -CDR1 comprising the amino acid sequence of SEQ ID NO: 221, the HC -CDR2 comprising the amino acid sequence of SEQ ID NO: 222, and the HC -CDR3 comprising the amino acid sequence of SEQ ID NO: 223, and the V_{L} comprises the LC -CDR1 comprising the amino acid sequence of SEQ ID NO: 224, the LC -CDR2 comprising the amino acid sequence of SEQ ID NO: 225, and the LC -CDR3 comprising the amino acid sequence of SEQ ID NO: 226.

4. The anti-CD137 construct of any one of claim 1-3, comprising an anti-CD137 antibody moiety comprising a heavy chain variable region (V_{H}) that comprises HC -CDR1, HC -CDR2, and HC -CDR3 domains; and a light chain variable region (V_{L}) that comprises LC -CDR1, LC -CDR2, and LC -CDR3 domains, wherein the V_{H} and the V_{L} are selected from the group consisting of:
a) the V_{H} comprises the HC-CDR1 comprising the amino acid sequence of SEQ ID NO: 1, the HC-CDR2 comprising the amino acid sequence of SEQ ID NO: 2, and the HC - CDR3 comprising the amino acid sequence of SEQ ID NO: 3, and the V_{L} comprises the LC-CDR1 comprising the amino acid sequence of SEQ ID NO: 4, the LC-CDR2 comprising the amino acid sequence of SEQ ID NO: 5, and the LC-CDR3 comprising the amino acid sequence of SEQ ID NO: 6;
b) the V_{H} comprises the HC -CDR1 comprising the amino acid sequence of SEQ ID NO: 11, the HC -CDR2 comprising the amino acid sequence of SEQ ID NO: 12, and the HC -CDR3 comprising the amino acid sequence of SEQ ID NO: 13, and the V_{L} comprises the LC -CDR1 comprising the amino acid sequence of SEQ ID NO: 14, the LC -CDR2 comprising the amino acid sequence of SEQ ID NO: 15, and the LC -CDR3 comprising the amino acid sequence of SEQ ID NO: 16;
c) the V_{H} comprises the HC -CDR1 comprising the amino acid sequence of SEQ ID NO: 21, the HC -CDR2 comprising the amino acid sequence of SEQ ID NO: 22, and the HC -CDR3 comprising the amino acid sequence of SEQ ID NO: 23, and the V_{L} comprises the LC -CDR1 comprising the amino acid sequence of SEQ ID NO: 24, the LC -CDR2 comprising the amino acid sequence of SEQ ID NO: 25, and the LC -CDR3 comprising the amino acid sequence of SEQ ID NO: 26;
d) the V_{H} comprises the HC -CDR1 comprising the amino acid sequence of SEQ ID NO: 31, the HC -CDR2 comprising the amino acid sequence of SEQ ID NO: 32, and the HC -CDR3 comprising the amino acid sequence of SEQ ID NO: 33, and the V_{L} comprises the LC -CDR1 comprising the amino acid sequence of SEQ ID NO: 34, the LC -CDR2 comprising the amino acid sequence of SEQ ID NO: 35, and the LC -CDR3 comprising the amino acid sequence of SEQ ID NO: 36;
e) the V_{H} comprises the HC -CDR1 comprising the amino acid sequence of SEQ ID NO: 41, the HC -CDR2 comprising the amino acid sequence of SEQ ID NO: 42, and the HC -CDR3 comprising the amino acid sequence of SEQ ID NO: 43, and the V_{L} comprises the LC -CDR1 comprising the amino acid sequence of SEQ ID NO: 44, the LC -CDR2 comprising the amino acid sequence of SEQ ID NO: 45, and the LC -CDR3 comprising the amino acid sequence of SEQ ID NO: 46;
f) the V_{H} comprises the HC -CDR1 comprising the amino acid sequence of SEQ ID NO: 51, the HC -CDR2 comprising the amino acid sequence of SEQ ID NO: 52, and the HC -CDR3 comprising the amino acid sequence of SEQ ID NO: 53, and the V_{L} comprises the LC -CDR1 comprising the amino acid sequence of SEQ ID NO: 54, the LC -CDR2 comprising the amino acid sequence of SEQ ID NO: 55, and the LC -CDR3 comprising the amino acid sequence of SEQ ID NO: 56;
g) the V_{H} comprises the HC -CDR1 comprising the amino acid sequence of SEQ ID NO: 61, the HC -CDR2 comprising the amino acid sequence of SEQ ID NO: 62, and the HC -CDR3 comprising the amino acid sequence of SEQ ID NO: 63, and the V_{L} comprises the LC -CDR1 comprising the amino acid sequence of SEQ ID NO: 64, the LC -CDR2 comprising the amino acid sequence of SEQ ID NO: 65, and the LC -CDR3 comprising the amino acid sequence of SEQ ID NO: 66;
h) the V_{H} comprises the HC -CDR1 comprising the amino acid sequence of SEQ ID NO: 71, the HC -CDR2 comprising the amino acid sequence of SEQ ID NO: 72, and the HC -CDR3 comprising the amino acid sequence of SEQ ID NO: 73, and the V_{L} comprises the LC -CDR1 comprising the amino acid sequence of SEQ ID NO: 74, the LC -CDR2 comprising the amino acid sequence of SEQ ID NO: 75, and the LC -CDR3 comprising the amino acid sequence of SEQ ID NO: 76;
i) the V_{H} comprises the HC -CDR1 comprising the amino acid sequence of SEQ ID NO: 81, the HC -CDR2 comprising the amino acid sequence of SEQ ID NO: 82, and the HC -CDR3 comprising the amino acid sequence of SEQ ID NO: 83, and the V_{L} comprises the LC -CDR1 comprising the amino acid sequence of SEQ ID NO: 84, the LC -CDR2 comprising the amino acid sequence of SEQ ID NO: 85, and the LC -CDR3 comprising the amino acid sequence of SEQ ID NO: 86;
j) the V_{H} comprises the HC -CDR1 comprising the amino acid sequence of SEQ ID NO: 91, the HC -CDR2 comprising the amino acid sequence of SEQ ID NO: 92, and the HC -CDR3 comprising the amino acid sequence of SEQ ID NO: 93, and the V_{L} comprises the LC -CDR1 comprising the amino acid sequence of SEQ ID NO: 94, the LC -CDR2 comprising the amino acid sequence of SEQ ID NO: 95, and the LC -CDR3 comprising the amino acid sequence of SEQ ID NO: 96;
k) the V_{H} comprises the HC -CDR1 comprising the amino acid sequence of SEQ ID NO: 101, the HC -CDR2 comprising the amino acid sequence of SEQ ID NO: 102, and the HC -CDR3 comprising the amino acid sequence of SEQ ID NO: 103, and the V_{L} comprises the LC -CDR1 comprising the amino acid sequence of SEQ ID NO: 104, the LC -CDR2 comprising the amino acid sequence of SEQ ID NO: 105, and the LC -CDR3 comprising the amino acid sequence of SEQ ID NO:106;
l) the V_{H} comprises the HC -CDR1 comprising the amino acid sequence of SEQ ID NO: 111, the HC -CDR2 comprising the amino acid sequence of SEQ ID NO: 112, and the HC -CDR3 comprising the amino acid sequence of SEQ ID NO: 113, and the V_{L} comprises the LC -CDR1 comprising the amino acid sequence of SEQ ID NO: 114, the LC -CDR2 comprising the amino acid sequence of SEQ ID NO: 115, and the LC -CDR3 comprising the amino acid sequence of SEQ ID NO: 116;
m) the V_{H} comprises the HC -CDR1 comprising the amino acid sequence of SEQ ID NO: 121, the HC -CDR2 comprising the amino acid sequence of SEQ ID NO: 122, and the HC -CDR3 comprising the amino acid sequence of SEQ ID NO: 123, and the V_{L} comprises the LC -CDR1 comprising the amino acid sequence of SEQ ID NO: 124, the LC -CDR2 comprising the amino acid sequence of SEQ ID NO: 125, and the LC -CDR3 comprising the amino acid sequence of SEQ ID NO: 126;
n) the V_{H} comprises the HC -CDR1 comprising the amino acid sequence of SEQ ID NO: 131, the HC -CDR2 comprising the amino acid sequence of SEQ ID NO: 132, and the HC -CDR3 comprising the amino acid sequence of SEQ ID NO: 133, and the V_{L} comprises the LC -CDR1 comprising the amino acid sequence of SEQ ID NO: 134, the LC -CDR2 comprising the amino acid sequence of SEQ ID NO: 135, and the LC -CDR3 comprising the amino acid sequence of SEQ ID NO: 136;
o) the V_{H} comprises the HC -CDR1 comprising the amino acid sequence of SEQ ID NO: 211, the HC -CDR2 comprising the amino acid sequence of SEQ ID NO: 212, and the HC -CDR3 comprising the amino acid sequence of SEQ ID NO: 213, and the V_{L} comprises the LC -CDR1 comprising the amino acid sequence of SEQ ID NO: 214, the LC -CDR2 comprising the amino acid sequence of SEQ ID NO: 215, and the LC -CDR3 comprising the amino acid sequence of SEQ ID NO: 216; and
p) the V_{H} comprises the HC -CDR1 comprising the amino acid sequence of SEQ ID NO: 221, the HC -CDR2 comprising the amino acid sequence of SEQ ID NO: 222, and the HC -CDR3 comprising the amino acid sequence of SEQ ID NO: 223, and the V_{L} comprises the LC -CDR1 comprising the amino acid sequence of SEQ ID NO: 224, the LC -CDR2 comprising the amino acid sequence of SEQ ID NO: 225, and the LC -CDR3 comprising the amino acid sequence of SEQ ID NO: 226.

5. The anti-CD137 construct of any one of claim 1-4, wherein the V_{H} comprises the HC -CDR1 comprising the amino acid sequence of SEQ ID NO: 1, the HC -CDR2 comprising the amino acid sequence of SEQ ID NO: 2, and the HC -CDR3 comprising the amino acid sequence of SEQ ID NO: 3, and the V_{L} comprises the LC -CDR1 comprising the amino acid sequence of SEQ ID NO: 4, the LC -CDR2 comprising the amino acid sequence of SEQ ID NO: 5, and the LC -CDR3 comprising the amino acid sequence of SEQ ID NO: 6.

6. The anti-CD137 construct of any one of claim 1-4, wherein the V_{H} comprises the HC -CDR1 comprising the amino acid sequence of SEQ ID NO: 11, the HC -CDR2 comprising the amino acid sequence of SEQ ID NO: 12, and the HC -CDR3 comprising the amino acid sequence of SEQ ID NO: 13, and the V_{L} comprises the LC -CDR1 comprising the amino acid sequence of SEQ ID NO: 14, the LC -CDR2 comprising the amino acid sequence of SEQ ID NO: 15, and the LC -CDR3 comprising the amino acid sequence of SEQ ID NO: 16.

7. The anti-CD137 construct of any one of claim 1-4, wherein the V_{H} comprises the HC -CDR1 comprising the amino acid sequence of SEQ ID NO: 21, the HC -CDR2 comprising the amino acid sequence of SEQ ID NO: 22, and the HC -CDR3 comprising the amino acid sequence of SEQ ID NO: 23, and the V_{L} comprises the LC -CDR1 comprising the amino acid sequence of SEQ ID NO: 24, the LC -CDR2 comprising the amino acid sequence of SEQ ID NO: 25, and the LC -CDR3 comprising the amino acid sequence of SEQ ID NO: 26.

8. The anti-CD137 construct of any one of claim 1-4, wherein the V_{H} comprises the HC -CDR1 comprising the amino acid sequence of SEQ ID NO: 31, the HC -CDR2 comprising the amino acid sequence of SEQ ID NO: 32, and the HC -CDR3 comprising the amino acid sequence of SEQ ID NO: 33, and the V_{L} comprises the LC -CDR1 comprising the amino acid sequence of SEQ ID NO: 34, the LC -CDR2 comprising the amino acid sequence of SEQ ID NO: 35, and the LC -CDR3 comprising the amino acid sequence of SEQ ID NO: 36.

9. The anti-CD137 construct of any one of claim 1-4, wherein the V_{H} comprises the HC -CDR1 comprising the amino acid sequence of SEQ ID NO: 41, the HC -CDR2 comprising the amino acid sequence of SEQ ID NO: 42, and the HC -CDR3 comprising the amino acid sequence of SEQ ID NO: 43, and the V_{L} comprises the LC -CDR1 comprising the amino acid sequence of SEQ ID NO: 44, the LC -CDR2 comprising the amino acid sequence of SEQ ID NO: 45, and the LC -CDR3 comprising the amino acid sequence of SEQ ID NO: 46.

10. The anti-CD137 construct of any one of claim 1-4, wherein the V_{H} comprises the HC -CDR1 comprising the amino acid sequence of SEQ ID NO: 51, the HC -CDR2 comprising the amino acid sequence of SEQ ID NO: 52, and the HC -CDR3 comprising the amino acid sequence of SEQ ID NO: 53, and the V_{L} comprises the LC -CDR1 comprising the amino acid sequence of SEQ ID NO: 54, the LC -CDR2 comprising the amino acid sequence of SEQ ID NO: 55, and the LC -CDR3 comprising the amino acid sequence of SEQ ID NO: 56.

11. The anti-CD137 construct of any one of claim 1-4, wherein the V_{H} comprises the HC -CDR1 comprising the amino acid sequence of SEQ ID NO: 61, the HC -CDR2 comprising the amino acid sequence of SEQ ID NO: 62, and the HC -CDR3 comprising the amino acid sequence of SEQ ID NO: 63, and the V_{L} comprises the LC -CDR1 comprising the amino acid sequence of SEQ ID NO: 64, the LC -CDR2 comprising the amino acid sequence of SEQ ID NO: 65, and the LC -CDR3 comprising the amino acid sequence of SEQ ID NO: 66.

12. The anti-CD137 construct of any one of claim 1-4, wherein the V_{H} comprises the HC -CDR1 comprising the amino acid sequence of SEQ ID NO: 71, the HC -CDR2 comprising the amino acid sequence of SEQ ID NO: 72, and the HC -CDR3 comprising the amino acid sequence of SEQ ID NO: 73, and the V_{L} comprises the LC -CDR1 comprising the amino acid sequence of SEQ ID NO: 74, the LC -CDR2 comprising the amino acid sequence of SEQ ID NO: 75, and the LC -CDR3 comprising the amino acid sequence of SEQ ID NO: 76.

13. The anti-CD137 construct of any one of claim 1-4, wherein the V_{H} comprises the HC -CDR1 comprising the amino acid sequence of SEQ ID NO: 81, the HC -CDR2 comprising the amino acid sequence of SEQ ID NO: 82, and the HC -CDR3 comprising the amino acid sequence of SEQ ID NO: 83, and the V_{L} comprises the LC -CDR1 comprising the amino acid sequence of SEQ ID NO: 84, the LC -CDR2 comprising the amino acid sequence of SEQ ID NO: 85, and the LC -CDR3 comprising the amino acid sequence of SEQ ID NO: 86.

14. The anti-CD137 construct of any one of claim 1-4, wherein the V_{H} comprises the HC -CDR1 comprising the amino acid sequence of SEQ ID NO: 91, the HC -CDR2 comprising the amino acid sequence of SEQ ID NO: 92, and the HC -CDR3 comprising the amino acid sequence of SEQ ID NO: 93, and the V_{L} comprises the LC -CDR1 comprising the amino acid sequence of SEQ ID NO: 94, the LC -CDR2 comprising the amino acid sequence of SEQ ID NO: 95, and the LC -CDR3 comprising the amino acid sequence of SEQ ID NO: 96.

15. The anti-CD137 construct of any one of claim 1-4, wherein the V_{H} comprises the HC -CDR1 comprising the amino acid sequence of SEQ ID NO: 101, the HC -CDR2 comprising the amino acid sequence of SEQ ID NO: 102, and the HC -CDR3 comprising the amino acid sequence of SEQ ID NO: 103, and the V_{L} comprises the LC -CDR1 comprising the amino acid sequence of SEQ ID NO: 104, the LC -CDR2 comprising the amino acid sequence of SEQ ID NO: 105, and the LC -CDR3 comprising the amino acid sequence of SEQ ID NO:106.

16. The anti-CD137 construct of any one of claim 1-4, wherein the V_{H} comprises the HC -CDR1 comprising the amino acid sequence of SEQ ID NO: 111, the HC -CDR2 comprising the amino acid sequence of SEQ ID NO: 112, and the HC -CDR3 comprising the amino acid sequence of SEQ ID NO: 113, and the V_{L} comprises the LC-CDR1 comprising the amino acid sequence of SEQ ID NO: 114, the LC-CDR2 comprising the amino acid sequence of SEQ ID NO: 115, and the LC-CDR3 comprising the amino acid sequence of SEQ ID NO: 116.

17. The anti-CD137 construct of any one of claim 1-4, wherein the V_{H} comprises the HC -CDR1 comprising the amino acid sequence of SEQ ID NO: 121, the HC -CDR2 comprising the amino acid sequence of SEQ ID NO: 122, and the HC -CDR3 comprising the amino acid sequence of SEQ ID NO: 123, and the V_{L} comprises the LC-CDR1 comprising the amino acid sequence of SEQ ID NO: 124, the LC-CDR2 comprising the amino acid sequence of SEQ ID NO: 125, and the LC-CDR3 comprising the amino acid sequence of SEQ ID NO: 126.

18. The anti-CD137 construct of any one of claim 1-4, wherein the V_{H} comprises the HC -CDR1 comprising the amino acid sequence of SEQ ID NO: 131, the HC -CDR2 comprising the amino acid sequence of SEQ ID NO: 132, and the HC -CDR3 comprising the amino acid sequence of SEQ ID NO: 133, and the V_{L} comprises the LC-CDR1 comprising the amino acid sequence of SEQ ID NO: 134, the LC-CDR2 comprising the amino acid sequence of SEQ ID NO: 135, and the LC-CDR3 comprising the amino acid sequence of SEQ ID NO: 136.

19. The anti-CD137 construct of any one of claim 1-4, wherein the V_{H} comprises the HC -CDR1 comprising the amino acid sequence of SEQ ID NO: 141, the HC -CDR2 comprising the amino acid sequence of SEQ ID NO: 142, and the HC -CDR3 comprising the amino acid sequence of SEQ ID NO: 143, and the V_{L} comprises the LC-CDR1 comprising the amino acid sequence of SEQ ID NO: 144, the LC-CDR2 comprising the amino acid sequence of SEQ ID NO: 145, and the LC-CDR3 comprising the amino acid sequence of SEQ ID NO: 146.

20. The anti-CD137 construct of any one of claim 1-4, wherein the V_{H} comprises the HC -CDR1 comprising the amino acid sequence of SEQ ID NO: 211, the HC -CDR2 comprising the amino acid sequence of SEQ ID NO: 212, and the HC -CDR3 comprising the amino acid sequence of SEQ ID NO: 213, and the V_{L} comprises the LC-CDR1 comprising the amino acid sequence of SEQ ID NO: 214, the LC-CDR2 comprising the amino acid sequence of SEQ ID NO: 215, and the LC-CDR3 comprising the amino acid sequence of SEQ ID NO: 216.

21. The anti-CD137 construct of any one of claim 1-4, wherein the V_{H} comprises the HC -CDR1 comprising the amino acid sequence of SEQ ID NO: 221, the HC -CDR2 comprising the amino acid sequence of SEQ ID NO: 222, and the HC -CDR3 comprising the amino acid sequence of SEQ ID NO: 223, and the V_{L} comprises the LC-CDR1 comprising the amino acid sequence of SEQ ID NO: 224, the LC-CDR2 comprising the amino acid sequence of SEQ ID NO: 225, and the LC-CDR3 comprising the amino acid sequence of SEQ ID NO: 226.

22. An isolated anti-CD137 construct comprising an antibody moiety that binds to CD137 comprising:
a) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 7; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 8;
b) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 17; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 18;
c) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 27; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 28;
d) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 37; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 38;
e) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 47; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 48;
f) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 57; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 58;
g) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 67; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 68;
h) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 77; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 78;
i) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 87; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 88;
j) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 97; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 98;
k) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 107; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 108;
l) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 117; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 118;
m) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 127; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 128;
n) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 137; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 138;
o) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 217; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 218; and
p) a HC-CDR1, a HC-CDR2, and a HC-CDR3, respectively comprising the amino acid sequences of a CDR1, a CDR2, and a CDR3 within a V_{H} chain region having the sequence set forth in SEQ ID No: 227; and a LC-CDR1, a LC-CDR2, and a LC-CDR3, respectively comprising the amino acid sequences of a CDR1, a CDR2, and a CDR3 within a V_{L} chain region having the sequence set forth in SEQ ID No: 228.

23. The anti-CD137 construct of any one of claim 1-22, comprising an anti-CD137 antibody moiety comprising:
(a) a heavy chain variable region comprising amino acids having the sequence set forth in SEQ ID NO: 7; and a light chain variable region comprising amino acids having the sequence set forth in SEQ ID NO: 8;
(b) a heavy chain variable region comprising amino acids having the sequence set forth in SEQ ID NO: 17; and a light chain variable region comprising amino acids having the sequence set forth in SEQ ID NO: 18;
(c) a heavy chain variable region comprising amino acids having the sequence set forth in SEQ ID NO: 27; and a light chain variable region comprising amino acids having the sequence set forth in SEQ ID NO: 28;
(d) a heavy chain variable region comprising amino acids having the sequence set forth in SEQ ID NO: 37; and a light chain variable region comprising amino acids having the sequence set forth in SEQ ID NO: 38;
(e) a heavy chain variable region comprising amino acids having the sequence set forth in SEQ ID NO: 47; and a light chain variable region comprising amino acids having the sequence set forth in SEQ ID NO: 48;
(f) a heavy chain variable region comprising amino acids having the sequence set forth in SEQ ID NO: 57; and a light chain variable region comprising amino acids having the sequence set forth in SEQ ID NO: 58;
(g) a heavy chain variable region comprising amino acids having the sequence set forth in SEQ ID NO: 67; and a light chain variable region comprising amino acids having the sequence set forth in SEQ ID NO: 68;
(h) a heavy chain variable region comprising amino acids having the sequence set forth in SEQ ID NO: 77; and a light chain variable region comprising amino acids having the sequence set forth in SEQ ID NO: 78;
(i) a heavy chain variable region comprising amino acids having the sequence set forth in SEQ ID NO: 87; and a light chain variable region comprising amino acids having the sequence set forth in SEQ ID NO: 88;
(j) a heavy chain variable region comprising amino acids having the sequence set forth in SEQ ID NO: 97; and a light chain variable region comprising amino acids having the sequence set forth in SEQ ID NO: 98;
(k) a heavy chain variable region comprising amino acids having the sequence set forth in SEQ ID NO: 107; and a light chain variable region comprising amino acids having the sequence set forth in SEQ ID NO: 108;
(l) a heavy chain variable region comprising amino acids having the sequence set forth in SEQ ID NO: 117; and a light chain variable region comprising amino acids having the sequence set forth in SEQ ID NO: 118;
(m) a heavy chain variable region comprising amino acids having the sequence set forth in SEQ ID NO: 127; and a light chain variable region comprising amino acids having the sequence set forth in SEQ ID NO: 128;
(n) a heavy chain variable region comprising amino acids having the sequence set forth in SEQ ID NO: 137; and a light chain variable region comprising amino acids having the sequence set forth in SEQ ID NO: 138;
(o) a heavy chain variable region comprising amino acids having the sequence set forth in SEQ ID NO: 217; and a light chain variable region comprising amino acids having the sequence set forth in SEQ ID NO: 218; or
(p) a heavy chain variable region comprising amino acids having the sequence set forth in SEQ ID NO: 227; and a light chain variable region comprising amino acids having the sequence set forth in SEQ ID NO: 228.

24. An isolated anti-CD137 construct comprising an anti-CD137 antibody moiety that binds to CD137, comprising a heavy chain variable region (V_{H}) and a light chain variable region (V_{L}), wherein:
a) the V_{H} comprises:
i) an HC-CDR1 comprising an amino acid sequence of any one of SEQ ID NOs: 151-153, or a variant thereof comprising up to about 3 amino acid substitutions;
ii) an HC-CDR2 comprising an amino acid sequence of any one of SEQ ID NOs: 154-156, or a variant thereof comprising up to about 3 amino acid substitutions;
iii) an HC-CDR3 comprising an amino acid sequence of any one of SEQ ID NOs: 157-159, or a variant thereof comprising up to about 3 amino acid substitutions; and
b) the V_{L} comprises:
i) an LC-CDR1 comprising an amino acid sequence of any one of SEQ ID NOs: 160-163, or a variant thereof comprising up to about 3 amino acid substitutions;
ii) a LC-CDR2 comprising an amino acid sequence of any one of SEQ ID NOs: 164-166, or a variant thereof comprising up to about 3 amino acid substitutions;
iii) a LC-CDR3 comprising an amino acid sequence of any one of SEQ ID NOs: 167-169, or a variant thereof comprising up to about 3 amino acid substitutions.

25. The anti-CD137 construct of claim 24, wherein:
a) the V_{H} comprises the HC-CDR1 comprising the amino acid sequence of SEQ ID NO: 151, an HC-CDR2 comprising the amino acid sequence of SEQ ID NO: 154, and the HC-CDR3 comprising the amino acid sequence of SEQ ID NO: 157 , and the V_{L} comprises the LC-CDR1 comprising the amino acid sequence of SEQ ID NO: 160, an LC-CDR2 comprising the amino acid sequence of SEQ ID NO: 164, and the LC-CDR3 comprising the amino acid sequence of SEQ ID NO: 167;
b) the V_{H} comprises the HC-CDR1 comprising the amino acid sequence of SEQ ID NO: 151, an HC-CDR2 comprising the amino acid sequence of SEQ ID NO: 154, and the HC-CDR3 comprising the amino acid sequence of SEQ ID NO: 157, and the V_{L} comprises the LC-CDR1 comprising the amino acid sequence of SEQ ID NO: 162, an LC-CDR2 comprising the amino acid sequence of SEQ ID NO: 166, and the LC-CDR3 comprising the amino acid sequence of SEQ ID NO: 169;
c) the V_{H} comprises the HC-CDR1 comprising the amino acid sequence of SEQ ID NO: 152, an HC-CDR2 comprising the amino acid sequence of SEQ ID NO: 155, and the HC-CDR3 comprising the amino acid sequence of SEQ ID NO: 158, and the V_{L} comprises the LC-CDR1 comprising the amino acid sequence of SEQ ID NO: 163, an LC-CDR2 comprising the amino acid sequence of SEQ ID NO: 166, and the LC-CDR3 comprising the amino acid sequence of SEQ ID NO: 169;
d) the V_{H} comprises the HC-CDR1 comprising the amino acid sequence of SEQ ID NO: 153, an HC-CDR2 comprising the amino acid sequence of SEQ ID NO: 156, and the HC-CDR3 comprising the amino acid sequence of SEQ ID NO: 159, and the V_{L} comprises the LC-CDR1 comprising the amino acid sequence of SEQ ID NO: 160, an LC-CDR2 comprising the amino acid sequence of SEQ ID NO: 164, and the LC-CDR3 comprising the amino acid sequence of SEQ ID NO: 167; or
e) the V_{H} comprises the HC-CDR1 comprising the amino acid sequence of SEQ ID NO: 153, an HC-CDR2 comprising the amino acid sequence of SEQ ID NO: 156, and the HC-CDR3 comprising the amino acid sequence of SEQ ID NO: 159, and the V_{L} comprises the LC-CDR1 comprising the amino acid sequence of SEQ ID NO: 161, an LC-CDR2 comprising the amino acid sequence of SEQ ID NO: 165, and the LC-CDR3 comprising the amino acid sequence of SEQ ID NO: 168.

26. The anti-CD137 construct of any one of claims 1-25, wherein the construct comprises an antibody or antigen-binding fragment thereof selected from the group consisting of a full-length antibody, a multispecific antibody (e.g., a bispecific antibody), a single-chain Fv (scFv), a Fab fragment, a Fab' fragment, a F(ab')2, an Fv fragment, a disulfide stabilized Fv fragment (dsFv), a (dsFv)₂, a V_{H}H, a Fv-Fc fusion, a scFv-Fc fusion, a scFv-Fv fusion, a diabody, a tribody, and a tetrabody.

27. The anti-CD137 construct of claim 26, wherein the construct comprises a humanized anti-CD137 full-length antibody.

28. The anti-CD137 construct of claim 26, wherein the construct comprises a humanized anti-CD137 single chain Fv.

29. The anti-CD137 construct of any one of claims 1-28, wherein the anti-CD137 antibody moiety is a CD137 agonist antibody.

30. The anti-CD137 construct of any one of claims 1-29, wherein the construct comprises an anti-CD137 antibody moiety comprising a Fc fragment of a human immunoglobulin.

31. The anti-CD137 construct of any one of claims 1-30, wherein the Fc fragment is selected from the group consisting of Fc fragments of IgG, IgA, IgD, IgE, and IgM.

32. The anti-CD137 construct of any one of claims 1-31, wherein the Fc fragment is selected from the group consisting of Fc fragments of IgG1, IgG2, IgG3 and IgG4.

33. The anti-CD137 construct of claim 32, wherein the Fc fragment is an IgG1 Fc fragment.

34. The anti-CD137 construct of claim 32, wherein the Fc fragment is an IgG2 Fc fragment.

35. The anti-CD137 construct of claim 32, wherein the Fc fragment is an IgG4 Fc fragment.

36. The anti-CD137 construct of any one of claims 1 to 35, wherein the antibody moiety comprises an IgG1 Fc fragment comprising one or more mutation selected from the group consisting of S267E, L328F and combination thereof.

37. The anti-CD137 construct of any one of claims 1 to 35, wherein the antibody moiety comprises an IgG2 Fc fragment comprising one or more mutation selected from the group consisting of S267E, L328F and combination thereof.

38. The anti-CD137 construct of any one of claims 1 to 35, wherein the antibody moiety comprises an IgG4 Fc fragment comprising a S228P mutation.

39. The anti-CD137 construct of any one of claims 1-38, wherein the antibody moiety binds to a human CD137 and a simian CD137.

40. The anti-CD137 construct of any one of claims 1-39, wherein the antibody moiety does not bind to a murine CD137.

41. The anti-CD137 construct of any one of claims 1-40, wherein the construct is a multispecific antibody.

42. The anti-CD137 construct of any one of claims 1-41, wherein the construct further comprises a second antibody moiety that binds to a second antigen, wherein the second antibody moiety comprises a second heavy chain variable region (V_{H-2}) and a second light chain variable region (V_{L-2}).

43. The anti-CD137 construct of claim 42, wherein the second antigen is a tumor associated antigen.

44. The anti-CD137 construct of claim 43, wherein the tumor associated antigen is selected from the group consisting of Her-2, EGFR, PD-L1, c-Met, B Cell Maturation Antigen (BCMA), carbonic anhydrase IX (CA1X), carcinoembryonic antigen (CEA), CD5, CD7, CD10, CD19, CD20, CD22, CD30, CD33, CD34, CD38, CD41, CD44, CD49f, CD56, CD74, CD123, CD133, CD138, CD276 (B7H3), epithelial glycoprotein (EGP2), trophoblast cell-surface antigen 2 (TROP-2), epithelial glycoprotein- 40 (EGP-40), epithelial cell adhesion molecule (EpCAM), receptor tyrosine-protein kinases erb-B2,3,4, folate-binding protein (FBP), fetal acetylcholine receptor (AChR), folate receptor-a, Ganglioside G2 (GD2), Ganglioside G3 (GD3), human telomerase reverse transcriptase (hTERT), kinase insert domain receptor (KDR), Lewis A (CA 1.9.9), Lewis Y (LeY), Glypican-3 (GPC3), L1 cell adhesion molecule (LICAM), Mucin 16 (Muc-16), Mucin 1 (Muc-1), NG2D ligands, oncofetal antigen (h5T4), prostate stem cell antigen (PSCA), prostate-specific membrane antigen (PSMA), tumor- associated glycoprotein 72 (TAG-72), Claudin18.2 (CLDN18.2), vascular endothelial growth factor R2 (VEGF- R2), Wilms tumor protein (WT-1), type 1 tyrosine-protein kinase transmembrane receptor (ROR1) and any combination thereof.

45. An immunoconjugate comprising the anti-CD137 construct of any one of claims 1-44, linked to a therapeutic agent or a label.

46. The immunoconjugate of claim 45, wherein the label is selected from the group consisting of a radioisotope, a fluorescent dye and an enzyme.

47. A pharmaceutical composition comprising the anti-CD137 construct of any one of claims 1-44 or the immunoconjugate of claim 45 or 46 and a pharmaceutically acceptable carrier.

48. An isolated nucleic acid encoding the anti-CD137 construct of any one of claims 1-44.

49. A vector comprising the isolated nucleic acid of claim 48.

50. An isolated host cell comprising the isolated nucleic acid of claim 48, or the vector of claim 49.

51. A method of producing an anti-CD137 construct, comprising:
a) culturing the isolated host cell of claim 50 under conditions effective to express the anti-CD137 construct; and
b) obtaining the expressed anti-CD137 construct from the host cell.

52. A method of treating or preventing a disease in an individual, comprising administering to the individual an effective amount of the anti-CD137 construct of any one of claims 1-44, the immunoconjugate of claim 45 or 46 or the pharmaceutical composition of claim 47.

53. The method of claim 52, wherein the disease is a cancer or a tumor.

54. The method of claim 53, wherein the cancer is selected from the group consisting of breast cancer, gastric cancer, ovarian cancer, lung cancer, mesothelioma, endometrial cancer, cervical cancer, esophageal cancer, bladder cancer, salivary gland cancer, testicular cancer, renal cancer, liver cancer, pancreatic cancer, colorectal cancer, skin cancer, thymus cancer, adrenal cancer, head and neck cancer, brain cancer, thyroid cancer, sarcoma, myeloma and leukemia.

55. The method of any one of claims 52-54, wherein the cancer is breast cancer or gastric cancer.

56. The method of any one of claims 52-54, wherein the cancer is lung cancer, colorectal cancer or head and neck cancer.

57. The method of any one of claims 52-56, wherein the individual is a human.

58. An anti-CD 137 construct of any one of claims 1-44 for use as a medicament.

59. An anti-CD 137 construct of any one of claims 1-44 for use in treating cancer.

60. The anti-CD137 construct of claim 59, wherein the cancer is selected from the group consisting of breast cancer, gastric cancer, ovarian cancer, lung cancer, mesothelioma, endometrial cancer, cervical cancer, esophageal cancer, bladder cancer, salivary gland cancer, testicular cancer, renal cancer, liver cancer, pancreatic cancer, colorectal cancer, skin cancer, thymus cancer, adrenal cancer, head and neck cancer, brain cancer, thyroid cancer, sarcoma, myeloma and leukemia.

61. A kit comprising anti-CD137 construct or the multispecific antibody of any one of claims 1-44, the immunoconjugate of any one of claim 45 or 46, a pharmaceutical composition of claim 47, the nucleic acid of claim 48, the vector of claim 49 or the host cell of claim 50.

62. The kit of claim 61, further comprising a written instruction for treating and/or preventing a cancer or a tumor.
